# EUROPEAN PATENT APPLICATION

(11) **EP 2 639 305 A1**
(43) Date of publication of application: **18.09.2013**
(21) Application number: 11840492.0
(22) Date of filing: 09.11.2011
(51) Int. Cl.: C12N 15/09, C12Q 1/04, C12Q 1/68, G01N 33/53, C12N 5/0783

(54) **MARKERS FOR DETECTING HUMAN FOLLICULAR HELPER T CELLS AND METHOD FOR DETECTING HUMAN FOLLICULAR HELPER T CELLS**

(30) Priority: 11.11.2010 JP 2010253140
(71) Applicant: Sysmex Corporation, Kobe-shi, Hyogo 651-0073 (JP)
(72) Inventor: YANAGIDA, Masatoshi, Kobe-shi Hyogo 651-0073 (JP); UGA, Hitoshi, Kobe-shi Hyogo 651-0073 (JP); OKAZAWA, Takahiro, Kobe-shi Hyogo 651-0073 (JP); MIYAMOTO, Yoshiaki, Kobe-shi Hyogo 651-0073 (JP); IKEDA, Masafumi, Kobe-shi Hyogo 651-0073 (JP); KURATA, Hirokazu, Kobe-shi Hyogo 651-0073 (JP)
(74) Representative: HOFFMANN EITLE
(86) International application number: PCT/JP2011/075856
(87) International publication number: WO 2012/063875

(57) **Abstract**

The present invention relates to polynucleotide markers and protein markers for detecting human follicular helper T cells. The present invention also relates to methods for detecting human follicular helper T cells using the markers.

SEQUENCE LISTING

<110>  Sysmex Corporation

<120>  Markers for detection of T follicular helper cells and Method for
       detection of T follicular helper cells

<130>  TM5626PC

<150>  JP 2010-253140
<151>  2010-11-11

<160>  182

<170>  PatentIn version 3.5

<210>  1
<211>  534
<212>  DNA
<213>  Homo sapiens

<400>  1
gaatattttc aacccatcta tgtctacaac cctggtgaga aaaaccagaa gcttgaagac      60

catagtgaga aaaactggaa gcttgaagac catggtgaga aaaaccagaa gcttgaagac     120

catggtgtga aaatccttga acccacccaa atacctgctc caggtccagt tcctgttcca     180

ggtcccaaaa gccatccttc tgcatcctcg ggcaaactgc tgcttccaca gtttgggatg     240

gtcatcattt taatcagtgt ttctgctata aatctctttg ttgctctgta gttttgatgc     300

attgttttat ctctcttatt cctttacttg aaataactta tagggatcca caggagatca     360

aaaggaatga tgtatttttt acgtgttggc caaagtcact ggataaaatg agaattgtat     420

atttgttatt cattttgcaa attcagaaag ttggtccaga tatatgtcac agaacttttc     480

acttgtatac tactcttaca atggaaaaaa atcccgaaaa ctgtatactt ctga           534


<210>  2
<211>  438
<212>  DNA
<213>  Homo sapiens

<400>  2
gtactcgaaa aatcaaacct tctgaaaatt atgatatttg tacttcattt gtgtattagt      60

tcattttctg tttctattac atagttttta aagtcaatga aagagattga gaatgttaac     120

atttgttgat caatgtggag ctgtccacat tcggtatctt ttcattttta tatatatttt     180

acagtttcaa gaaaacttag tactactagt atctgcctta ttttcttaag tctgatgtta     240

atttttgttt caggagtgag atataaacat tttgcttttt ttcagttttg aaatatagtt     300

tgtcatcctg tgaaaaccac ctcgtttgct ttttgtttgt ttgttttgct tttttagtgt     360

agacctaaaa ttatttttgc ctataaacag tgaacttaat atgtttctgt gagagtgaca     420

gagctgtgcc cgtttatc                                                   438

<210> 3
<211> 444
<212> DNA
<213> Homo sapiens

<400> 3
cagtgtcttc aggcaatgat tgtgtatcct gttacagatg gttgtgatac aagaagaacc 60

actcttcttt gaaaataaac tcttggaagc ttttgccagc tatctggggg gtaggaggaa 120

tattagcaac tgtattggtt gtctacagat acagaattgc ctgttgtgag ggaactgatt 180

gttttgttgg gaaaagaaat ttaccaggag aaagagtttt gtgctgtatt gtgagagatc 240

tcgcctctca gttaaatgag ccctgggtta aagtcagtgt gaagggcagc tgtgtgcggg 300

cacgagccag agtgtctgcc tcagactaga tttgacttga gttctttatg acccaggact 360

ctggataatg tgaatttgct ttcctattta actagaagat acatgtacta tagatcattg 420

tctcatttta gtgattgttc ctta 444

<210> 4
<211> 156
<212> DNA
<213> Homo sapiens

<400> 4
tctcaactta ttcccttcaa ttcaagtaac aggaaacaag atttggtgga agcagtcgcc 60

catgcttgta gcgtacgaca atgcggtcaa ccttagctac aatgagaaga gcaatggaac 120

cattatccat gtgaaaggga aacacctttg tccaag 156

<210> 5
<211> 185
<212> DNA
<213> Homo sapiens

<400> 5
ctgatgccat caaagaggtc ttcgacaata aattccacat catcggcgca gtgggcatcg 60

gcattgccgt ggtcatgata tttggcatga tcttcagtat gatcttgtgc tgtgctatcc 120

gcaggaaccg cgagatggtc tagagtcagc ttacatccct gagcaggaaa gtttacccat 180

gaaga 185

<210> 6
<211> 477
<212> DNA
<213> Homo sapiens

<400> 6
gtggccgtag caacttggcg gagacaggct atgagtctga cgttagagtg gttgcttcct 60

tagcctttca ggatggagga atgtgggcag tttgacttca gcactgaaaa cctctccacc 120

tgggccaggg ttgcctcaga ggccaagttt ccagaagcct cttacctgcc gtaaaatgct 180

caaccctgtg tcctgggcct gggcctgctg tgactgacct acagtggact ttctctctgg 240

aatggaacct tcttaggcct cctggtgcaa cttaattttt ttttttaatg ctatcttcaa 300

aacgttagag aaagttcttc aaaagtgcag cccagagctg ctgggcccac tggccgtcct 360

gcatttctgg tttccagacc ccaatgcctc ccattcggat ggatctctgc gtttttatac 420

tgagtgtgcc taggttgccc cttatttttt attttccctg ttgcgttgct atagatg 477

<210> 7
<211> 518
<212> DNA
<213> Homo sapiens

<220>
<221> misc_feature
<222> (159)..(166)
<223> n is a, c, g, or t

<400> 7
ttcttctatc caactctctt atgaaaagga actctgtatt ttctattagc cataaataat 60

ctgtccactg tattttacag gtcttcatac ttttacttaa ttttctttat ctgtatggca 120

aaccactgca atcctgaatg acatggaaag catcacaann nnnnnncctt tgcttgaatt 180

cctggaatgc atacatataa gctaaacaga tgtctgcagt tataaatgtc ataagtagag 240

gtacaatctc accctgctcc ttagaaacat ttccatataa atcgctaaaa taatttcaca 300

tttttgttag tttaatatat acatgagttt atttctgata taaataataa atacagagag 360

tgagcatatc agagaggcaa attcttaaag aatgattttt aaaatcagct ctaggaagag 420

ctcaagatca attggtcata gaacagcatt tgacgcctag aactatgacc acctcatggt 480

cagagatgag aatgtagcct ttgtgaccag attatatt 518

<210> 8
<211> 493
<212> DNA
<213> Homo sapiens

<220>
<221> misc_feature
<222> (35)..(35)
<223> n is a, c, g, or t

<400> 8
taacctccct gtagtcttta aaatgtttaa cttgnctata agctaacatg taataaaaca 60

catactcaat tatatgaata gaagtggaga gccagaatgc tacaaaagaa atcacagctg 120

ctagaagtat cctccataga aacatcaaca tgattgtgga tcaaaatgat tttcactgga 180

tatggaattt gtatgggcca tatttattaa aagagttctg tgtggtcaca tagagttcct 240

ttgggatttc atcccacttt tcaggactta atttgtttgg gtttgcttac ctaacaagca 300

acaacaacaa caaaataaat agttccaaaa tctagtttat tacataaatc tctatgaatc 360

cttgagggcc tatatctata ttataaataa atacaaatat agatttttaa atatctatgg 420

gactttgcca tttacagcct taagtataaa attacgagat tatattcttt ccattaccct 480

ttatttctgc taa 493

<210> 9
<211> 395
<212> DNA
<213> Homo sapiens

<220>
<221> misc_feature
<222> (28)..(28)
<223> n is a, c, g, or t

<220>
<221> misc_feature
<222> (47)..(48)
<223> n is a, c, g, or t

<400> 9
gagaatgctt gacttgttgg tctgtgtnaa tgtgtatata tatatanncc tgaatacagg 60

aacatcggag acctattcac tcccacacac tctgctatag tttgcgtgct tttgtggaca 120

cccctcatga acaggctggc gctctaggac gctctgtgtt cactgatgat gaagaaacct 180

agaactccaa gcctgtttgt aaacacacta aacacagtgg cctagataga aactgtatcg 240

tagtttaaaa tctgcctcgc gggatgttac taaactcgct aatagtttaa aggttactta 300

caatagagca agttggacaa ttttgtggtg ttggggaaat gttagggcaa ggcctagagg 360

ttcattttga atcttggttt gtgactttag ggtag 395

<210> 10
<211> 476
<212> DNA
<213> Homo sapiens

<220>
<221> misc_feature
<222> (181)..(181)
<223> n is a, c, g, or t

<220>
<221> misc_feature
<222> (209)..(209)
<223> n is a, c, g, or t

<400> 10
tctggccttc tataattttc tggtagtcac gatggaaaca aacaaaacac cttagaacca 60

gagatcgacc ccctcaagca atacatcaat tcccttcaca agaaacgtcg gggctacatg 120

agtatctgtg ttgaatgcgg tctgaaatga tcctatggat tttcccggct ggttgccact 180

nctgtacaac attcagtgcc cacatccanc tgtgccatta agcttttttg agacatgaga 240

gatgcctctt ccctgctgta tgacatgcat ttgggaagtt ggaaagaaat gacaaaatca 300

gggagaaaac atccaagctt cttacctgta gatagaatca gccctcactt ggtgcttatt 360

accagttatt caagaacaat aacaacaaca aaattagtag acatccaaga agcacatatt 420

aggaccaaag atagcatcaa ctgtatttga aggaactgta gtttgcgcat tttatg 476

<210> 11
<211> 375
<212> DNA
<213> Homo sapiens

<400> 11
gcaaccccaa cagcttgaag aggacaggga cggagagctc cagacctaac actgccttgt 60

tggatccaag ctcccctgaa ttctctgctg tggtatcagt gggcgattgg ctccaggcca 120

ttaaaatgga ccggtataag gataacttca cagctgctgg ttataccaca ctagaggctg 180

tggtgcacgt gaaccaggag gacctggcaa gaattggtat cacagccatc acgcaccaga 240

ataagatttt gagcagtgtc caggcaatgc gaacccaaat gcagcagatg cacggcagaa 300

tggttcccgt ctgagccagt actgaataaa ctcaaaactc ttgaaattag tttacctcat 360

ccatgcactt taatt 375

<210> 12
<211> 136
<212> DNA
<213> Homo sapiens

<400> 12
tctactcatt caaaaggtca taactcagga gtgctgttta taccagatga atctacaaag 60

ccaagaacag gaatcaccgt atctccttca gtagacctga tactgtgagt cttctctttt 120

ctgttgacat atttgt 136

<210> 13
<211> 496
<212> DNA
<213> Homo sapiens

<220>
<221> misc_feature
<222> (126)..(128)
<223> n is a, c, g, or t

<220>
<221> misc_feature
<222> (130)..(132)
<223> n is a, c, g, or t

<220>
<221>  misc_feature
<222>  (137)..(139)
<223>  n is a, c, g, or t

<400>  13
gcaaagcctt taagtttttc cactggaatt ctaacctcac cattctgaaa accaacataa      60

gtcacaatgg cacctaccat tgctcaggca tgggaaagca tcgctacaca tcagcaggaa     120

tatcannntn nnctgtnnna ggcctccagt taccaactcc tgtctggttt catgtccttt     180

tctatctggc agtgggaata atgtttttag tgaacactgt tctctgggtg acaatacgta     240

aagaactgaa aagaaagaaa aagtggaatt tagaaatctc tttggattct ggtcatgaga     300

agaaggtaat ttccagcctt caagaagaca gacatttaga agaagagctg aaatgtcagg     360

aacaaaaaga agaacagctg caggaagggg tgcaccggaa ggagccccag ggggccacgt      420

agcagcggct cagttggtgg ccatcgatct ggaccgtccc ctgcccactt gctccccatg     480

agcactgcgt acaaac                                                     496

<210>  14
<211>  217
<212>  DNA
<213>  Homo sapiens

<400>  14
gtggcagaac cctgcaggga catggaatta aggccattgc aatgtatcat ctttgtagca      60

ttgtcatcac tcctaagctg ccttcacagt tttagtacac taagatgagg aaatcgaaaa     120

tgggcagaga aagctcatac tgtataattg aagacagtga cagagaacgt gtcagttatg     180

ccaaaactct tttgatttct gttccaggat ttccaac                              217

<210>  15
<211>  455
<212>  DNA
<213>  Homo sapiens

<400>  15
tctacctgtc ctttgagaac tctcagcacc gcgactacat tacggagaaa ttctggcgca      60

acgcactggt ggctggcact gtgccagtgg tgctggggcc cccacgggcc acctatgagg     120

ccttcgtgcc ggctgacgcc ttcgtgcatg tggatgactt tggctcagcc cgagagctgg     180

cggctttcct cactggcatg aatgagagcc gataccaacg cttctttgcc tggcgtgaca     240

ggctccgcgt gcgactgttc accgactggc gggaacgttt ctgtgccatc tgtgaccgct     300

acccacacct accccgcagc caagtctatg aggaccttga gggttggttt caggcctgag     360

atccgctggc cgggggaggt gggtgtgggt ggaagggctg ggtgtcgaaa tcaaaccacc     420

aggcatccgg cccttaccgg caagcagcgg gctaa                                455

<210>  16
<211>  508
<212>  DNA
<213>  Homo sapiens

<400>  16
gatcttaaag ccacggagaa gcctctcatc ttatggcatt gacaaagaga agaccatcca      60

ccttaccctg aaagtggtga agcccagtga tgaggagctg cccttgtttc ttgtggagtc     120

aggtgatgag gcaaagaggc acctcctcca ggtgcgaagg tccagctcag tggcacaagt     180

gaaagcaatg atcgagacta agacgggtat aatccctgag acccagattg tgacttgcaa     240

tggaaagaga ctggaagatg ggaagatgat ggcagattac ggcatcagaa agggcaactt     300

actcttcctg gcatcttatt gtattggagg gtgaccaccc tggggatggg gtgttggcag     360

gggtcaaaaa gcttatttct tttaatctct tactcaacga acacatcttc tgatgatttc     420

ccaaaattaa tgagaatgag atgagtagag taagatttgg gtgggatggg taggatgaag     480

tatattgccc aactctatgt ttctttga                                        508


<210>  17
<211>  245
<212>  DNA
<213>  Homo sapiens

<400>  17
ttctctctgt gtggcaaaca tttaaaaaaa aaaaaaaaaa gcaggaaaga tcccaagtca      60

aacagtgtgg cttaaacatt ttttgtttct tggtgttgtt atggcaagtt tttggtaatg     120

atgattcaat cattttggga aattcttgca ctgtatccaa gttatttgat ctggtgcgtg     180

tggccctgtg ggagtccact ttcctctctc tctctctctc tgttccaagt gtgtgtgcaa     240

tgttc                                                                 245


<210>  18
<211>  466
<212>  DNA
<213>  Homo sapiens


<220>
<221>  misc_feature
<222>  (245)..(245)
<223>  n is a, c, g, or t

<400>  18
gggagaacta ggtgtccaga tttttgtatg tgttgttttt cttgggggat ggggtggggt      60

gggagtaggt agagctgaga atactacatc ttagtggtga cctttagcca cgtgggtgaa     120

gtggcaaagg ccatggccat atctgttgtc ccaggccaaa gactaacaac tgccttggga     180

atcccttcct tgtgtcctta ccaaatgata gctcataaaa ctctgataat gtaacaaatc     240

acttncaaag gagttcccag aagtcttcag aaagactaaa attctgtctc ttcctgcttt 300

agacagccat taagatccca actaatttta ccgaacctaa aacccacaaa gaggttgttt 360

gtgttattgt tcaatcttca gttgtaagag taattctcta tttttatatt gaaacataat 420

tacttgatag ctcagggtct acacttcatt caacttttta caccaa 466

<210> 19
<211> 164
<212> DNA
<213> Homo sapiens

<400> 19
atgcatacgg gtgaatgttt gtgtgcccat gagtatatgc atatgggtat atgtttgtgt 60

gcccgtgatt atatgcatat gggtgaatgt ttgtgtgccc gtgattatat gcatgtgggt 120

gaatgttcat gaacacaaac agctacactc ggagtagaaa acaa 164

<210> 20
<211> 481
<212> DNA
<213> Homo sapiens

<400> 20
atctgccaaa gcccagccaa ttaaaggagc cagaagagct ccggattctt catcggaatt 60

tgggaaggtg actcgaatgt gttttgctgt gttcctctgc tttgccctga gctacatccc 120

cttcttgctg ctcaacattc tggatgccag agtccaggct ccccgggtgg tccacatgct 180

tgctgccaac ctcacctggc tcaatggttg catcaaccct gtgctctatg cagccatgaa 240

ccgccaattc cgccaagcat atggctccat tttaaaaaga gggccccgga gtttccatag 300

gctccattag aactgtgacc ctagtcacca gaattcagga ctgtctcctc caggaccaaa 360

gtggccaggt aataggagaa taggtgaaat aacacatgtg ggcattttca caacaatctc 420

tccccagcct cccaaatcaa gtctctccat cacttgatca atgtttcagc cctagactgc 480

c 481

<210> 21
<211> 546
<212> DNA
<213> Homo sapiens

<400> 21
cttcactgtg tggacctaga gactccaact tgaattccta gtaactttct tggtatgctg 60

gccagaaagg gaaatgagga ggagagtaga aaccacagct cttagtagta atggcataca 120

gtctagagga ccattcatgc aatgactatt tctaaagcac ctgctacaca gcaggctgta 180

cacagcagat cagtactgtt caacagaact tcctgagatg atggaaatgt tctacctctg 240

cactcactgt ccagtacatt agacactagg cacattggct gttaatcact tggaatgtgt 300

ttagcttgac tgaggaatta aattttgatt gtaaatttaa atcgccacac atggctagtg 360

gctactgtat tggagtgcac agctctagat ggctcctaga ttattgagag cctccaaaac 420

aaatcaacct agttctatag atgaagacat aaaagacact ggtaaacacc aatgtaaaag 480

ggcccccaag gtggtcatga ctggtctcat ttgcagaagt ctaagaatgt acctttttct 540

ggccgg 546

<210> 22
<211> 263
<212> DNA
<213> Homo sapiens

<220>
<221> misc_feature
<222> (28)..(28)
<223> n is a, c, g, or t

<220>
<221> misc_feature
<222> (30)..(30)
<223> n is a, c, g, or t

<220>
<221> misc_feature
<222> (50)..(50)
<223> n is a, c, g, or t

<220>
<221> misc_feature
<222> (54)..(54)
<223> n is a, c, g, or t

<220>
<221> misc_feature
<222> (77)..(77)
<223> n is a, c, g, or t

<400> 22
catgtaagaa ttcccgggag ctccatgncn ttttaatttt agccattctn ctgncctcat 60

ttcttaaaat tagagantta aggtcccgaa ggtggaacat gcttcatggt cacacataca 120

ggcacaaaaa cagcattatg tggacgcctc atgtattttt tatagagtca actatttcct 180

ctttattttc cctcattgaa agatgcaaaa cagctctcta ttgtgtacag aaagggtaaa 240

taatgcaaaa tacctggtag taa 263

<210> 23
<211> 496
<212> DNA
<213> Homo sapiens

<400> 23
ttcaaatttt ctgcctgagg gaagccatct cagcaataat aatgaaatta cttccttaac 60

acttaaacca ccagttgatt ccttagactc aggaaataat cccaggttac aaaagcatcc 120

taattttgct ttttctgttt caagtgtgaa ttcactaagc aacacaatat ttcttggaga 180

attaagcctc atattaaatc aaggagaatg cagttctcct gacatacaaa actcagtaga 240

ggaggaaacc accatgcttt tggaaaatga ttcacccagt gaaactattc cagaacagac 300

cctgcttcct gatgaatttg tctcctgttt ggggatcgtg aatgaggagt tgccatctat 360

taatacttat tttccacaaa atattttgga aagccacttc aataggattt cactcttgga 420

aaagtagagc tgtgtggtca aaatcaatat gagaaagctg ccttgcaatc tgaacttggg 480

ttttccctgc aataga 496

<210> 24
<211> 477
<212> DNA
<213> Homo sapiens

<400> 24
   attgtggtga gcgatcctga ctccacagat gcttcaagca ttgaagacaa tgaggacatt 60
   tgtaacacca cctccttgga gaattgcaca gcaaaatgag cgggggtgtt tgtgcctgtt 120
   tctcttatcc tttcccaaca ttaggttaac acagctttat aaacctcagt gggttcgtta 180
   aaatcattta attctcaggg tgtacctttc cagccatagt tggacattca ttgctgaatt 240
   ctgaaatgat agaattgtct ttatttttct ctgtgaggtc aattaaatgc cttgttctga 300
   aatttatttt ttacaagaga gagttgtgat atagtttgga atataagata aatggtattg 360
   ggtggggttt gtggctacag cttatgcatc attctgtgtt tgtcatttac tcacattgag 420
   ctaactttaa attactgaca agtagaatca aaggtgcagc tgactgagac gacatgc 477

<210> 25
<211> 371
<212> DNA
<213> Homo sapiens

<400> 25
   tgggatatca gtgaactatg ttgtatactt ttgaattttt acattttata aatggaattg 60
   aaagttggat aactgctttt tttaaatttt ccaacagaag taacaccaca gttgctttgt 120
   ttctttttat agcttacctg aggttcagtt cttctttgtg aacctgtgag tactccacag 180
   tttactgggg gaaaaggctt cagtaaagca gaggctagaa ttacagtatt tatacatagc 240
   aacttttcat aaagtagaaa aattcaaagg aagctgtctc aatttgagaa taccagctgg 300
   gcacggtggc tcacgcctgt aatcccagca cttactttgg gaggccaagg tgggcagata 360
   acctgcggtc a 371

<210> 26
<211> 201
<212> DNA

<213>  Homo sapiens


<220>
<221>  misc_feature
<222>  (172)..(172)
<223>  n is a, c, g, or t

<220>
<221>  misc_feature
<222>  (175)..(175)
<223>  n is a, c, g, or t

<400>  26
tctctgagcg gacccggtgg gaatccgagc ggctccctcg ggagcgggga ctgctggaac      60

agcggctatt cttctgttat tagtattttt cttactgtcc aagatcaact gtggaaatat     120

tttggtctct tgtgacgttc ggtgcagttt cgttgtgaag tttgctattg anatngaaat     180

tatgacttct gtgtttcctg a                                               201


<210>  27
<211>  442
<212>  DNA
<213>  Homo sapiens


<220>
<221>  misc_feature
<222>  (75)..(75)
<223>  n is a, c, g, or t

<220>
<221>  misc_feature
<222>  (88)..(88)
<223>  n is a, c, g, or t

<220>
<221>  misc_feature
<222>  (119)..(120)
<223>  n is a, c, g, or t

<400>  27
   ttataactgc catctccaac tacatcctta tgatgttttt aacaataaaa ttaaaacaac      60
   tgttaaacta aaaanccaca ccgttttncc agtacttgat ctctgagcta caatactcnn     120
   ctaaatataa ttttccaatc aaaatattct attctatatt ctaagggtta atatgtgatt     180
   atagtgtcca cttgccacca tttttttaaa tcaatggact tgaaaagtat taatttagat     240
   ggatgcgcag atataccctc agttcagtca tagattggag tttgcatata ataatgtaaa     300
   tgtatgtcga cactattcta aatagttcta ttatgactga aatttaatta aataaaaaag     360
   gttgtaaaat gtgatgtgta tgtgtatata ctgtatgtgt actttttaaa ataggtgtat     420
   gtcccaaccc ttttttatac ag                                               442


<210>  28

<211>  472
<212>  DNA
<213>  Homo sapiens

<400>  28
acatacagac atatgcatcc ccacacacgc ctatgcacaa acgtggatta tcgcacagac      60

tgggaggttt agtggtgcat ttctcctctg ttttcttttt aatatacatt taaaatacag     120

tattatcact ttataaaaca tacattaagc ctaataaatg gaccaataag ccaaactatc     180

agtattttgt atatcctgca taaactctaa tttagttcct caacatattt tcagtgttta     240

tgcagacctt tagagttaag cctttgtatt tccatgttat tccacaatat gcaatatttc     300

tctgagtagc ttctgctatg atattcttat gaagaaaagg ggcaactttc tgtccactat     360

aggagagaat tcagccgaag atatgagagt aatgagagac attttccagt cattggatcg     420

tgttttcttt tgtccattat tgtactgtgc tgtaccacat ttatttctat at             472


<210>  29
<211>  444
<212>  DNA
<213>  Homo sapiens

<400>  29
agcccacctg ctggatgagg aacttgaggc aagtcaccag cccctgatca tttcgcctaa      60

aagagcaagg actagagttc ctgacctcca ggccagtccc tgatccctga cctaatgtta     120

tcgcggaatg atgatatatg tatctacggg ggcctggggc tgggcgggct cctgcttctg     180

gcagtggtcc ttctgtccgc ctgcctgtgt tggctgcatc gaagagcacc ttctgtcctg     240

gtcccaggcc cagggctcct cagagcagga actccactat gcatctctgc agaggctgcc     300

agtgcccagc agtgagggac ctgacctcag gggcagagac aagagaggca ccaaggagga     360

tccaagagct gactatgcct gcattgctga gaacaaaccc acctgagcac cccagacacc     420

ttcctcaacc caggcgggtg gaca                                            444


<210>  30
<211>  535
<212>  DNA
<213>  Homo sapiens


<220>
<221>  misc_feature
<222>  (45)..(81)
<223>  n is a, c, g, or t

<220>
<221>  misc_feature
<222>  (148)..(148)
<223>  n is a, c, g, or t

<220>
<221>  misc_feature

<222>  (152)..(154)
<223>  n is a, c, g, or t

<220>
<221>  misc_feature
<222>  (156)..(156)
<223>  n is a, c, g, or t

<220>
<221>  misc_feature
<222>  (202)..(205)
<223>  n is a, c, g, or t

<220>
<221>  misc_feature
<222>  (492)..(492)
<223>  n is a, c, g, or t

<400>  30
cgggcaccat gataccctat gttctgggga ttagcagtga ggaannnnnn nnnnnnnnnn        60

nnnnnnnnnn nnnnnnnnnn ngatgaggaa cttgaggcaa gtcaccagcc cctgatcatt       120

tcgcctaaaa gagcaaggac tagagttnct gnnntncagg ccagtccctg atccctgacc       180

taatgttatc gcggaatgat gnnnngccca gggctcctca gagcaggaac tccactatgc       240

atctctgcag aggctgccag tgcccagcag tgagggacct gacctcaggg gcagagacaa       300

gagaggcacc aaggaggatc caagagctga ctatgcctgc attgctgaga acaaacccac       360

ctgagcaccc cagacacctt cctcaaccca ggcgggtgga cagggtcccc ctgtggtcca       420

gccagtaaaa accatggtcc ccccacttct gtgtctcagt cctctcagtc catctcgagc       480

ctccgttcaa antgatcatc atcaaaactt atgtggcttt ttgacctttg aatag            535


<210>  31
<211>  431
<212>  DNA
<213>  Homo sapiens


<220>
<221>  misc_feature
<222>  (131)..(131)
<223>  n is a, c, g, or t

<400>  31
aagcagccta gggatgagca tttctttgaa agcaattagg ttattcacct ggtattaaaa        60

ctatttactg ttaaaaaatc tgtgacttca tgaagttgat ttttaaaggc agcatcaaaa       120

actgaaaagg naagggaaaa aataggcagc ttctctgcac ttgtttggag ctccccaaaa       180

caggagccat ggagaagtgg catcaagacc gggctgccct ttcgagaaca ccctgtggca       240

gttcagagac acgcttttcc tacactgcat gcagcccctc tttccagcac tggaaagaag       300

tggtcttgag cccagctgag aagcacttca cactcctctc tcttgttctg aatggtgttt       360

gtgtcagtct gcagctgtgt atggtattat gtcttataat cctgcatcac ttctatccta       420

tccagtcata t                                                          431

<210>  32
<211>  563
<212>  DNA
<213>  Homo sapiens

<220>
<221>  misc_feature
<222>  (35)..(35)
<223>  n is a, c, g, or t

<220>
<221>  misc_feature
<222>  (55)..(55)
<223>  n is a, c, g, or t

<220>
<221>  misc_feature
<222>  (96)..(96)
<223>  n is a, c, g, or t

<400>  32
aatggcttct atgatcagaa ctgggaaaac agtgnatctt atggtggaag aggtnctcag      60

caagtgtaca gtatttacct tcctttgtct tacatnggct ttttaaattt tccattaatt     120

tcaacataat tatgggaaca agtgtacaga agaatttttt ttttaagata tgtgagaact     180

tttcatagat gaacttttta acaaatgttt tcatttacag gaaattgcaa agaaaattct     240

caagtgatag tctttttttt taagtgtttc gtaagacaaa aattgaataa tgttttttga     300

agttctggca agattgaagt ctgatattgc agtaatgata tttattaaaa acccataact     360

accaggaata atgatacctc ccaccccttg attcccataa cataaaagtg ctacttgaga     420

gtgggggaga atggcatggt aggctacttt tcagggcctt gacaagtaca tcacccagtg     480

gtatcctaca tacttctttc aagatcttca accatgaggt aaaagagcca agttcaaaga     540

accctagcac aaatttgctt tgg                                            563

<210>  33
<211>  218
<212>  DNA
<213>  Homo sapiens

<400>  33
tcacttccct aatttcccta agtagtcctc acccatcact ctttccatct tttggccctg      60

ccgcttcctc acttacagtt tttctttcct gtcttaacta gaagagacct tcgacttaaa     120

ggtaaccact ttccctctga gatgctgtat aaaactggag gttaggggat atttatggtt     180

gctggtgata actttgttgt tctccaggca aaaacgtc                            218

<210>  34

<211>  535
<212>  DNA
<213>  Homo sapiens


<220>
<221>  misc_feature
<222>  (256)..(256)
<223>  n is a, c, g, or t

<220>
<221>  misc_feature
<222>  (502)..(502)
<223>  n is a, c, g, or t

<400>  34
tagagatcat ctagtcccat caactcacta tatatatgag gaacctgagg tccagagtgg      60

ggaagtgtct tacccaaggt cacatggttt cagagaaatt atgttgaatc caataagcct     120

tcccggacat tccaagcctc ttaaccatgg catctatgtt gaggatgtca atgtttattt     180

cagcaaagga cgtcatggct tttaaaaact ccttttaagc ctccttgttt tgatgtcacc     240

ttggtaggct gggccntctg agaggttgga agctctaggc attgttctct ttggatccag     300

ggatgctaag tagaaactgc atgagccacc agtgccccgg caccctttaa caccaccaga     360

tgggtgtttt cccccatcca ccactggcag ggttgcccct tccctccaat catcactgtg     420

ctcctttttt cccggcctac gaggcagctc ctgccactat ctttagagcc aataaagaga     480

attaaaaacc tgtgcaccag gnagcatctt ttaaatacac tagccattct cttgc          535


<210>  35
<211>  377
<212>  DNA
<213>  Homo sapiens

<400>  35
ttgaaagttg tgggtcagct gaccaggtag aggattcaag actcaatgtg gaaaaaatat      60

tttaaactac tgattgaatg ttaatggtca atgctagcac aatattccta tgctgcaata     120

cattaaaata actaagcaag tatatttatt tctagcaaac agatgtttgt tttcaaaata     180

cttctttttc attattggtt ttaaaaaagc attatccttt tatctcacaa ataagtaata     240

tctttcagtt attaaatgat agataatgcc tttttggttt tgtgtggtat tcaactaata     300

catggtttaa agtcacagcc gtttgaatat attttatctt ggtagtacat tttctccctt     360

aggaatatac atagtct                                                    377


<210>  36
<211>  488
<212>  DNA
<213>  Homo sapiens

<400>  36
gatctctgat tcattgtcca ttttactacc aaatattaac taaggcctta ttaattttta      60

tataaattat atcttgtcct attaaatcta gttacaattt atttcatgca taagagctaa 120

tgttattttg caaatgccat atattcaaaa aagctcaaag ataatttctt ttactattat 180

gttcaaataa tattcaatat gcatattatc tttaaaaagt taaatgtttt tttaatcttc 240

aagaaatcat gctacactta acttctccta gaagctaatc tataccataa tattttcata 300

ttcacaagat attaaattac caattttcaa attattgtta gtaaagaaca aaatgattct 360

ctcccaaaga aagacacatt ttaaatactc cttcactcta aaactctggt attataactt 420

ttgaaagtta atatttctac atgaaatgtt tagctcttac actctatcct tcctagaaaa 480

tggtaatt 488

<210> 37
<211> 457
<212> DNA
<213> Homo sapiens

<220>
<221> misc_feature
<222> (292)..(292)
<223> n is a, c, g, or t

<220>
<221> misc_feature
<222> (300)..(307)
<223> n is a, c, g, or t

<400> 37
acttggcacc catgtctacc aaagtttaca attcaaaatg agaaaagaga ggatgtacta 60

aaaataagtg gtccatgtgt tgtgtgcagc tgttgtggag atgttgattt tgagattaaa 120

tctcttgatg aacagtgtgt ggttggcaaa atttccaagc actggactgg aattttgaga 180

gaggcattta cagacgctga taactttgga atccagttcc ctttagacct tgatgttaaa 240

atgaaagctg taatgattgg tgcctgtttc ctcattgact tcatgttttt tngaaagcan 300

nnnnnnngcc aggaacaaaa atcaggagtg tggtagtgga ttagtgaaag tctcctcagg 360

aaatctgaag tctgtatatt gattgagact atctaaactc atacctgtat gaattaagct 420

gtaaggcctg tagctctggt tgtatacttt tgctttt 457

<210> 38
<211> 110
<212> DNA
<213> Homo sapiens

<400> 38
gaaagagcga gagaagggga aagacaagtc gggagaggcc ggtaggcgtg aggcgggcct 60

gaagcggcag cgggcggcct tcgtccggcg agagctaggc cgaggacccg 110

<210>  39
<211>  438
<212>  DNA
<213>  Homo sapiens

<400>  39
aatgggtgcc ggagattcga tgccactgtc ccaaagcccc catcatccta gttggaacgc      60

agtcggatct cagagaagat gtcaaagtcc tcattgagtt ggacaaatgc aaagaaaagc     120

cagtgcctga agaggcggct aagctgtgcg ccgaggaaat caaagccgcc tcctacatcg     180

agtgttcagc cttgactcaa aaaaacctca aagaggtctt tgatgcagcc atcgtcgctg     240

gcattcaata ctcggacact cagcaacagc caaagaagtc taaaagcagg actccagata     300

aaatgaaaaa cctctccaag tcctggtgga agaagtactg ctgtttcgta tgatgctggc     360

aagacaccca gaaaggctat tttcagatga aatcgatatt agaagctata ttagctgaaa     420

caactccttt tactgcgt                                                   438


<210>  40
<211>  477
<212>  DNA
<213>  Homo sapiens


<220>
<221>  misc_feature
<222>  (30)..(30)
<223>  n is a, c, g, or t

<220>
<221>  misc_feature
<222>  (33)..(33)
<223>  n is a, c, g, or t

<400>  40
tactgtactg tttcaatgtg ttaagtgccn tgntgtaaag taaaatttta agtctagatt      60

cattattttc ctgacatata tttttcatta tgatatctac tgtatgctat tgtgatagtt     120

ttatgaaatg cttacatttt aatcaaatat gtaaatttca gaagctcttt tttcctaccc     180

accagtacct taatcattgg tttatcacat tggattcaaa ttcaggttcc ttttttgata     240

aagaggaaat ttgttttcca tgcttgtgat tttgtgtttg taaacatttc aggaggaatt     300

taggagtgta actatagttt atacttctat aatgttgtct tacatttaca tttttaagtg     360

cctaattgtt aaaactgatt tatgtttctc ttctaaagag gatatgtgta cttggtttct     420

aatctctttg gttttgcttt ttaaaaaatg caagagccta tactttgtat ttaccta        477


<210>  41
<211>  405
<212>  DNA
<213>  Homo sapiens

<220>
<221>  misc_feature
<222>  (362)..(362)
<223>  n is a, c, g, or t

<400>  41
gtggagaact ggcagtgacc tctactgggg gccatggcag ggaggggagc cttctggaag      60

ggctgccttg gagattggaa tggggactcc cagggagacc tgcgttccat ccctgcctgc     120

ctcacccctg ccacagactc tgcacaccac tggatggtgg gtccaagcct ggcacagtcc     180

ctgtgcttgt cagagtcatt attatgatta atatcaatta cgatgccaaa aattgctggg     240

caaactttga agacctcaac ttgttacaat gacgatgatg atgattcttg gcggttacac     300

aatccttcct cctggggggg aggcagctag gaggcccagc aggggggctt ctatgctgct     360

gngctcccct agggagttgg ggtagtctgt gccaactcca ggcag                     405


<210>  42
<211>  520
<212>  DNA
<213>  Homo sapiens

<400>  42
aactttgtat agcccatgta cctaccttgt atagaaaaat aattttaaaa atttgaatgg      60

aagggggtaa aggaagtcat gaagtttttt tgcattttta tttaaatgaa ggaattccaa     120

ataactcacc tacagatttt tagcacaaaa atagccattg taaagtgtta aaatttacga     180

taagtattct attggggagg aaaggtaact ctgatctcag ttacagtttt tttttccttt     240

ttaatttcat tattttgggt ttttggtttt tgcagtccta tttatctgca gtcgtattaa     300

gtcctattgc tagaataggt tactacaaaa aaggttatat tctgaaagaa aaataactga     360

cattatatat aaccaattaa tttaaagtat tgccatttaa attacacact gagagcatgt     420

cctatgcaga catagatttt tctgttcatt tatttttctt cattgcagtg gattgatttg     480

ataaatagat gtgttgaatt actacatttg ctgtacatat                           520


<210>  43
<211>  486
<212>  DNA
<213>  Homo sapiens

<400>  43
ttcagcttca tttgtgtcaa tgggcaatga caggtaaatt aagacatgca ctatgaggaa      60

taattattta tttaataaca attgtttggg gttgaaaatt caaaaagtgt ttatttttca     120

tattgtgcca atatgtattg taaacatgtg ttttaattcc aatatgatga ctcccttaaa     180

atagaaataa gtggttattt ctcaacaaag cacagtgtta aatgaaattg taaaacctgt     240

caatgataca gtccctaaag aaaaaaaatc attgctttga agcagttgtg tcagctactg     300

cggaaaagga aggaaactcc tgacagtctt gtgcttttcc tatttgtttt catggtgaaa     360

atgtactgag attttggtat tacactgtat ttgtatctct gaagcatgtt tcatgttttg 420

tgactatata gagatgtttt taaaagtttc aatgtgattc taatgtcttc atttcattgt 480

atgatg 486

<210> 44
<211> 558
<212> DNA
<213> Homo sapiens

<220>
<221> misc_feature
<222> (124)..(124)
<223> n is a, c, g, or t

<220>
<221> misc_feature
<222> (422)..(422)
<223> n is a, c, g, or t

<400> 44
tcatgatgct gactggcgtt agctgattaa cccatgtaaa taggcactta aatagaagca 60

ggaaagggag acaaagactg gcttctggac ttcctccctg atccccaccc ttactcatca 120

cctngcagtg gccagaatta gggaatcaga atcaaaccag tgtaaggcag tgctggctgc 180

cattgcctgg tcacattgaa attggtggct tcattctaga tgtagcttgt gcagatgtag 240

caggaaaata ggaaaaccta ccatctcagt gagcaccagc tgcctcccaa aggaggggca 300

gccgtgctta tatttttatg gttacaatgg cacaaaatta ttatcaacct aactaaaaca 360

ttccttttct cttttttcct gaattatcat ggagttttct aattctctct tttggaatgt 420

angatttttt ttaaatgctt tacgatgtaa aatatttatt ttttacttat tctggaagat 480

ctggctgaag gattattcat ggaacaggaa gaagcgtaaa gactatccat gtcatctttg 540

ttgagagtct tcgtgact 558

<210> 45
<211> 552
<212> DNA
<213> Homo sapiens

<400> 45
aacttacata caaatattac ctcatttgtt gtgtgactga gtaaagaatt tttggatcaa 60

gcggaaagag tttaagtgtc taacaaactt aaagctactg tagtacctaa aaagtcagtg 120

ttgtacatag cataaaaact ctgcagagaa gtattcccaa taaggaaata gcattgaaat 180

gttaaataca atttctgaaa gttatgtttt ttttctatca tctggtatac cattgcttta 240

tttttataaa ttattttctc attgccattg gaatagaata ttcagattgt gtagatatgc 300

tatttaaata atttatcagg aaatactgcc tgtagagtta gtatttctat ttttatataa 360

tgtttgcaca ctgaattgaa gaattgttgg ttttttcttt tttttgtttt tttttttttt 420

tttttttttt ttgcttttga cctcccattt ttactatttg ccaatacctt tttctaggaa 480

tgtgcttttt tttgtacaca tttttatcca ttttacattc taaagcagtg taagttgtat 540

attactgttt ct 552

<210> 46
<211> 379
<212> DNA
<213> Homo sapiens

<400> 46
gttttcactg cttgtatgat gtttcccatt catacaccta taaatctcta acaagaggcc 60

ctttgaactg ccttgtgttc tgtgagaaac aaatatttac ttagagtgga aggactgatt 120

gagaatgttc caatccaaat gaatgcatca caacttacaa tgctgctcat tgttgtgagt 180

actatgagat tcaaattttt ctaacatatg gaaagccttt tgtcctccaa agatgagtac 240

tagggatcat gtgtttaaaa aaagaaaggc tacgatgact gggcaagaag aaagatggga 300

aactgaataa agcagttgat cagcatcatt ggaacatggg gacgagtgac ggcaggagga 360

ccacgaggaa ataccctca 379

<210> 47
<211> 519
<212> DNA
<213> Homo sapiens

<400> 47
ttggctcttg gtggaattga attcatcttg tgtcttattc aagtaataaa tggagtgctt 60

ggaggcatat gtggcttttg ctgctctcac caacagcaat atgactgcta aaagaaccaa 120

cccaggacag agccacaatc ttcctctatt tcattgtaat ttatatattt cacttgtatt 180

catttgtaaa actttgtatt agtgtaacat actccccaca gtctactttt acaaacgcct 240

gtaaagactg gcatcttcac aggatgtcag tgtttaaatt tagtaaactt cttttttgtt 300

tgtttatttg tgtaacatac tccccacagt ctacttttac aaacgcctgt aaagactggc 360

atcttcacag gatgtcagtg tttaaattta gtaaacttct tttttgtttg tttatttgtt 420

tttgtttttt tttaaggaat gaggaaacaa accaccctct gggggtagtt tacagactga 480

gtgacagtac tcagtatatc tgagataaac tctataatg 519

<210> 48
<211> 178
<212> DNA
<213> Homo sapiens

<220>

<221>  misc_feature
<222>  (119)..(119)
<223>  n is a, c, g, or t

<220>
<221>  misc_feature
<222>  (124)..(124)
<223>  n is a, c, g, or t

<400>  48
tatgactgct aaaagaacca acccaggaca gagccacaat cttcctctat ttcattgtaa      60

tttatatatt tcacttgtat tcatttgtaa aactttgtat tagtgtaaca tactccccnc     120

agtntacttt tacaaacgcc tgtaaagact ggcatcttca caggatgtca gtgtttaa       178


<210>  49
<211>  496
<212>  DNA
<213>  Homo sapiens

<400>  49
ctttctgcct gtactggatc tgttattttc agggaaacag gccccagggc ccccctgagc      60

ctcaccctaa gcccttaggc ctctgagagt gctgttgggt tctatttatt tatttatttg     120

ttcctttgtt ccctacccgt gcccccagtg tcttccctgc tgagtaccag gagaggtcct     180

gccccatcct ctctctgaag ccagggccct tccattccat ttagcctttg gatcatcctg     240

gctgggagaa gtgggaccga gccacccagc cccactatcc ccaagcagcc ctacagccgg     300

gatgggaggc acgtggcctc tcttttatcc gtctatttat tttgtaagtg tattcgtgtg     360

gaggaggttg ttgctttatt tttttaaggc tctggagtgt tgtgtatggt ttcttttcac     420

atcccagcct cccatgggca cttctaagaa gagaggggat ttcttggaaa aggagagagg     480

aatcccctag agcagg                                                     496


<210>  50
<211>  527
<212>  DNA
<213>  Homo sapiens


<220>
<221>  misc_feature
<222>  (446)..(446)
<223>  n is a, c, g, or t

<400>  50
gagtggacgt tgcacatcac tgtgaaacct tgcagaggag gagagggcag gttcatcaga      60

agaaagaaag gacaaaatga ctccttatga agcattttgt gccttctgtg agaaaacatg     120

tatttagatc agataaactc tagtcaaaat acaaaaagga aaaatgaaag acctctgaaa     180

taggaacaat ctcttgaaga ggcaaatgac tcaaaactgc tcagtggctc tttcagaaaa     240

tctaagtaaa gttccctgac aacagaaact gaagagattg cctggttcat cttgtagtct     300

tccaaaacag cagataattt ctgaatctca gatgttgaat cagtacaacg ggatggattt 360

cttgttccta agtgttaaat gatcacatac ataaaagagt ctgagcctga gcaacatagt 420

agagaccctg tctctacaaa aaaaanaaaa aattagccag gcgtggtggt gcacactgat 480

agtcccagct actcaggagg ctgagggagg cggatcaatt gagctag 527

<210> 51
<211> 356
<212> DNA
<213> Homo sapiens

<220>
<221> misc_feature
<222> (58)..(58)
<223> n is a, c, g, or t

<220>
<221> misc_feature
<222> (71)..(71)
<223> n is a, c, g, or t

<220>
<221> misc_feature
<222> (92)..(92)
<223> n is a, c, g, or t

<220>
<221> misc_feature
<222> (285)..(285)
<223> n is a, c, g, or t

<400> 51
ccaggagccc tggctagggt agtgatgaca tgcacccaaa cacccctaaa cacaggtngc 60

ctcgctttga ngggaccttg taaagaaaat angccccagg ccccagtggt gggaggcagg 120

ttgtctagaa cacagccttg cctcaaaaag aaaaaaataa agaaaagaaa atctgaaagg 180

gaaagcaggc atcaacttcc agggatctaa taacttccta acttaaagca acagaactta 240

cctaactttt ggacttttca ttttaaaact ttgcctctag cagtngacac tgtcctcaac 300

acagctacag tttatgtaac acagagtcca tattatatgg ggcatgtctg tatcaa 356

<210> 52
<211> 499
<212> DNA
<213> Homo sapiens

<400> 52
gagacctgga gatctaattc tgtttagaca ttttttgtcc cagaaataca gaagcttgaa 60

atgctatgaa ggcagagctt ctattcttta tgggatgaaa tatttcaaaa gaggataaat 120

cctctgtggt aagccatttg gaaaatccta ccaagaattg gcttatttaa ttttccagaa 180

ccaggaatga gtatctaata gcttttgtag aaccttccag aatatgtggg gaaaaagggc 240

tattgctaag tgagctttat ctaatatcct cctaagagtt ttactagtgc ttttttgagg 300

aattacaggg aagctcctgg aattgtacat ggatatcttt atccctaggg ggaaatcaag 360

gagctgggca cccctaattc tttatggaag tgtttaaaac tattttaatt ttattacaag 420

tattactaga gtagtggttc tactctaaga tttcaaaagt gcatttaaaa tcatacatgt 480

tcccgcctgc aaatatatt 499

<210> 53
<211> 464
<212> DNA
<213> Homo sapiens

<220>
<221> misc_feature
<222> (117)..(117)
<223> n is a, c, g, or t

<400> 53
gaaaaacacc atgcatctga tgagataatt cataaatgtt gccccttttt taaatgatac 60

aaccctaaaa gtgactgaat tgcccaagtg cttgaacatg gcagaggtag ttactcntat 120

tttgcagttt gtgcacttaa aaattcctac agtgattgtt actttactgg ggaaaaaaga 180

tgaggtgaaa cttcctccca aggaattaaa atatctgtag aagccatggc ttgcttttat 240

aatgtggaaa tcatttgatt tgctgtaatt cacgcagatc cctccttttg tcagggggaa 300

atgatttgca tcatgttctt ttttcataat gcttttactt cctgtttgga tcagttgtat 360

gtaaatgtac atttttgtta ctttggctgt gcccgttaga atttatcttc cataaagtat 420

ttctcccatt gagtctaatg atgtatactt tgcctaggtc tttc 464

<210> 54
<211> 443
<212> DNA
<213> Homo sapiens

<400> 54
ctttgctatg acttctggcc agatgtggaa ccatatccgt ggacctccat atgctcataa 60

gaacccacac aatggacaag tgagctacat tcatgggagc agccaggctc agtttgtggc 120

agaatcacac attattctgg tactgaatgc cgctatcacc atggggatgg ttcttctaaa 180

tgaagcagca acttcgaaag gcgatgttgg aaaaagacgg ataatttgcc tagtgggatt 240

gggcctggtg gtcttcttct tcagttttct actttcaata tttcgttcca agtaccacgg 300

ctatccttat agctttttaa ttaaatgaag ccaagtggga tttgcataaa gtgaatgttt 360

accatgaaga taaactgttc ctgactttat actattttga attcattcat ttcattgtga 420

tcagctagct tattcttgtg tac 443

<210>  55
<211>  540
<212>  DNA
<213>  Homo sapiens

<400>  55
tgagaatgga cgctgcatcc aggccaacta ctcactaatg gaaaacggaa agatcaaagt      60

gttaaaccag gagttgagag ctgatggaac tgtgaatcaa atcgaaggtg aagccacccc     120

agttaacctc acagagcctg ccaagctgga agttaagttt tcctggttta tgccatcggc     180

accgtactgg atcctggcca ccgactatga gaactatgcc ctcgtgtatt cctgtacctg     240

catcatccaa ctttttcacg tggattttgc ttggatcttg gcaagaaacc ctaatctccc     300

tccagaaaca gtggactctc taaaaaatat cctgacttct aataacattg atgtcaagaa     360

aatgacggtc acagaccagg tgaactgccc caagctctcg taaccaggtt ctacagggag     420

gctgcaccca ctccatgtta cttctgcttc gctttcccct accccacccc cccccataaa     480

gacaaaccaa tcaaccacga caaaggaagt tgacctaaac atgtaaccat gccctaccct     540


<210>  56
<211>  566
<212>  DNA
<213>  Homo sapiens

<400>  56
tctatacctc ctggcagatt ccacaaaagt tcatagttga ctattctgaa accagccccc      60

agtgccccaa gccaggtgtc atcctcctaa ccaagagagg ccggcagatc tgtgctgacc     120

ccaataagaa gtgggtccag aaatacatca gcgacctgaa gctgaatgcc tgaggggcct     180

ggaagctgcg agggcccagt gaacttggtg ggcccaggag ggaacaggag cctgagccag     240

ggcaatggcc ctgccaccct ggaggccacc tcttctaaga gtcccatctg ctatgcccag     300

ccacattaac taactttaat cttagtttat gcatcatatt tcattttgaa attgatttct     360

attgttgagc tgcattatga aattagtatt ttctctgaca tctcatgaca ttgtctttat     420

catcctttcc cctttccctt caactcttcg tacattcaat gcatggatca atcagtgtga     480

ttagctttct cagcagacat tgtgccatat gtatcaaatg acaaatcttt attgaatggt     540

tttgctcagc accacctttt aatata                                          566


<210>  57
<211>  519
<212>  DNA
<213>  Homo sapiens


<220>
<221>  misc_feature
<222>  (362)..(389)
<223>  n is a, c, g, or t

<400>  57
atagttgact attctgaaac cagcccccag tgccccaagc caggtgtcat cctcctaacc      60

aagagaggcc ggcagatctg tgctgacccc aataagaagt gggtccagaa atacatcagc     120

gacctgaagc tgaatgcctg aggggcctgg aagctgcgag ggcccagtga acttggtggg     180

cccaggaggg aacaggagcc tgagccaggg caatggccct gccaccctgg aggccacctc     240

ttctaagagt cccatctgct atgcccagcc acattaacta actttaatct tagtttatgc     300

atcatatttc attttgaaat tgatttctat tgttgagctg cattatgaaa ttagtatttt     360

cnnnnnnnnn nnnnnnnnnn nnnnnnnnna tcctttcccc tttcccttca actcttcgta     420

cattcaatgc atggatcaat cagtgtgatt agctttctca gcagacattg tgccatatgt     480

atcaaatgac aaatctttat tgaatggttt tgctcagca                            519


<210>  58
<211>  475
<212>  DNA
<213>  Homo sapiens

<400>  58
ctcgctcagc cagatgcaat caatgcccca gtcacctgct gctataactt caccaatagg      60

aagatctcag tgcagaggct cgcgagctat agaagaatca ccagcagcaa gtgtcccaaa     120

gaagctgtga tcttcaagac cattgtggcc aaggagatct gtgctgaccc caagcagaag     180

tgggttcagg attccatgga ccacctggac aagcaaaccc aaactccgaa gacttgaaca     240

ctcactccac aacccaagaa tctgcagcta acttattttc ccctagcttt ccccagacat     300

cctgttttat tttattataa tgaattttgt ttgttgatgt gaaacattat gccttaagta     360

atgttaattc ttatttaagt tattgatgtt ttaagtttat ctttcatggt actagtgttt     420

tttagataca gagacttggg gaaattgctt ttcctcttga accacagttc taccc          475


<210>  59
<211>  361
<212>  DNA
<213>  Homo sapiens

<400>  59
gcacgccatc aatgccatcg tgcgcagccc gcgtggcggg gcccggaggc acgcagagct      60

gtccttcgtg ttcctcacgg acggcgtcac gggcaacgac agtctgcacg agtcggcgca     120

ctccatgcgc aacgagaacg tggtacccac cgtcctggcc ttgggcagcg acgtggacat     180

ggacgtgctc accacgctca gcctgggtga ccgcgccgcc gtgttccacg agaaggacta     240

tgacagcctg gcgcaacccg gcttcttcga ccgcttcatc cgctggatct gctagcgccg     300

ccgcccgggc cccgcagtcg agggtcgtga gcccaccccg tccatggtgc taagcgggcc     360

c                                                                     361

<210>  60
<211>  287
<212>  DNA
<213>  Homo sapiens

<400>  60
ccctgggagc atgtgaatgc catccaggag gcccggcgtc tcctgaacct gagtagagac      60

actgctgctg agatgaatga aacagtagaa gtcatctcag aaatgtttga cctccaggag     120

ccgacctgcc tacagacccg cctggagctg tacaagcagg gcctgcgggg cagcctcacc     180

aagctcaagg gccccttgac catgatggcc agccactaca aacagcactg ccctccaacc     240

ccggaaactt cctgtgcaac ccagattatc acctttgaaa gtttcaa                   287


<210>  61
<211>  544
<212>  DNA
<213>  Homo sapiens

<400>  61
taactctacc ctggcactat aatgtaagct ctactgaggt gctatgttct tagtggatgt      60

tctgaccctg cttcaaatat ttccctcacc tttcccatct tccaagggta ctaaggaatc     120

tttctgcttt ggggtttatc agaattctca gaatctcaaa taactaaaag gtatgcaatc     180

aaatctgctt tttaaagaat gctctttact tcatggactt ccactgccat cctcccaagg     240

ggcccaaatt ctttcagtgg ctacctacat acaattccaa acacatacag gaaggtagaa     300

atatctgaaa atgtatgtgt aagtattctt atttaatgaa agactgtaca aagtataagt     360

cttagatgta tatatttcct atattgtttt cagtgtacat ggaataacat gtaattaagt     420

actatgtatc aatgagtaac aggaaaattt taaaaataca gatagatata tgctctgcat     480

gttacataag ataaatgtgc tgaatggttt tcaaataaaa atgaggtact ctcctggaaa     540

tatt                                                                  544


<210>  62
<211>  399
<212>  DNA
<213>  Homo sapiens

<400>  62
ttctttcccc aaatatcatg tagcacatca atatgtaggg aaacattctt atgcatcatt      60

tggtttgttt tataaccaat tcattaaatg taattcataa aatgtactat gaaaaaaatt     120

atacgctatg ggatactggc aacagtgcac atatttcata accaaattag cagcaccggt     180

cttaatttga tgtttttcaa cttttattca ttgagatgtt ttgaagcaat taggatatgt     240

gtgtttactg tactttttgt tttgatccgt ttgtataaat gatagcaata tcttggacac     300

atttgaaata caaaatgttt ttgtctacca aagaaaaatg ttgaaaaata agcaaatgta     360

tacctagcaa tcacttttac tttttgtaat tctgtctct 399

<210> 63
<211> 416
<212> DNA
<213> Homo sapiens

<400> 63
gagacttttc tatggttttg tgactttcaa cttttgtaca gttatgtgaa ggatgaaagg 60

tgggtgaaag gaccaaaaac agaaatacag tcttcctgaa tgaatgacaa tcagaattcc 120

actgcccaaa ggagtccaac aattaaatgg atttctagga aaagctacct taagaaaggc 180

tggttaccat cggagtttac aaagtgcttt cacgttctta cttgttgtat tatacattca 240

tgcatttcta ggctagagaa ccttctagat ttgatgctta caactattct gttgtgacta 300

tgagaacatt tctgtctcta gaagttatct gtctgtattg atctttatgc tatattacta 360

tctgtggtta cagtggagac attgacatta ttactggagt caagccctta taagtc 416

<210> 64
<211> 367
<212> DNA
<213> Homo sapiens

<400> 64
gattatcaat taccacacca tctcccatga agaaagggaa cggtgaagta ctaagcgcta 60

gaggaagcag ccaagtcggt tagtggaagc atgattggtg cccagttagc ctctgcagga 120

tgtggaaacc tccttccagg ggaggttcag tgaattgtgt aggagaggtt gtctgtggcc 180

agaatttaaa cctatactca ctttcccaaa ttgaatcact gctcacactg ctgatgattt 240

agagtgctgt ccggtggaga tcccacccga acgtcttatc taatcatgaa actccctagt 300

tccttcatgt aacttccctg aaaaatctaa gtgtttcata aatttgagag tctgtgaccc 360

acttacc 367

<210> 65
<211> 515
<212> DNA
<213> Homo sapiens

<220>
<221> misc_feature
<222> (149)..(150)
<223> n is a, c, g, or t

<220>
<221> misc_feature
<222> (436)..(436)
<223> n is a, c, g, or t

<400> 65

gggggactgc tttcaaatca gccaaagagc ctgtcagatc ttcttagaag gaagaggttg 60

gtagttccct gctctgtttt gaacatgctc tagtttatta acctggggac attcccattg 120

ctgtcttaag taagtctcat agccagctnn tgtcacgtga ctctcatatg gattcatttt 180

cgggccagct ctgaacaaag catcatgaac atatgtgctt ttggtcgttt gcaatgtgat 240

ggtggtggag gtaggtattg gtttccttgg aaggcatgat aagaaagatt cacaatggcc 300

aacagtgtgt atgaacaaaa aactgattgg agcatcagct agtactgaag gtccttgctt 360

tgtgtcagag gcaaaggaac ccaaggcgcc aagtcctcag ccttgagtgt actgctgaca 420

actaaactca caggcngcaa agcagacctc tgatgaagat gcctgttatt tcacatcact 480

gtctttttgt gtatcatagt ctgcacctta caaat 515

<210> 66
<211> 494
<212> DNA
<213> Homo sapiens

<400> 66
tacacggccc tgatgtcggc taagtactcc ggctggtacg tgggcttcac caagaagggg 60

cggccgcgga agggccccaa gacccgggag aaccagcagg acgtgcattt catgaagcgc 120

taccccaagg ggcagccgga gcttcagaag cccttcaagt acacgacggt gaccaagagg 180

tcccgtcgga tccggcccac acaccctgcc taggccaccc cgccgcggcc cctcaggtcg 240

ccctggccac actcacactc ccagaaaact gcatcagagg aatattttta catgaaaaat 300

aaggaagaag ctctattttt gtacattgtg tttaaaagaa gacaaaaact gaaccaaaac 360

tcttgggggg aggggtgata aggattttat tgttgacttg aaacccccga tgacaaaaga 420

ctcacgcaaa gggactgtag tcaacccaca ggtgcttgtc tctctctagg aacagacaac 480

tctaaactcg tccc 494

<210> 67
<211> 497
<212> DNA
<213> Homo sapiens

<400> 67
tacacggccc tgatgtcggc taagtactcc ggctggtacg tgggcttcac caagaagggg 60

cggccgcgga agggccccaa gacccgggag aaccagcagg acgtgcattt catgaagcgc 120

taccccaagg ggcagccgga gcttcagaag cccttcaagt acacgacggt gaccaagagg 180

tcccgtcgga tccggcccac acaccctgcc taggccaccc cgccgcggcc cctcaggtcg 240

ccctggccac actcacactc ccagaaaact gcatcagagg aatattttta catgaaaaat 300

aaggaagaag ctctattttt gtacattgtg tttaaaagaa gacaaaaact gaaccaaaac 360

tcttgggggg aggggtgata aggattttat tgttgacttg aaacccccga tgacaaaaga 420

ctcacgcaaa gggactgtag tcaacccaca ggtgcttgtc tctctctagg aacagacaac 480

tttaaactcg tccccag 497

<210> 68
<211> 521
<212> DNA
<213> Homo sapiens

<400> 68
atatcttctt caggctctga caggcctcct ggaaacttcc acatattttt caactgcagt 60

ataaagtcag aaaataaagt taacataact ttcactaaca cacacatatg tagatttcac 120

aaaatccacc tataattggt caaagtggtt gagaatatat tttttagtaa ttgcatgcaa 180

aatttttcta gcttccatcc tttctccctc gtttcttctt tttttggggg agctggtaac 240

tgatgaaatc ttttcccacc ttttctcttc aggaaatata agtggttttg tttggttaac 300

gtgatacatt ctgtatgaat gaaacattgg agggaaacat ctactgaatt tctgtaattt 360

aaaatatttt gctgctagtt aactatgaac agatagaaga atcttacaga tgctgctata 420

aataagtaga aaatataaat ttcatcacta aaatatgcta ttttaaaatc tatttcctat 480

attgtatttc taatcagatg tattactctt attatttcta t 521

<210> 69
<211> 464
<212> DNA
<213> Homo sapiens

<400> 69
atgaactttg acatccctga ggagattaag cagctgcagc agttccagaa ggaggacgcc 60

gcattgacca tctatgagat gctccagaac atctttgcta ttttcagaca agattcatct 120

agcactggct ggaatgagac tattgttgag aacctcctgg ctaatgtcta tcatcagata 180

aaccatctga agacagtcct ggaagaaaaa ctggagaaag aagattttac caggggaaaa 240

ctcatgagca gtctgcacct gaaaagatat tatgggagga ttctgcatta cctgaaggcc 300

aaggagtaca gtcactgtgc ctggaccata gtcagagtgg aaatcctaag gaacttttac 360

ttcattaaca gacttacagg ttacctccga aactgaagat ctcctagcct gtccctctgg 420

gactggacaa ttgcttcaag cattcttcaa ccagcagatg ctgt 464

<210> 70
<211> 464
<212> DNA
<213> Homo sapiens

<400> 70
accaggatgc tcacatttaa gttttacatg cccaagaagg ccacagaact gaaacagctt 60

cagtgtctag aagaagaact caaacctctg gaggaagtgc tgaatttagc tcaaagcaaa 120

aactttcact taagacccag ggacttaatc agcaatatca acgtaatagt tctggaacta 180

aagggatctg aaacaacatt catgtgtgaa tatgcagatg agacagcaac cattgtagaa 240

tttctgaaca gatggattac cttttgtcaa agcatcatct caacactaac ttgataatta 300

agtgcttccc acttaaaaca tatcaggcct tctatttatt tatttaaata tttaaatttt 360

atatttattg ttgaatgtat ggttgctacc tattgtaact attattctta atcttaaaac 420

tataaatatg gatcttttat gattcttttt gtaagcccta gggg 464

<210> 71
<211> 216
<212> DNA
<213> Homo sapiens

<400> 71
gtgtgaaggt gcagttttgc caaggagtgc taaagaactt agatgtcagt gcataaagac 60

atactccaaa cctttccacc ccaaatttat caaagaactg agagtgattg agagtggacc 120

acactgcgcc aacacagaaa ttattgtaaa gctttctgat ggaagagagc tctgtctgga 180

ccccaaggaa aactgggtgc agagggttgt ggagaa 216

<210> 72
<211> 441
<212> DNA
<213> Homo sapiens

<400> 72
aaaggagcag tcgcacagac ctttcctcat gctgcaggcc cggcagtctg aagaccaccc 60

tcatcgccgg cgtcggcggg gcttggagtg tgatggcaag gtcaacatct gctgtaagaa 120

acagttcttt gtcagtttca aggacatcgg ctggaatgac tggatcattg ctccctctgg 180

ctatcatgcc aactactgcg agggtgagtg cccgagccat atagcaggca cgtccgggtc 240

ctcactgtcc ttccactcaa cagtcatcaa ccactaccgc atgcggggcc atagcccctt 300

tgccaacctc aaatcgtgct gtgtgcccac caagctgaga cccatgtcca tgttgtacta 360

tgatgatggt caaaacatca tcaaaaagga cattcagaac atgatcgtgg aggagtgtgg 420

gtgctcatag agttgcccag c 441

<210> 73
<211> 425
<212> DNA
<213> Homo sapiens

<400> 73
ctccgcggtc gccaaaaaga aagataaggt gaagaagggc ggcccgggga gcgagtgcgc 60

tgagtgggcc tgggggccct gcacccccag cagcaaggat tgcggcgtgg gtttccgcga 120

gggcacctgc ggggcccaga cccagcgcat ccggtgcagg gtgccctgca actggaagaa 180

ggagtttgga gccgactgca agtacaagtt tgagaactgg ggtgcgtgtg atgggggcac 240

aggcaccaaa gtccgccaag gcaccctgaa gaaggcgcgc tacaatgctc agtgccagga 300

gaccatccgc gtcaccaagc cctgcacccc caagaccaaa gcaaaggcca aagccaagaa 360

agggaaggga aaggactaga cgccaagcct ggatgccaag gagcccctgg tgtcacatgg 420

ggcct 425

<210> 74
<211> 510
<212> DNA
<213> Homo sapiens

<400> 74
agatgatgga ccctggttat cccaaactga ttaccaagaa cttccaagga atcgggccta 60

aaattgatgc agtcttctat tctaaaaaca aatactacta tttcttccaa ggatctaacc 120

aatttgaata tgacttccta ctccaacgta tcaccaaaac actgaaaagc aatagctggt 180

ttggttgtta gaaatggtgt aattaatggt ttttgttagt tcacttcagc ttaataagta 240

tttattgcat atttgctatg tcctcagtgt accactactt agagatatgt atcataaaaa 300

taaaatctgt aaaccatagg taatgattat ataaaataca taatattttt caattttgaa 360

aactctaatt gtccattctt gcttgactct actattaagt ttgaaaatag ttaccttcaa 420

agcaagataa ttctatttga agcatgctct gtaagttgct tcctaacatc cttggactga 480

gaaattatac ttacttctgg cataactaaa 510

<210> 75
<211> 360
<212> DNA
<213> Homo sapiens

<220>
<221> misc_feature
<222> (99)..(99)
<223> n is a, c, g, or t

<220>
<221> misc_feature
<222> (101)..(102)
<223> n is a, c, g, or t

<220>
<221> misc_feature
<222> (146)..(146)
<223> n is a, c, g, or t

<220>
<221> misc_feature
<222> (153)..(153)
<223> n is a, c, g, or t

<220>
<221> misc_feature
<222> (175)..(175)
<223> n is a, c, g, or t

<220>
<221> misc_feature
<222> (187)..(187)
<223> n is a, c, g, or t

<220>
<221> misc_feature
<222> (219)..(219)
<223> n is a, c, g, or t

<400> 75
ctggcacata ggcactgaaa cactgcatgt taatccacac cccaccccac ctatgagtgt 60

agtcaaagct ggtaagtgac aagggctttc gtggaaacnt nncctgacct aatgttgggc 120

atcaggttac ccaaagagct tcaggnaaat gangaaagga cttgcaggtc ttgantgaga 180

atggagnggt aactgccaat gagggctttg gctttagcng aaagtctgaa agggaagcca 240

taggaactta aacgtaccga ctataaagct ctgagaaaag ctgatgtttt agaaagacca 300

tacattctag gtacaaatac ctaaaaacta aaaaataagt acgttggcca ggcgggcgga 360


<210> 76
<211> 436
<212> DNA
<213> Homo sapiens

<400> 76
tagtgtattc acttacccta gttcattata aaaaatatct aggcattgtg gatataaaac 60

tgttgggtat tctacaactt caatggaaat tattacaagc agattaatcc ctctttttgt 120

gacacaagta caatctaaaa gttatattgg aaaacatgga aatattaaaa ttttacactt 180

ttactagcta aaacataatc acaaagcttt atcgtgttgt ataaaaaaat taacaatata 240

atggcaatag gtagagatac aacaaatgaa tataacacta taacacttca tattttccaa 300

atcttaattt ggatttaagg aagaaatcaa taaatataaa atataagcac atatttatta 360

tatatctaag gtatacaaat ctgtctacat gaagtttaca gattggtaaa tatcacctgc 420

tcaacatgta attatt 436


<210> 77
<211> 497
<212> DNA
<213> Homo sapiens

<400> 77
cctgtcctcc agcaccatag agaggcgcta gagcccattc ctctttctcc accgtcaccc 60

aacatcaatc ctttaccact ctaccaaata atttcatatt caagcttcag aagctagtga 120

ccatcttcat aatttgctgg agaagtgtat ttcttcccct tactctcaca cctgggcaaa 180

ctttcttcag tgtttttcat ttcttacgtt ctttcacttc aagggagaat atagaagcat 240

ttgatattat ctacaaacac tgcagaacag catcatgtca taaacgattc tgagccattc 300

acacttttta tttaattaaa tgtatttaat taaatctcaa atttatttta atgtaaagaa 360

cttaaattat gttttaaaca catgccttaa atttgtttaa ttaaatttaa ctctggtttc 420

taccagctca tacaaaataa atggtttctg aaaatgttta agtattaact tacaaggata 480

taggtttttc tcatgta 497

<210> 78
<211> 445
<212> DNA
<213> Homo sapiens

<400> 78
gagctcgatt cacggaggca ttgaaatttt cagcagagac cttccaagga catattgcag 60

gattctgtaa tagtgaacat atggaaagta ttagaaatat ttattgtctg taaatactgt 120

aaatgcattg gaataaaact gtctccccca ttgctctatg aaactgcaca ttggtcattg 180

tgaatatttt tttttttgcc aaggctaatc caattattat tatcacattt accataattt 240

attttgtcca ttgatgtatt tattttgtaa atgtatcttg gtgctgctga atttctatat 300

tttttgtaac ataatgcact ttagatatac atatcaagta tgttgataaa tgacacaatg 360

aagtgtctct attttgtggt tgattttaat gaatgcctaa atataattat ccaaattgat 420

tttcctttgt gcatgtaaaa ataac 445

<210> 79
<211> 252
<212> DNA
<213> Homo sapiens

<400> 79
aaaacttcac atttatgtgg cttgtttatc cttagctcac agattgaggt aataatgaca 60

ctcctagact ttgggatcaa ataacttagg gccaagtctt gggtctgaat ttatttaagt 120

tcacaaccta gggcaagtta ctctgccttt ctaagactca cttacatctt ctgtgaaata 180

taattgtacc aacctcatag agtttggtgt caactaaatg agattatatg tggactaaat 240

atctgtcata ta 252

<210> 80
<211> 513
<212> DNA
<213> Homo sapiens

<400> 80
gacgaccagc caggatatgc tctcaatcat ggagaaattg gaattcttcg atttttctta 60

tgaccttaac ctgtgtgggc tgacagagga cccagatctt caggtttctg cgatgcagca 120

ccagacagtg ctggaactga cagagactgg ggtggaggcg gctgcagcct ccgccatctc 180

tgtggcccgc accctgctgg tctttgaagt gcagcagccc ttcctcttcg tgctctggga 240

ccagcagcac aagttccctg tcttcatggg gcgagtatat gaccccaggg cctgagacct 300

gcaggatcag gttagggcga gcgctacctc tccagcctca gctctcagtt gcagccctgc 360

tgctgcctgc ctggacttgc ccctgccacc tcctgcctca ggtgtccgct atccaccaaa 420

agggctcctg agggtctggg caagggacct gcttctatta gcccttctcc atggccctgc 480

catgctctcc aaaccacttt ttgcagcttt ctc 513

<210> 81
<211> 416
<212> DNA
<213> Homo sapiens

<400> 81
gttggttcaa acttttggga gcacggactg tcagttctct gggaagtggt cagcgcatcc 60

tgcagggctt ctcctcctct gtcttttgga gaaccagggc tcttctcagg ggctctaggg 120

actgccaggc tgtttcagcc aggaaggcca aaatcaagag tgagatgtag aaagttgtaa 180

aatagaaaaa gtggagttgg tgaatcggtt gttctttcct cacatttgga tgattgtcat 240

aaggttttta gcatgttcct ccttttcttc accctcccct ttgttcttct attaatcaag 300

agaaacttca aagttaatgg gatggtcgga tctcacaggc tgagaactcg ttcacctcca 360

agcatttcat gaaaaagctg cttcttatta atcatacaaa ctctcaccat gatgtg 416

<210> 82
<211> 526
<212> DNA
<213> Homo sapiens

<400> 82
gtccacattc ctgcaagcat tgattgagac atttgcacaa tctaaaatgt aagcaaagta 60

gtcattaaaa atacaccctc tacttgggct ttatactgca tacaaattta ctcatgagcc 120

ttcctttgag gaaggatgtg gatctccaaa taaagattta gtgtttattt tgagctctgc 180

atcttaacaa gatgatctga acacctctcc tttgtatcaa taaatagccc tgttattctg 240

aagtgagagg accaagtata gtaaaatgct gacatctaaa actaaataaa tagaaaacac 300

caggccagaa ctatagtcat actcacacaa agggagaaat ttaaactcga accaagcaaa 360

aggcttcacg gaaatagcat ggaaaaacaa tgcttccagt ggccacttcc taaggaggaa 420

caaccccgtc tgatctcaga attggcacca cgtgagcttg ctaagtgata atatctgttt 480

ctactacgga tttaggcaac aggacctgta cattgtcaca ttgcat 526

<210> 83

<211>  380
<212>  DNA
<213>  Homo sapiens

<400>  83
tactgcttgc agtaattcaa ctggaaatta aaaaaaaaaa actagactcc attgtgcctt      60

actaaatatg ggaatgtcta acttaaatag ctttgagatt tcagctatgc tagaggcttt      120

tattagaaag ccatattttt ttctgtaaaa gttactaata tatctgtaac actattacag      180

tattgctatt tatattcatt cagatataag atttgtacat attatcatcc tataaagaaa      240

cggtatgact taattttaga aagaaaatta tattctgttt attatgacaa atgaaagaga      300

aaatatatat ttttaatgga aagtttgtag catttttcta ataggtactg ccatattttt      360

ctgtgtggag tatttttata                                                  380

<210>  84
<211>  406
<212>  DNA
<213>  Homo sapiens

<400>  84
gataaaacgg caacacagct cacaagaaca gactttccag ctgctgaagt tatggaaaca      60

tcaaaacaaa gcccaagata tagtcaagaa gatcatccaa gatattgacc tctgtgaaaa      120

cagcgtgcag cggcacattg gacatgctaa cctcaccttc gagcagcttc gtagcttgat      180

ggaaagctta ccgggaaaga aagtgggagc agaagacatt gaaaaaacaa taaaggcatg      240

caaacccagt gaccagatcc tgaagctgct cagtttgtgg cgaataaaaa atggcgacca      300

agacaccttg aagggcctaa tgcacgcact aaagcactca aagacgtacc actttcccaa      360

aactgtcact cagagtctaa agaagaccat caggttcctt cacagc                    406

<210>  85
<211>  570
<212>  DNA
<213>  Homo sapiens

<400>  85
   tcctaatgcc taaatcaggg cctaggtagt gagctgtggg cacatattaa gtattggtta      60
   aactaaaaat aataagcaaa atggacatta tctataaaag cttttgtgga aatggctaga      120
   gctagggtaa ggaaacaaat ttggttcccc atacctgccc tccaagaaaa taaagctgtc      180
   aaggaaaatc tgggctaaga gtaggatatg agggatgatg gataaggcat gagacatgag      240
   aaataagggg gattaaatta ttattactat tatacaaatg atgcctgagt agatttttaa      300
   aatgattaaa tacccaatga tgtaaaaaac atttataaaa taggaaagta agactgactc      360
   aaccataatt tgttgagtca acccaaaaat ctatttggtt attttcaaac agaaatagcc      420
   tacagatgat atctgagatt gttccaaact ttttctatga atatgtatac tttttttaca      480
   taattaacat aatactgtat attaatttgt tacctgcttt ttcaattaac aatatatcat 540
   aagcatctat gccaataaac acaattctgc 570

<210> 86
<211> 382
<212> DNA
<213> Homo sapiens

<400> 86
gaagacttac tgttggccat agttaattgt gtgaggaaca cgccggcctt tttagccgaa 60

agactgcatc gagccttgaa gggtattgga actgatgagt ttactctgaa ccgaataatg 120

gtgtccagat cagaaattga ccttttggac attcgaacag agttcaagaa gcattatggc 180

tattccctat attcagcaat taaatcggat acttctggag actatgaaat cacactctta 240

aaaatctgtg gtggagatga ctgaaccaag aagataatct ccaaaggtcc acgatgggct 300

tttccaacag ctccacctta cttcttctca tactatttaa gagaacaagc aaatataaac 360

agcaacttgt gttcctaaca gg 382

<210> 87
<211> 486
<212> DNA
<213> Homo sapiens

<400> 87
   ctggagaagc actgccattc agcctcctgc tccagctgtt cacatgcaga aatgctctct 60
   tcacaggcag agaagcctgt ggctaaagtt tccacatccc attaactcag tgcttttgtc 120
   tttttcatga catggcacat agagaaaata tttttttcta gcacacaaga gcaacctgaa 180
   aggctgctcc tggctagggg actctgtccc gggggaccgt gtcctccccc atgtcctgcc 240
   taggccctca gaggaccagg ggatcatgtc tccaggtaac ccgactgtag cccctgctgg 300
   ctgagctcca gcctgtgccc actgataata gcagggacgg cctttctctt agagcagctg 360
   ataagtttcc ctacctgatg gccccctctg acataaactg cacacctggg gtgatggctt 420
   aaagccagaa agagctgagg gagttaagag ggccaacctt agggcacgtg ggcattatta 480
   aaggtc 486

<210> 88
<211> 374
<212> DNA
<213> Homo sapiens

<220>
<221> misc_feature
<222> (202)..(202)
<223> n is a, c, g, or t

<400> 88

tgccccgcaa gggttctggc agtgactgcc gtctgggcca caatgccttc tcctgggtct 60

tctctcgcta tgatcaggag tttcgtttct cacacaatgg gcagcacgag cccctggggc 120

tgctgcgggg cccagcccag ctgggtgtag tgctggactt gcaggttcag gagctgctct 180

tctatgagcc agcctccggc anagtgctct gtgcccatca tgtgtccttc ccggggcccc 240

tcttcccagt ctttgctgtg gccgatcaga ccatttctat cgtccgctga cctctggcca 300

caggaagcca ggtccaccgc ccaccaccct ttcaggccat gtttctactc agtgtgcttt 360

tcccaaatga tgtg 374

<210> 89
<211> 487
<212> DNA
<213> Homo sapiens

<400> 89
aactcatacg tcctgtggtg gcattgggag agttccccca tgatgagggc caagatagaa 60

tctgtaccac tcagtgctac catccccacc cctacaccac ttccacacag gggcctcatg 120

gcatggtcag ggtcccagct gtaggtgaga gcagggcact gtccagctgt ccactgggga 180

agtcaagatg tcctaaggcc caggtcaggg catctggagt ctgaaggacc ctagttccta 240

gaggcatctg gcagcaagaa ggtgaggcat cagggaacgg gaatcaggct gggactgatc 300

agaggtgaag ggacagagag aggagaggag gaagattgag ctgggggcaa cagccaagct 360

cacctgggca ggtctctgcc acctccttgc tctgtgagct gtcagtctag gttattctct 420

ttttttgtgg ctatttttaa ttgctttgga tttgttaaat gttttctgtc ttctgttaag 480

tgtgttt 487

<210> 90
<211> 380
<212> DNA
<213> Homo sapiens

<400> 90
cctatgcgaa tgacgtgtat ccctgtaccc atggtaaatg taaatatact gtgtctcttt 60

tgggagagcc ttttagtaga ggagtcttat atgagtctct acataagtag tttcacttga 120

gttttgcagt ttgaaatctt aaaggagctt taattgacat ttattatacc aattaagctt 180

ggaatggggc aatggatgca tttcccaaaa cgtgtgaaag cactaacagc ttatattgct 240

gaatgagaat ctcctgggtg taatttagcc acttagggaa ctgcgtgaac actcccaggc 300

cattatgatg ctgttacagc ttcagtgtat aaatgcatga gtattctttc tgttctgttt 360

tgtgctctct tgtacattta 380

<210> 91
<211> 518

<212>  DNA
<213>  Homo sapiens

<400>  91
gaaagcaccc taattgactt ggaaaaaaaa aacagcaaaa gcaaaagtag caacatatgt      60

caacatatgt cactgaaata ggaaacagtc attggaatgt tgcacagagg ctaatagcta     120

tggactgttg gatacaggat acagtggtga gaggagcccc attttaggtc tttcttttag     180

gtttttggtt ttcattactc caagtagccc ttgacccaag aacaaaggct tgttgtatga     240

gttccactgc cagatttatg ggatgcctgg atcattcaga aggatgcttc aactattatt     300

tgtcaggtcc aaaggtcgta cttgataacc ccattttcta tgtatggggt agtctaatat     360

attattttat ctactttatt tttccctttt cagaaagtcc ttagtgcaaa ccaccattgg     420

aatctagtca gaaatgtctg tcagatagtt agaattgtaa catctaaacc tgccacggat     480

cgaatggtac ttacaggtac ctctcttagg gactctgt                             518


<210>  92
<211>  313
<212>  DNA
<213>  Homo sapiens

<400>  92
aggaaagtgg gcgcagagcc ctcctgctca cacagctgct gagacttcag ggacccatca      60

gaacttggtg cagcacagcc ccgcccgtgg agggtccctt ttacgcaccc caaggcccac     120

acctaagctt ccatgtagcc ctcatccagg gaagttttgc gatcctttag gaagacactg     180

tcctcttatt acagattgtg tatttccgta ggcttcttag tagcagcttt gtacactgag     240

gacactgtag ccaggaacct gtgcatgcca cccaccgcct ggacaggcag tcatcctgcc     300

tctgatgtga atc                                                        313


<210>  93
<211>  201
<212>  DNA
<213>  Homo sapiens

<400>  93
tgggagcatg gtgagcagcc ctggtgctca gcagccatac ctatgggaca cacactacga      60

aaaggatgcc tttagggttt gggggagatt ttactccttt cttcaacaac tattcactgg     120

acaagttctc tgctcccatg acgcgccagg cacagttctg caagtatatt gtgaatgtat     180

tgttctagtg ggatacacaa a                                               201


<210>  94
<211>  51
<212>  DNA
<213>  Homo sapiens

<400>  94

tttcactgag gccatctatc cgatttcaag gaagccaagg gcacatctcc a 51

<210> 95
<211> 411
<212> DNA
<213> Homo sapiens

<400> 95
ggtccgcctt ctggatgtgt atgaaaagga agtggtcaag ttctcagcct cacctgaccc 60

ggtccttcag tggactgaga ggggctgccg acaggtctcc cacgtcttca ccaactttgg 120

caagggcatc cgctacgtat cttttgagca gtacgggaga gacgtgagtt cctgggtggg 180

gcactacggc gcccttgtga cccactccag tgtgagggtc aggatccgtc tgtcctagcg 240

actggactac tgcctgacgt tgtcagtcaa gaccagcctt gcagccaggt gcagtggctc 300

acacctgtgg aatcccagca cttcaggagg ccgaggtggg aggatcactt ggagctcggg 360

agttcaagac cagcctgggc aacatagtga aaccgtctcc acaaataatt t 411

<210> 96
<211> 515
<212> DNA
<213> Homo sapiens

<400> 96
tgtctccccg ctatctaaaa aagtataaga acaagcagat aacagcgaga atcttggagg 60

cccatcagaa tgtagctcag atgagtctaa ttgaagccaa gatgagattt attcaagctt 120

ggcagtcact acctgaattt ggcatcactc acttcattgc aaggttccaa gggggcaaaa 180

aagaagaact tattggaatt gcatacaaca gactgattcg gatggatgcc agcactggag 240

atgcaattaa aacatggcgt ttcagcaaca tgaaacagtg gaatgtcaac tgggaaatca 300

aaatggtcac cgtagagttt gcagatgaag tacgattgtc cttcatttgt actgaagtag 360

attgcaaagt ggttcatgaa ttcattggtg gctacatatt tctctcaaca cgtgcaaaag 420

accaaaacga gagtttagat gaagagatgt tctacaaact taccagtggt tgggtgtgaa 480

tagaaatact gtttaatgaa actccacggc cataa 515

<210> 97
<211> 536
<212> DNA
<213> Homo sapiens

<220>
<221> misc_feature
<222> (26)..(26)
<223> n is a, c, g, or t

<220>
<221> misc_feature
<222> (64)..(64)

<223>  n is a, c, g, or t

<220>
<221>  misc_feature
<222>  (124)..(124)
<223>  n is a, c, g, or t

<400>  97
aaaatgctat tagtccgtcg tgcttnattt gtttttgtcc ttgaataagc atgttatgta      60

tatngtctcg tgtttttatt tttacaccat attgtattac acttttagta ttcaccagca      120

taancactgt ctgcctaaaa tatgcaactc tttgcattac aatatgaagt aaagttctat      180

gaagtatgca ttttgtgtaa ctaatgtaaa aacacaaatt ttataaaatt gtacagtttt      240

ttaaaaacta ctcacaacta gcagatggct taaatgtagc aatctctgcg ttaattaaat      300

gcctttaaga gatataatta acgtgcagtt ttaatatcta ctaaattaag aatgacttca      360

ttatgatcat gatttgccac aatgtcctta actctaatgc ctggactggc catgttctag      420

tctgttgcgc tgttacaatc tgtattggtg ctagtcagaa aattcctagc tcacatagcc      480

caaaagggtg cgagggagag gtggattacc agtattgttc aataatccat ggttca          536


<210>  98
<211>  555
<212>  DNA
<213>  Homo sapiens


<220>
<221>  misc_feature
<222>  (143)..(143)
<223>  n is a, c, g, or t

<220>
<221>  misc_feature
<222>  (148)..(148)
<223>  n is a, c, g, or t

<220>
<221>  misc_feature
<222>  (151)..(151)
<223>  n is a, c, g, or t

<220>
<221>  misc_feature
<222>  (155)..(155)
<223>  n is a, c, g, or t

<220>
<221>  misc_feature
<222>  (157)..(158)
<223>  n is a, c, g, or t

<220>
<221>  misc_feature
<222>  (167)..(167)
<223>  n is a, c, g, or t

<220>
<221>  misc_feature
<222>  (173)..(174)
<223>  n is a, c, g, or t

<220>
<221>  misc_feature
<222>  (463)..(466)
<223>  n is a, c, g, or t

<220>
<221>  misc_feature
<222>  (468)..(470)
<223>  n is a, c, g, or t

<220>
<221>  misc_feature
<222>  (472)..(472)
<223>  n is a, c, g, or t

<220>
<221>  misc_feature
<222>  (474)..(474)
<223>  n is a, c, g, or t

<220>
<221>  misc_feature
<222>  (476)..(480)
<223>  n is a, c, g, or t

<220>
<221>  misc_feature
<222>  (482)..(488)
<223>  n is a, c, g, or t

<220>
<221>  misc_feature
<222>  (490)..(491)
<223>  n is a, c, g, or t

<400>  98
caaagccctc actgctggag agaaggccat agcagcggag cgggccatga aggaagcagc      60

tgagaaggaa caggagctgc taagagaaaa acagaaggag cagcagcaaa tgatggaggc     120

tcaagagaga agcttccagg aanacatngc ncaantnnag aagaagntgg agnnggaaag     180

ggaaaacctt ctcagagagc atgaaaggct gctaaaacac aagctgaagg tacaagaaga     240

aatgcttaag gaagaatttc aaaagaaatc tgagcagtta aataaagaga ttaatcaact     300

gaaagaaaaa attgaaagca ctaaaaatga acagttaagg ctcttaaaga tccttgacat     360

ggctagcaac ataatgattg tcactctacc tggggcttcc aagctacttg gagtagggac     420

aaaatatctt ggctcacgta tttaagagcc tgaatattcc agnnnngnnn ananannnnn     480

annnnnnntn nattttaata acataacact gttgctcatt ttgtaagtat aagtgttata     540

gcagtttcat tcaag                                                      555

<210>  99

<211>  509
<212>  DNA
<213>  Homo sapiens

<400>  99
gtcagagctg atcgtgcagg ctgcttcctc ctctaaggag cgtggtggtg tgtcgttggc      60

agctcttaaa aaggcgctgg cggccgcagg ctacgacgtg gagaagaaca acagccgcat      120

taagctgggc attaagagcc tagtaagcaa gggaacgttg gtgcagacaa agggtaccgg      180

agcctcgggt tccttcaagc tcaacaagaa ggcgtcctcc gtggaaacca agcccggcgc      240

ctcaaaggtg gctacaaaaa ctaaggcaac gggtgcatct aaaaagctca aaaaggccac      300

gggggctagc aaaaagagcg tcaagactcc gaaaaaggct aaaaagcctg cggcaacaag      360

gaaatcctcc aagaatccaa aaaaacccaa aactgtaaag cccaagaaag tagctaaaag      420

ccctgctaaa gctaaggctg taaaacccaa ggcggccaag gctagggtga cgaagccaaa      480

gactgccaaa cccaagaaag cggcaccca                                        509


<210>  100
<211>  409
<212>  DNA
<213>  Homo sapiens


<220>
<221>  misc_feature
<222>  (53)..(53)
<223>  n is a, c, g, or t

<400>  100
tccttcttac tctgcagcaa catggaggag agttttgtgt agtgagtgtg ggngaagaaa      60

tacatttggc tgttctcaca ccccctctga ctatgcacca gtgaacacat ctgagtacat      120

accagctctc ctcatcttct tatttatact taacttattt ttgtgtgaaa taaatggagg      180

acgaaatctt agagcaacat catcaaacag tctttggtcc ttgagaatct tctttgtgtt      240

ttattttttg atttctgtag cttttcagtt gcagatgttg aaattcgtaa tgacaaatat      300

gacaaattgt catgggtgat tccacttcat cttatttttt ctactctcac tatacaatct      360

tgcctcattt tttaaaactt tggaaccaga ggatttcaac tgcctagca                  409


<210>  101
<211>  140
<212>  DNA
<213>  Homo sapiens

<400>  101
atcacagctc acgtacttag gagtttagct taagtcgtca tatataaaat acagtctcta      60

atatcatgat tgatggagag cattcagctt gccttagatg ctgtaaattc aggaaaagct      120

gaacattaag gcgtttttgt                                                  140

<210>  102
<211>  523
<212>  DNA
<213>  Homo sapiens

<220>
<221>  misc_feature
<222>  (29)..(29)
<223>  n is a, c, g, or t

<400>  102
atccgacttg aatattcctg gacttacana atgccaaggg ggtgactgga agttgtggat      60

atcagggtat aaattatatc cgtgagttgg gggagggaag accagaattc ccttgaattg     120

tgtattgatg caatataagc ataaaagatc accttgtatt ctctttacct tctaaaagcc     180

attattatga tgttagaaga agaggaagaa attcaggtac agaaaacatg tttaaatagc     240

ctaaatgatg gtgcttggtg agtcttggtt ctaaaggtac caaacaagga agccaaagtt     300

ttcaaactgc tgcatacttt gacaaggaaa atctatattt gtcttccgat caacatttat     360

gacctaagtc aggtaatata cctggtttac ttctttagca tttttatgca gacagtctgt     420

tatgcactgt ggtttcagat gtgcaataat ttgtacaatg gtttattccc aagtatgcct     480

taagcagaac aaatgtgttt ttctatatag ttccttgcct taa                       523

<210>  103
<211>  256
<212>  DNA
<213>  Homo sapiens

<400>  103
gagcaggacg aggagcttga ccacatatta tcccctccac ccatgccgtt tcggaaatgc      60

agcaacccag atgtggcttc tggccctgga aaatcactga agtataaaag acagctgagt     120

gaggatggaa gacagctaag gcgagggagc ctgggaggag ccctgactgg gaggtacctt     180

cttccaaacc cggtggcggg acaggcctgg ccggcctctg cagagacgtc caacctcgtg     240

cgcatgcgca gccagg                                                     256

<210>  104
<211>  232
<212>  DNA
<213>  Homo sapiens

<220>
<221>  misc_feature
<222>  (128)..(128)
<223>  n is a, c, g, or t

<220>
<221>  misc_feature
<222>  (141)..(141)

<223>  n is a, c, g, or t

<220>
<221>  misc_feature
<222>  (148)..(148)
<223>  n is a, c, g, or t

<400>  104
ctgttatctg tggtgagcag gttggtgtca aaggaaatcc ccaaggcttc aaccagggtc      60

tggattgtga tgtgatcgta gctgaggtat gtgcttccca ggcctccaaa gcttccacat     120

ttttgttngg tatcagttat ncatgttngg gtgtattctc atcccagatt tttctcattt     180

agatcataaa cataatagaa agggctagaa ttgcaatctt gtgtaactta aa             232


<210>  105
<211>  228
<212>  DNA
<213>  Homo sapiens


<220>
<221>  misc_feature
<222>  (55)..(55)
<223>  n is a, c, g, or t

<220>
<221>  misc_feature
<222>  (75)..(75)
<223>  n is a, c, g, or t

<220>
<221>  misc_feature
<222>  (87)..(87)
<223>  n is a, c, g, or t

<400>  105
gaaaaaatgg ttgacctcac ccaggtaatg gatgatgaag tattcatggc ttttncatcc      60

tatgcaacaa ttatnctttc aaaaatnatg cttatgagta ctgcaactgc attctataga     120

ttgacaagaa aggtttttgc caatccagaa gactgtgtag catttggcaa aggagaaaat     180

gccaagaagt atcttcgaac agatgacaga gtagaacgtg tacgcaga                  228


<210>  106
<211>  255
<212>  DNA
<213>  Homo sapiens

<400>  106
aagaacctgc tgcggctaca ggacctggtg gacaagctgc aactgaaggt caaggcctac      60

aagcgccagg ccgaggaggc ggaggagcaa gccaacacca acctgtccaa gttccgcaag     120

gtgcagcatg agctggatga ggcagaggag cgggcggaca tcgctgagtc ccaggtcaac     180

aagcttcgag ccaagagccg tgacattggt gccaagcaaa aaatgcacga tgaggagtga     240

cactgcctcg ggaac                                                      255

<210>  107
<211>  120
<212>  DNA
<213>  Homo sapiens

<400>  107
ctgcagaagc tggaagcgcg ggtgcgggag ctggagaatg agctggaggc cgagcagaag      60

cgcaacgcag agtcggtgaa gggcatgagg aagagcgagc ggcgcatcaa ggagctcacc     120


<210>  108
<211>  536
<212>  DNA
<213>  Homo sapiens

<400>  108
gaccatggaa cctctgatcc aagcagccca gctcctgcaa ttaaagaaga aaacccagga      60

ggacgcagag gctatctgct ccctgtgtac ctccctcagc acccagcaga ttgtcaaaat     120

tttaaacctt tatactcccc tgaatgaatt tgaagaacgg gtaacagtgg cctttatacg     180

aacaatccag gcacaactac aagagcggaa tgaccctcag caactgctat tagatgccaa     240

gcacatgttt cctgttttgt ttccatttaa tccatcttct ctaaccatgg actcaatcca     300

catcccagcg tgtctcaatc tggaattcct caatgaagtc tgaagatgca tgtttccagc     360

attagtttga ttcccaatgt gagcaagaag gaagtatata cagtaaagta aattcaagga     420

tctgttaaat ctggtaaaag tagatcaaat cagagattga cagcctgtgg agggtgctga     480

actatacaga attagacaca actatgtcat tattttttgt acctactgct cagaat          536


<210>  109
<211>  484
<212>  DNA
<213>  Homo sapiens

<400>  109
cagaaacttt cccagaagat ggaggagagt atatgtgtaa agcagtcaac aataaaggat      60

ctgcagctag tacctgtatt cttaccattg aaagtaagaa ttaatcactc tttttatctt     120

ttattctatt aatttttttt tccttaaaat cacttttctt cttctctttt ttagctgatg     180

actactagct cccctcccct ctccctggaa ctttctcttt cactccaact ttcttactac     240

atccatcttt tctgtggcgg ggccaaaaaa ggaaaccagg agtgccacta tgctgacttc     300

ttattccttt tcataacagt cttcaaagca cagctcatct aaagaatgcc tacttctttt     360

ccaaataagc atcagattta tcgcctatta tgcagtaaca gtcaataaaa tgtacttatg     420

ggggggaatt actcaattat tctatcagaa cctattataa agactgtatt tcccatagac     480

gttt                                                                  484

<210> 110
<211> 205
<212> DNA
<213> Homo sapiens

<220>
<221> misc_feature
<222> (38)..(38)
<223> n is a, c, g, or t

<400> 110
agatttcctc aagtgtgcat agatctgaga ggagtcgngg agtcgagact cgagattcca 60

tcatagcgta ggtgtgtggg gttgggagcc ccctgatggg cttgtctgtg tttgcacctt 120

gtcctgtgtc tgaggtcctg tgactgtacc ctcctttgcc ctgggacatc tgtatctctt 180

ggctttgtaa taaatgctgc atact 205

<210> 111
<211> 198
<212> DNA
<213> Homo sapiens

<400> 111
catacgctgg acgagtcgga ccgaggctag gacgtggccg gcgctctcca gccctgcagc 60

agaagaactt cccgtgcgcg cggatcctcg ctccgttgca cgggcgcctt aagttattgg 120

actatctaat atctatgtat ttatttcgct ggttctttgt agtcacatat tttatagtct 180

taatatcttg tttttgca 198

<210> 112
<211> 530
<212> DNA
<213> Homo sapiens

<220>
<221> misc_feature
<222> (371)..(371)
<223> n is a, c, g, or t

<400> 112
ggagttcatt gcaccagtta cactgaaggt ctttgcaggc tattatacga agggatttgc 60

actactgaat tttgtagtaa aatactcccc tgaacgacag cgttctcttc gtcctcatca 120

tgatgcttct acatttacca taaacattgc acttaataac gtgggagaag actttcaggg 180

aggtggttgc aaatttctaa ggtacaattg ctctattgag tcaccacgaa aaggctggag 240

cttcatgcat cctgggagac tcacacattt gcatgaagga cttcctgtta aaaatggaac 300

aagatacatt gcagtgtcat ttatagatcc ctaagttatt tacttttcat tgaattgaaa 360

tttattttgg ntgaatgact ggcatgaaca cgtctttgaa gttgtggctg agaagatgag 420

aggaatattt aaataacatc aacagaacaa cttcactttg ggccaaacat ttgaaaaact 480

ttttataaaa aattgtttga tatttcttaa tgtctgctct gagccttaaa 530

<210> 113
<211> 502
<212> DNA
<213> Homo sapiens

<400> 113
   ttatcaagtg tcaagatcag caagtgtcct taagtcaaat aggttttttt ttgttggtgg 60
   ttgtgcttgc tttccttttt tagaaagttc tagaaaatag gaaaacgaaa aatttcattg 120
   agatgagtag tgcatttaat tattttttaa aaaacttttt aagtacttga attttatatc 180
   aggaaaacaa agttgttgag ccttgcttct tccgttttgc cctttgtctc gctccttatt 240
   ctttttgggg gggagggtta tttgcttttt tatcttcctg gcataatttc cattttattc 300
   ttctgagtgt ctatgttaac ttccctctat cccgcttata aaaaaattct ccaacaaaaa 360
   tacttgttga cttgatgttt tatcacttct ctaagtaagg ttgaaatatc cttattgtag 420
   ctactgtttt taatgtaaag gttaaacttg aaaagaaatt cttaatcacg gtgccaaaat 480
   tcattttcta acaccatgtg tt 502

<210> 114
<211> 493
<212> DNA
<213> Homo sapiens

<400> 114
taagcacgtt gcatgacatc tggaacacag aaagccctca atacattgaa gctcttagga 60

ttttcacgat gttcctgtct gctcaatgca tgctttcttt attgttctga cagttgtgtg 120

gtaacaagct aatatgcttc cagttgactt ccagtctacc ctggtgttag aaaccgtttc 180

atctcttatt gtaaatttga gtgcttgttg ttttttatat ttgtgatgac tcttccagca 240

gttgttgaca attgttagag gtttgacttt taaataatta cttatttttt ctgattgtgg 300

ttcagtttaa ctgaagaata tcctgagatt gtaagaaaag cattttttaa aaggtatcac 360

ttgtgatcat ttatctttct aaattctatt tttaatactg ttccaccaaa gtgatgcagt 420

ggttaccatg acaccctaat ttcatgtgtt tttgtattta tgaaaatagt ttcattgtca 480

tttattggcg gta 493

<210> 115
<211> 291
<212> DNA
<213> Homo sapiens

<400> 115
gttgagtcaa cgggggtgtt gccaccagat gtgctggtga gtgaagccat caaagtactg 60

atggggaagt gccggcgctt cttggatgaa ctagatgcgg ttcagatgga ctgagcttgg 120

atgcttctga ggcaagctga agctttgggt tctgactgac ccaccctaca ggactgctga 180

acagagagcc cagtgtgact agggatcctg agttttctgg gacaattcca gctttaatca 240

atacattttg ttaaatgtgc cataaaatga gactttttac gcctttataa g 291

<210> 116
<211> 449
<212> DNA
<213> Homo sapiens

<400> 116
tgtttctaac acgcttgcaa cttcccttat ggcattaatc ttgttgaggg agagagacag 60

aatcctggac tctccaaagt atttaactga aagtagggcc tgctctgaca gggcccatgt 120

cccacaaggc tgcttggcct cagtgggtgc ttggctgtgc tggatgatat gttgatctgt 180

attggataag gaccaatgac agcaaagcaa aaatggcttt aaagcttggt gttacttttc 240

ttaagttgtt taattatagt taagcaattt caaaaatgct ccaaagaaat gtgaaaggac 300

cttttgtcac agcacttcag aaaatacaca acagcccctt ctgcccccgc acagaaatgc 360

tgcagagtat ataaaacttg agacattttt gtaggatgcc tgacgaggtg tagcctttta 420

tcttgtttcc ggatgcatat ttattacga 449

<210> 117
<211> 386
<212> DNA
<213> Homo sapiens

<400> 117
gtagcatgtt gtcagtggcc aaggctacac agaaggctcc ctgctgcccg gagcaggtac 60

atccaccaga gcaaagggaa ccacttttat tttgcatgag tttggtaact gattactctc 120

ccctcaaaga aaagacattc aggtgtttct caacgacatc ttctgtccag caagctcggt 180

ttgaatacgt cacttaccag tgccattgca ggacccaaat tcacagttca taaaagatgt 240

gaccactaca tgtaaaaata gcattctact tgatcttaca gtatgtatgt atgtatgtat 300

ggagacatat gtgtgtgtgt ggatgtctac atggttaatg gaaagcactg tgctctgaag 360

tggatcagtc tcaagtgtct ggtaac 386

<210> 118
<211> 328
<212> DNA
<213> Homo sapiens

<220>
<221> misc_feature
<222> (263)..(263)
<223> n is a, c, g, or t

<400>  118
ggacccagtg tgcgatggct gcacacagca gcttccttgg tagtgtacgc agcctgttgg      60

ttgtatgggt tgctctaagg gaccttggag acaggccttt caggtggatg ttcatgtttc     120

tgaccttgca ctaccccaat gtaggctcca aacaggcatg cgaggtgcct ttggaaagcc     180

ccagggcact gtggccaggg ttcacattgg ccaagttatc atgtccatcc gcaccaagct     240

gcagaacaag gagcatgtga ttngaggccc tgcgcagggc caagttcaag tttcctggcc     300

gccagaaggt atgtagtgct gcagcccc                                        328


<210>  119
<211>  190
<212>  DNA
<213>  Homo sapiens

<400>  119
gtgagagaga aaggtgctgg cataggatct gcccagaaga gaaaatgacc catgcgcagt      60

tgggctctgg atacggcgct gtctatagca agttggccag tctggcctcc tgttcctctg     120

ctggacctgg ggtaggctgc aggggtgggc agaagcccct cttaaattgt ggttgccatg     180

gtaccgaggg                                                            190


<210>  120
<211>  478
<212>  DNA
<213>  Homo sapiens

<400>  120
gcaaaatgtc gcagctggaa cgcaacatag agaccatcat caacaccttc caccaatact      60

ctgtgaagct ggggcaccca gacaccctga accaggggga attcaaagag ctggtgcgaa     120

aagatctgca aaattttctc aagaaggaga ataagaatga aaaggtcata gaacacatca     180

tggaggacct ggacacaaat gcagacaagc agctgagctt cgaggagttc atcatgctga     240

tggcgaggct aacctgggcc tcccacgaga agatgcacga gggtgacgag ggccctggcc     300

accaccataa gccaggcctc ggggagggca ccccctaaga ccacagtggc caagatcaca     360

gtggccacgg ccatggccac agtcatggtg gccacggcca caggccacta atcaggaggc     420

caggccaccc tgcctctacc caaccagggc cccggggcct gttatgtcaa actgtctt       478


<210>  121
<211>  535
<212>  DNA
<213>  Homo sapiens


<220>
<221>  misc_feature
<222>  (98)..(98)
<223>  n is a, c, g, or t

<220>
<221>  misc_feature
<222>  (112)..(113)
<223>  n is a, c, g, or t

<220>
<221>  misc_feature
<222>  (115)..(115)
<223>  n is a, c, g, or t

<220>
<221>  misc_feature
<222>  (125)..(125)
<223>  n is a, c, g, or t

<220>
<221>  misc_feature
<222>  (129)..(131)
<223>  n is a, c, g, or t

<220>
<221>  misc_feature
<222>  (141)..(141)
<223>  n is a, c, g, or t

<220>
<221>  misc_feature
<222>  (227)..(227)
<223>  n is a, c, g, or t

<220>
<221>  misc_feature
<222>  (260)..(260)
<223>  n is a, c, g, or t

<220>
<221>  misc_feature
<222>  (265)..(265)
<223>  n is a, c, g, or t

<220>
<221>  misc_feature
<222>  (274)..(274)
<223>  n is a, c, g, or t

<220>
<221>  misc_feature
<222>  (301)..(301)
<223>  n is a, c, g, or t

<220>
<221>  misc_feature
<222>  (336)..(336)
<223>  n is a, c, g, or t

<220>
<221>  misc_feature
<222>  (342)..(342)
<223>  n is a, c, g, or t

<220>
<221>  misc_feature
<222>  (351)..(351)

<223>  n is a, c, g, or t

<220>
<221>  misc_feature
<222>  (366)..(366)
<223>  n is a, c, g, or t

<220>
<221>  misc_feature
<222>  (370)..(371)
<223>  n is a, c, g, or t

<220>
<221>  misc_feature
<222>  (375)..(375)
<223>  n is a, c, g, or t

<400>  121
cctgtcccat ggagagggac ccagcatagg aaagccacat actcagcctg gatgggtgga      60

gaggctgagg gactcactgg agggcaccaa gccagccnac agccagggaa gnngngaggg     120

ggggncggnn ncccatgcct nccagctgag cactgggaat gtcagcccag taagtattgg     180

ccagtcaggc gcctcgtggt cagagcagag ccaccaggtc ccactgnccc gagccctgca     240

cagccctccc tcctgcctgn gtggnggagg ctgnaggtca ttggagaggc tggactgctg     300

ncaccccggg tgctcccgct ctgccatagc actganc agt gncaatttac nggaatgtag     360

cagcgnatgn nattncctgg aacagttttc gttaagtata gtaaggtttt agtggggggg     420

ggcaactaaa caaacacaaa gtattctgtg tcaggtatag ggctggacag ggcagttgtg     480

tgttggggat ggtaaattac tctatttaaa gtaggtcttg tgttctaata atgtt          535


<210>  122
<211>  525
<212>  DNA
<213>  Homo sapiens

<400>  122
tcatgcatct gttctacgtg cgggcgggag tgtctcgtgc catgctgctg cgcttcttcc      60

tggccatgga gaagggcagg catatggagt atgaatgccc ctacttggta tatgtgcccg     120

tggtcgcctt ccgcttggag cccaaggatg ggaaaggtgt gtttgcagtg gatggggaat     180

tgatggttag cgaggccgtg cagggccagg tgcacccaaa ctacttctgg atggtcagtg     240

gttgcgtgga gcccccgccc agctggaagc cccagcagat gccaccgcca gaagagccct     300

tatgacccct gggccacgct gtgccttagt gtctacttgc aggacccttc ctccttccct     360

agggctgcag ggcctgtcca cagttcctgt gggggtggag gagactcctc tggagaaggg     420

tgagaaggtg gaggttatgc tttgggggga caggccagaa tgaagtcctg ggtcaggagc     480

ccagctggct gggcccagct gcctatgtaa ggccttctag tttgt                     525


<210>  123

<211>  530
<212>  DNA
<213>  Homo sapiens

<400>  123
gggtccacaa tatttccaga ctccacacaa cccttcaggt cctggcctgg gcaaccagct      60

gatgcgactc agccttatgg aagaggacga ggaagaagag ttagaaatcc aggatgagtc     120

aaatgaagaa cggcaggata cagacatgca agcaagtagc tactcttcca ccagtgagcg     180

cgggctggac ctcttagatt ctacagagct ggatattgag gatttctgac aggactctgg     240

gcccctcccc agctccactc cctacctcaa gaatgtgacc atttggaaaa ggcaaagaga     300

aaaggagcaa aatgaagcat tcccccaggc ttcagccctg ggctctgagg ggaaagagtt     360

gggcattgtt tttctaacct aacctttccc tctggggtag agaagccgag agaccctgtc     420

ctccctaatg cactgtggcc cagtcccctt gcctttttcc tgttctgttt ggagtggaga     480

agggcagcac ctctgtgttt aatggaaata gcccatagtc tcctggattt                530


<210>  124
<211>  539
<212>  DNA
<213>  Homo sapiens


<220>
<221>  misc_feature
<222>  (31)..(31)
<223>  n is a, c, g, or t

<220>
<221>  misc_feature
<222>  (53)..(53)
<223>  n is a, c, g, or t

<220>
<221>  misc_feature
<222>  (63)..(112)
<223>  n is a, c, g, or t

<400>  124
aacatcgagg atgaaggcag ctccttctat ngggtctcca gccagtatga ganccccgag      60

aannnnnnnn nnnnnnnnnn nnnnnnnnnn nnnnnnnnnn nnnnnnnnnn nntggagaaa     120

gttgagacag agtatgctcg ctatgagaat ggacactact cttaccgcat ccaccggtcc     180

ccgctctgtg agtacatgat caacttcatc cacaagctca agcacctccc tgagaagtac     240

atgatgaaca gcgtgctgga gaacttcacc atcctgcagg tggtcaccaa cagagacaca     300

caggagacct tgctgtgcat tgcctatgtc tttgaggtgt cagccagtga gcacggggct     360

cagcaccaca tctacaggct ggtgaaagaa tgagagactc ggggagcagg gaggggggaa      420

gagacgtgtg tgcaggaaac ggggacgtgg ggaggggacc tgcaggggca gccccctgaa     480

gtgccaagag agctgagagg agcagttgtg actctaccca ggaacaaact gtgcctgaa      539

<210>  125
<211>  375
<212>  DNA
<213>  Homo sapiens

<400>  125
ttacttagtc cttaggccaa ccaatttaac tgcagtgtca tgtttcacag gccttcctac      60

atttagaaat cgtcacacag ctgtgataag agtagattat tttactatga aataattctg     120

aatagatgaa agcataaaat gtgagaaact gaatgtatta ttcaggaaga atactgagtg     180

ccttcattta actaaagttg aatgtaaaag tcaatttgca cttctttata atcctctggt     240

ttagaattat aaattgttaa aaccttgata attgtcattt aattatattt caggtgtcct     300

gaacaggtca ctagactcta cattgggcag cctttaaata tgattctttg taatgctaaa     360

tagccttttt ttctc                                                      375


<210>  126
<211>  502
<212>  DNA
<213>  Homo sapiens

<400>  126
agcccatgat gccgtggcac aggaaggcca atgtcgggta gatgacaagg tgaatttcca      60

ttttattctg tttaacaacg tggatggcca cctctatgaa cttgatggac gaatgccttt     120

tccggtgaac catggcgcca gttcagagga caccctgctg aaggacgctg ccaaggtgtg     180

cagagaattc accgagcgtg agcaaggaga agtccgcttc tctgccgtgg ctctctgcaa     240

ggcagcctaa tgctctgtgg gagggacttt gctgatttcc cctcttccct tcaacatgaa     300

aatatatacc ccccatgcag tctaaaatgc ttcagtactt gtgaaacaca gctgttcttc     360

tgttctgcag acacgccttc ccctcagcca cacccaggca cttaagcaca agcagagtgc     420

acagctgtcc actgggccat tgtggtgtga gcttcagatg gtgaagcatt ctccccagtg     480

tatgtcttgt atccgatatc ta                                              502


<210>  127
<211>  244
<212>  DNA
<213>  Homo sapiens

<400>  127
cagtataatg actcacccgg aaggtttctt aattcgttct tccatttatt tttaaaaatt      60

ttgtttgaac gcctactaag ttctgggtgc agggtataac acagcaagca ccatggaaag     120

gtccctgctc ctagtgctca cactccaata agaagaagtg gctgggccgg gcacagcggc     180

tcacgctgta accccagcat ttcgggaggc ctgggcaggc agatcacctg gggtaaggaa     240

tttg                                                                  244

<210>  128
<211>  116
<212>  DNA
<213>  Homo sapiens

<400>  128
gtcttgagga tacacgggac ccagaatcca cggggtctgg agcataaggt ttatctcaag      60

tctcaattga actgcctcct cttgtcaggc agggacaact gggaggatga gcccca          116


<210>  129
<211>  547
<212>  DNA
<213>  Homo sapiens

<400>  129
ggaccagctc ccctagagga gataaagcag cctatgacat tctgaggaga tgttctcagt      60

gtggcatcct gcttcccctg ccgatcctaa atcaacatca ggagaaatgc cggtggttag     120

cttcatcaaa aaggaaaaca agtgagaaat ttcagctaga tttggaaaag gaaaggtact     180

acaaattcaa aagatttcac ttttaacact ggcattcctg cctacttgct gtggtggtct     240

tgtgaaaggt gatgggtttt attcgttggg ctttaaaaga aaaggtttgg cagaactaaa     300

aacaaaactc acgtatcatc tcaatagata cagaaaaggc ttttgataaa attcaacttg     360

acttcatgtt aaaaaccctc aacaaaccag gcgtcgaagg aacatacctc aaaataataa     420

gagccatcta tgacaaaacc acagccaaca tcatactgaa tgagcaaaag ctggagcatt     480

actcttgaga agtagaacaa ggcacttcag tcctattcaa catagtactg gaagtctcgc     540

cacagca                                                               547


<210>  130
<211>  546
<212>  DNA
<213>  Homo sapiens

<400>  130
ggagaaccta ctgcaggaag tcagccctga ctcaccatca gagaacacac acaggacaga      60

gaccctatga gtgtaatgaa tgtgggaaaa ccttctgtca gaagttctcc tttgttgaac     120

atcagcgaac tcacactggg gagaaaccat atgaatgtaa tgaatgtggg aaatccttct     180

gccataagtc agccttcaga gtccatagaa gaattcacac aggagagaaa ccatatgaat     240

gtaatcaatg tgggaaaacc taccgtcgcc tgtggactct cactgaacat cagaaaatac     300

acacaggaga gaaaccttat gaatgtaaca aatgtgagaa aacatttcgc cacaaatcaa     360

actttctttt acatcagaaa tcccacaagg aataagttcc aacaaagtaa taaattccaa     420

caaagcaaca aatcaaataa cttacacaat gctaaactgt gagtatgtat agaggagtaa     480

aatcttataa atgtaatgaa tatggaaaat ttttctgcca taagtcaacc ctcaatgtaa     540

catcag                                                                546

<210>  131
<211>  511
<212>  DNA
<213>  Homo sapiens

<220>
<221>  misc_feature
<222>  (205)..(205)
<223>  n is a, c, g, or t

<220>
<221>  misc_feature
<222>  (216)..(216)
<223>  n is a, c, g, or t

<220>
<221>  misc_feature
<222>  (232)..(232)
<223>  n is a, c, g, or t

<220>
<221>  misc_feature
<222>  (260)..(260)
<223>  n is a, c, g, or t

<220>
<221>  misc_feature
<222>  (378)..(378)
<223>  n is a, c, g, or t

<220>
<221>  misc_feature
<222>  (390)..(390)
<223>  n is a, c, g, or t

<400>  131
gtccagggag aggcgggatc acggcccagg ctgctgacac ccgcgcgtgg ggaggactcg      60

ggccacgaaa ggaaagaaat gtataccgta tctatctacc cgacagcagc gaccgagacc     120

cggtgggaca ctccccttct ccccactttc acctccccac ccaaacttta taaaagttga     180

aaaaatatca tttgactttt tatangaaaa aaaaanggaa aaaataattg angaaagtgt     240

tcatctgagg actgcatcgn gtggacactg gtatttattt atgttagctc caagcggacc     300

ggtggttcaa aagtgcatta tttagtttga gctctgtagg taaaaaggag gtgggaaaaa     360

ttttaaaact tgagggtnaa aatgtggaan acaaaccctc ccatcccttg tagattataa     420

ataaaagcaa aaccgccaca gaactagagg tcttctcttt aatgttactt taaaattgct     480

atgattgtat tgtacgttat ttaatgtctg a                                    511

<210>  132
<211>  259
<212>  DNA

<213>  Homo sapiens

<400>  132
gaagtggtgg acctttcagc agccacatag atagaagagt gaaccgacac agtgtgttat      60

catagaggag acaaaaatgg tagtgaagct tgtggttatg tatcttatct ttctacattt     120

acatgttttc tgtaaggagt gtggttatag agagactgtt ggaaccatga gaaggatgtt     180

tctgtgttct tgattatatt gttctgcaaa actgctaagc catgaacagt tttcttagct     240

acttagaatg gttatacat                                                  259


<210>  133
<211>  348
<212>  DNA
<213>  Homo sapiens

<400>  133
aagacctgtg aagtatccta gtcagggaaa gaacctgttt tctggaagaa tgtaagttgc      60

tatatgtgga ggggcatgta ggacaggggt tctaattact gtctgtgaga gttactactt     120

tgtaacttat ggtttctgct tcagtattgt gttggttctg tattataaat tgcaccttaa     180

tagaccaaaa atatctatac agcactgatt tccttgaata ttgtgtgtta attgatatat     240

cttctagagg caaaaggttt aaatgtgtta aaatggttat attgcctcct ttttaaacaa     300

attaaagagt tgtaaatact agtgtagatc tcttttgttt accacagc                  348


<210>  134
<211>  355
<212>  DNA
<213>  Homo sapiens

<400>  134
tcttgcatac tcagcttgct ttatcatttt tgctgtcctt ttaacatttg cgaggagtag      60

gggcctggtc ctgaatgact tggaggcttt cattatttat cctgtctgta ttgaccggtt     120

tttgtttttt cagaaggcag tgatgatgaa aacttaggaa gaaggtattt tgcaataagc     180

ttggctgagt gtccatggga agaatacttt ccctaaagag agagaagcac tcacagaggc     240

tgcctttctc ctgagctcgg ggagaaggca gcacatcaca acctgtcaca ttgaaatagg     300

cgctcattct gctatttcac cttccgtcct gagcagagcc tgaattacgt ttttg          355


<210>  135
<211>  80
<212>  DNA
<213>  Homo sapiens

<400>  135
   atcttcgaaa tgcttgaagg agtgcaagga cctactgcag tcaggaagcg attttttgaa      60
   tccatcatca aggaagcagc                                                  80

<210>  136
<211>  528
<212>  DNA
<213>  Homo sapiens

<220>
<221>  misc_feature
<222>  (29)..(29)
<223>  n is a, c, g, or t

<220>
<221>  misc_feature
<222>  (32)..(32)
<223>  n is a, c, g, or t

<220>
<221>  misc_feature
<222>  (56)..(56)
<223>  n is a, c, g, or t

<220>
<221>  misc_feature
<222>  (79)..(79)
<223>  n is a, c, g, or t

<220>
<221>  misc_feature
<222>  (82)..(82)
<223>  n is a, c, g, or t

<220>
<221>  misc_feature
<222>  (85)..(85)
<223>  n is a, c, g, or t

<220>
<221>  misc_feature
<222>  (97)..(97)
<223>  n is a, c, g, or t

<220>
<221>  misc_feature
<222>  (106)..(106)
<223>  n is a, c, g, or t

<220>
<221>  misc_feature
<222>  (113)..(113)
<223>  n is a, c, g, or t

<220>
<221>  misc_feature
<222>  (174)..(174)
<223>  n is a, c, g, or t

<220>
<221>  misc_feature
<222>  (407)..(407)
<223>  n is a, c, g, or t

<220>
<221>  misc_feature

<222>  (409)..(409)
<223>  n is a, c, g, or t

<220>
<221>  misc_feature
<222>  (463)..(464)
<223>  n is a, c, g, or t

<220>
<221>  misc_feature
<222>  (466)..(469)
<223>  n is a, c, g, or t

<220>
<221>  misc_feature
<222>  (471)..(472)
<223>  n is a, c, g, or t

<220>
<221>  misc_feature
<222>  (478)..(479)
<223>  n is a, c, g, or t

<220>
<221>  misc_feature
<222>  (481)..(481)
<223>  n is a, c, g, or t

<220>
<221>  misc_feature
<222>  (484)..(485)
<223>  n is a, c, g, or t

<400>  136
ttctctggag atgacctaga atgcagagna anagcctcct ctcccaaaag ccaacnagaa      60

attaatgctg atataaaang tnaantagtg aaggaantcc gatgcnttgg acnaaaatat     120

gaaaaaatct tcgaaatgct tgaaggagtg caaggaccta ctgcagtcag gaancgattt     180

tttgaatcca tcatcaagga agcagcaaga tgtatgagac gagactttgt taagcacctt     240

aagaagaaac tgaaacgtat gatttgagaa tacttgtccc tggaggatta tcacacccca     300

aatgcataat ctcgttaatg attgaggaga gaaaaggatc agattgctgt tttctacaat     360

ggagcaggat attgctgaag tctcctggca tatgttaccg aatcaantng ccttccagag     420

gctaagaaat ttctgttagt aaaagatgtt ctttttccca aannannnna nntgaaanna     480

naanntgtgt tttggttatt ttgtattccc acctgtgctg gtagatat                  528


<210>  137
<211>  560
<212>  DNA
<213>  Homo sapiens

<400>  137
cccaagcccc tagagaagtc agtctccccg caaaattcag attcttcagg ttttgcagat      60

gtgaatggtt ggcaccttcg tttccgctgg tccaaggatg ctccctcaga actcctgagg     120

aagttcagaa actatgaaat atgaaatatc tctgcttcaa aaaatgagga agagcaagac 180

tgtcccctat gctgccaaca tgcagtcttt gtttatgtct taaaaatgtc atgtttatgt 240

catgtctgtg aattgctgag tactaattga ttcctccatc cttgaatcag ttctcataat 300

gctttttaaa taagaaaaat tcagaagatg aatttcttcc aatatttgaa taaattaaag 360

ctcttagata cagagtagat tgtattatat gctttttcct attaatacta cttatagaaa 420

tccattaaaa agcaatctct gtacagtgta tttaaatatt tcattgacat actgtgatct 480

ctattagtga tggatgtaca aaaaatgttt tcttaccctt gacttacaat gaaatgtgaa 540

attacttgtc tgaaccccgt 560

<210> 138
<211> 255
<212> DNA
<213> Homo sapiens

<400> 138
gaagattact acacattact gggatgtgat gaactatctt cggttgaaca aatcctggca 60

gaatttaaag tcagagctct ggaatgtcac ccagacaagc atcctgaaaa ccccaaagct 120

gtggagactt ttcagaaact gcagaaggca aaggagattc tgaccaatga agagagtcga 180

gcccgctatg accactggcg aaggagccag atgtcgatgc cattccagca gtgggaagct 240

ttgaatgact cagtg 255

<210> 139
<211> 300
<212> DNA
<213> Homo sapiens

<400> 139
agtgaagacg gtgggtttct cgctgggtgc gacgtgaatt tgtgaagctc aggatgccca 60

tggattagac tcatgtagta gcttaaagag tcattaggcg ataggaggga gaaaaccaag 120

aagttagcag agtctggata taattcagtg tccgtaaatc ccatgaagag aagctcatca 180

gaataaaggc aatgaatttg tgccagaaga gcctgtgact acatttcttt caatctcttt 240

ggctgtttgt ttcagtatac acactccctg ttattgctta ctattttatg agctctaata 300

<210> 140
<211> 415
<212> DNA
<213> Homo sapiens

<400> 140
atcacacggc agcacagcac gggacagctc taccgcttgc aagatgtttc cagccctctg 60

aagcttcatc gaacagagac ttttccagcc tacagatctg agcactgaca gtaactgcca 120

agaattgtta acacacttgt gatgtgtcag ccacagattc acccaggagg tcacagaatg 180

acagcaaggg aaatgacgac caagagaaga agcttaaagt cctggctaat tgtgtggtca 240

ttggaaaact ctgcaataca ataattttct ttattttctt tttctttttt aaattcttag 300

tgtaattgaa acgtgctcta tagatattga ctctgtgttc cctcttttac agctggacag 360

aaagaagtca atgtcacgaa atgattttct attgtagata ctttgtccct tgcac 415

<210> 141
<211> 542
<212> DNA
<213> Homo sapiens

<220>
<221> misc_feature
<222> (499)..(499)
<223> n is a, c, g, or t

<220>
<221> misc_feature
<222> (507)..(507)
<223> n is a, c, g, or t

<400> 141
gtcctggcta attgtgtggt cattggaaaa ctctgcaata caataatttt ctttattttc 60

tttttctttt ttaaattctt agtgtaattg aaacgtgctc tatagatatt gactctgtgt 120

tccctctttt acagctggac agaaagaagt caatgtcacg aaatgatttt ctattgtaga 180

tactttgtcc cttgcacttc tctgaatctg tccttttgtg gattcttgtg attttccttc 240

caagtgtttc agttgtatga cagtcagtat tgacaataaa atggctttta attatttgtt 300

atttgtttac accctattcc tcagttatta ttactgtggt tctgattaac tactggaaat 360

tatatttgat tatatcacca attagttaaa tcagtgcttc gactcactct tatctgttct 420

gttcaaaact atttgttcaa agaacccgtt agtgttgttt acagggttac agtttctctc 480

acatgctttc ctcaccccnt taccccnctt tttgaaagcc tttattttgt tcggagtctc 540

tt 542

<210> 142
<211> 528
<212> DNA
<213> Homo sapiens

<220>
<221> misc_feature
<222> (73)..(73)
<223> n is a, c, g, or t

<400> 142
gcctggctag ctggaagacg cggtatggca gggacaggag ggcggcagga agggctttgt 60

ggccctgctg ggnccaaagg cagttttctg tgctcccttt agccccagcc tggctggcca 120

ggtctcctgt acttcagggg accatggggc cgcccaagcc agtcaccttc cccttccaag 180

ctctcccccc atgactgttg atttctggcg cgtggaggca gagttgtgaa tccacaaatg 240

ctgtagccca tatctcgtaa tgtgtctgtg tccacttaga gcctcacgac ctggctgtgt 300

gtgtgcatgt gtgtgtagta tagtttgtag ttgacaatat gtgtatataa tagctggtgt 360

atttatatgt gagtgcagaa ttagtgcccg tggcggtttt ctgtatttta aacccattga 420

attggttagt tttattttag ggaagtggga aaggcggagt ggggggaagt ataggaagaa 480

ggattcttag caaaatcatt ctctaggact attagtcgcg atctccca 528

<210> 143
<211> 570
<212> DNA
<213> Homo sapiens

<220>
<221> misc_feature
<222> (41)..(41)
<223> n is a, c, g, or t

<220>
<221> misc_feature
<222> (68)..(68)
<223> n is a, c, g, or t

<220>
<221> misc_feature
<222> (75)..(75)
<223> n is a, c, g, or t

<220>
<221> misc_feature
<222> (86)..(86)
<223> n is a, c, g, or t

<220>
<221> misc_feature
<222> (89)..(89)
<223> n is a, c, g, or t

<220>
<221> misc_feature
<222> (412)..(412)
<223> n is a, c, g, or t

<220>
<221> misc_feature
<222> (414)..(417)
<223> n is a, c, g, or t

<220>
<221> misc_feature
<222> (419)..(420)
<223> n is a, c, g, or t

<220>
<221> misc_feature

<222>  (422)..(428)
<223>  n is a, c, g, or t

<400>  143
cttgctatag atttgcagct taataacatt gttgtacaaa naaacactgt caaggatttc      60

aaattttngg tattntattg attctngtnt tgggcctaac ctcagcctat atctgtctat     120

ctaacctatc tatctatcta tctatctatc tatctatcta tctatctacc tacctaccta     180

tctattatct atctacctag atagacagag atatctatag atagctatgg taaattttga     240

aacacgttcc aaatacatta taataaattg tgtgttttgc tatttttggg tgcagttttt     300

atatcaaagt atttaatcct actgctttaa atttttctat tattattgat attttgtatc     360

ctttttgcta tcagttattg aaaaaggtat attaaagtct ttatgattga anannnngnn     420

annnnnnntt ttagttctat aaaaattgta aatgtgtgtt aactgtgtgt gagtgaatat     480

acatacacaa gtttgacaac ttcctcaata gttgattctt ttatatggga tgatgggata     540

catcttcctt tcaagtaata atttctgcct                                      570


<210>  144
<211>  478
<212>  DNA
<213>  Homo sapiens

<400>  144
cgctggaaga gctcgtggcg tgcgacaacg cggcgcagaa gatgcagacg gtgaccgccg      60

cggtggagga gcttttggtg gccgctcagc gccaggatcg cctcacagtg ggggtgtacg     120

agtcggccaa gttgatgaat gtggacccag acagcgtggt cctctgcctc ttggccattg     180

acgaggagga ggaggatgac atcgccctgc aaatccactt cacgctcatc cagtccttct     240

gctgtgacaa cgacatcaac atcgtgcggg tgtcgggcat gcagcgcctg gcgcagctcc     300

tgggagagcc ggccgagacc cagggcacca ccgaggcccg agacctgcat tgtctcctgg     360

tcacgaaccc tcacacggac gcctggaaga gccacggctt ggtggaggtg gccagctact     420

gcgaagaaag ccggggcaac aaccagtggg tcccctacat ctctcttcag gaacgctg      478


<210>  145
<211>  260
<212>  DNA
<213>  Homo sapiens

<400>  145
tgagtagggc aaattttaaa tgggtattat ttttcatctt caaacaggca gacctgttat      60

cctaaactag gtgagtcagc ttttggtaca tgtgatgatt ttcagtgtaa ccaatgatgt     120

aatgattctg ccaaatgaaa tataatgata tcactgtaaa accgttccat tttgattctg     180

aggttactct actaacaagc atcacacatt tgtattttgc cctgattaat atgttggctt     240

cgctttcagg gtttttaatg                                                 260

<210>  146
<211>  535
<212>  DNA
<213>  Homo sapiens

<400>  146
gaaaaacatg agcactctgc agacaaaatg accttaaatc acattgatta agatatttta      60

aaagttagca gtgaaccaaa agtagtttca gattagcaga aataaagagc tttaagtttt     120

aaaagttgag attgaatatt taatgaaagt ttattaattc tttttccagg aatagcagta     180

aggtcagttt ttttccctaa aataaaaagt tttaataaac agaaaattat agcaacagta     240

cttaggagaa tagttgaaga ttgtattaat tttaattata atagttattt gtcataggct     300

attgatgatt agaatttcat tagttttgtc cactataatt ttaaaaatag ttgtgtcaaa     360

tacaaattct ggtaaactgt taggttttaa caccagacag tattcaaagg aaattaatgt     420

ttgcaaacat aaatccatag tcttcatttc tttatattgt cacctttgta aaagtgttta     480

aaatttgtat tgtttgtttt gtatatcttt gggcatcttg tgtctagcta taata          535


<210>  147
<211>  177
<212>  DNA
<213>  Homo sapiens

<400>  147
gccgggcggg ggaatataca aagtgaagcc acattgccaa acttgcagca gcgattgcag      60

cagttgctgc cgctgcgccg cgcctgaagc cgcgccgcgc gggccgaggg ctcctgcagc     120

tgctcgcgcg cagtcggagg cggagaagga cgaagactga gactgacact tctgctc        177


<210>  148
<211>  547
<212>  DNA
<213>  Homo sapiens


<220>
<221>  misc_feature
<222>  (145)..(155)
<223>  n is a, c, g, or t

<220>
<221>  misc_feature
<222>  (161)..(161)
<223>  n is a, c, g, or t

<220>
<221>  misc_feature
<222>  (299)..(299)
<223>  n is a, c, g, or t

<220>
<221>  misc_feature

<222>  (316)..(316)
<223>  n is a, c, g, or t

<220>
<221>  misc_feature
<222>  (320)..(320)
<223>  n is a, c, g, or t

<220>
<221>  misc_feature
<222>  (345)..(345)
<223>  n is a, c, g, or t

<220>
<221>  misc_feature
<222>  (354)..(354)
<223>  n is a, c, g, or t

<220>
<221>  misc_feature
<222>  (364)..(364)
<223>  n is a, c, g, or t

<220>
<221>  misc_feature
<222>  (372)..(372)
<223>  n is a, c, g, or t

<220>
<221>  misc_feature
<222>  (401)..(403)
<223>  n is a, c, g, or t

<220>
<221>  misc_feature
<222>  (424)..(425)
<223>  n is a, c, g, or t

<400>  148
agatgtaaag ggtccattca tgaggaataa catcactttg accctttgtg ttagctccta      60

ctgctaatct ttccataagc gtgaacttac tttcaatttg aagaagatga aaaagtcttt     120

tgcaaaaccc caatactgtt ttctnnnnnn nnnnnctgaa nggagaaacg tagtcttacc     180

tcgttttttt ccactacaag ccaagctact tgtaatcctt ccaaaccttt aaaggggacc     240

tcccttgtta gcatctccca gagaacctgg aacaaaaaga aaatggaatt tttattttnt     300

gtactttata tttttntggn tacatattac tgagacctta tctanacatg taanttgtat     360

ctanattacc angacacaac caaccccaac tgtagaaact nnngcaaaac aattaagcac     420

acannttctt ctatatatta aatacttttt acgttctttt ttataaagaa aaaacctggt     480

ttagaaatta ggacatctag agccagactt aaaattttat gaaaccaatg ggcctgactg     540

atttaat                                                               547


<210>  149
<211>  353
<212>  DNA

<213>  Homo sapiens

<400>  149
ggacttgaat agacctttta cagagaagat acataaaagg ccaacaaatg catgaaagga      60

tgttcaacat cactaataac tagaagaaat gcaaatcaaa atgacagtga gatattgtct     120

tacactcatt aggatggata ctatattttt tttaaaaaga caagataaca agtgttagta     180

aggacataca gaaattggaa cccttgttca ttgttggtag gaatggaaga tggtacatct     240

gccttggaaa actatatggt ggttctcccc aaaattaaaa tagaattacc atctgatcca     300

gcaattccac ttctgggtac acacccaaaa gaactgaaaa catggctaac atg            353


<210>  150
<211>  567
<212>  DNA
<213>  Homo sapiens


<220>
<221>  misc_feature
<222>  (520)..(520)
<223>  n is a, c, g, or t

<400>  150
tctgctctgt tcagtttgct gttgtgtttt tctcccccat gttggggtgg tggggtacag      60

ggaaataaaa tgctttctcc caggccccta atccttcccc atgcctccat cagcctcaaa     120

gctgctgaca gtcatgaact gcaccttcca gccctgccca taagctactc aaagcaaatt     180

caaattctct tctggccagg aggaagggca gatgctccct ccttcctcaa gcctccctgg     240

ctcattgatc cattttgagg gcatttgggg gtcaaagttg agaccagatt gcttcagttt     300

gtataaaatt agcatttctt atcacaccaa ggccacacct gttctctggc ctcacaaacc     360

agtgaggatg taaaggtttg ttgaggtgga ggaacagaag tgaaatgagc aatctgctcc     420

atttagaagt cagtcgcttc ggctgttcat tccactaata tttatctagt acctattctg     480

tgccaagcat tgtctctacc tcagtttgcc acaaatatgn aaaaaaaaaa attcttggaa     540

ctgtgaggct tcaatgtgtt gtggacc                                         567


<210>  151
<211>  521
<212>  DNA
<213>  Homo sapiens

<400>  151
agatgaagtg tgccagctgg ttcccccaag ccagctcatg ctgctgacca ctgactcagc      60

tctgaccttc acatttgctc tgaagcaagt gcgttcagct gctggggcag tgatatcaca     120

tagtacatat attatttcct tagtttattt ccaaactggt attttaaata gacacttcga     180

actttgggct actctgttta aatctgccac tttctggact ggaccttagt actgtaaatt     240

ctttttaaag aataataatg ttaccaactg ctgagatttt tatgtatttt gtgactttgt 300

aacaactgct attgtaataa gtgtcatctt gtgggcatta tacaaaggca tattataaaa 360

taataatgat atttttgtat agaagagtca actgttcaga tgtaagatgt tgaaaaatgt 420

taaaatctaa agagtaattt atcctagtgg taatggttat atgtatttgt acagtttaaa 480

ttaatgtctc aaagctgtgc agtcttttgt tactgggaca c 521

<210> 152
<211> 524
<212> DNA
<213> Homo sapiens

<220>
<221> misc_feature
<222> (99)..(99)
<223> n is a, c, g, or t

<220>
<221> misc_feature
<222> (186)..(188)
<223> n is a, c, g, or t

<400> 152
gggtaaataa catctgacag ctaatgagat attttttcca tacaagataa aaagatttaa 60

tcaaaaaatt tcatatttga aatgaagtcc caaatctang ttcaagttca atagcttagc 120

cacataatac ggttgtgcga gcagagaatc tacctttcca cttctaagcc tgtttcttcc 180

tccatnnnat ggggataata ctttacaagg ttgttgtgag gcttagatga gatagagaat 240

tattccataa gataatcaag tgctacatta atgttatagt tagattaatc caagaactag 300

tcaccctact ttattagaga agagaaaagc taatgatttg atttgcagaa tatttaaggt 360

ttggatttct atgcagtttt tctaaataac catcacttac aaatatgtaa ccaaacgtaa 420

ttgttagtat atttaatgta aacttgtttt aacaactctt ctcaacattt tgtccaggtt 480

attcactgta accaaataaa tctcatgagt ctttagttga ttta 524

<210> 153
<211> 458
<212> DNA
<213> Homo sapiens

<400> 153
atgaccaggt taaatccctc tacatgtggt ttctgtttga aaaaaagaaa actgacattt 60

gaacaggact tttaatttgt ttaaaactct ggtaattact tgtaacagta gaaaatagaa 120

gtcattctta ttttagaaaa agtgacagaa gcagtccagt aagattatat gtttctgttt 180

ctggtaaata ccatatatga tcctcgaaat gataatatct ccagaatatt gttttcaccc 240

aaatttgagt agatatttta aacacctaac aaagtaaagg gctaaaagcc attcagatag 300

cagtaaaaca ttctgtatga tgtgcaataa aacatccaag atcttttttg aaagttttat 360

ttataatata catttttgta tgagaaaggt gattggtaca gggtgcctat tttagtcatg 420

gatcaaaatt tgtgtaactt gcagggcttt ctttcttt 458

<210> 154
<211> 454
<212> DNA
<213> Homo sapiens

<220>
<221> misc_feature
<222> (214)..(214)
<223> n is a, c, g, or t

<220>
<221> misc_feature
<222> (216)..(216)
<223> n is a, c, g, or t

<220>
<221> misc_feature
<222> (221)..(221)
<223> n is a, c, g, or t

<220>
<221> misc_feature
<222> (223)..(224)
<223> n is a, c, g, or t

<400> 154
gtacatctcg gattttcctc ttaacaaaca ataatctcct tcttgctgac caaaagtctg 60

gacaaatcaa gtcagaggtt ccactggtgg atgtgaccaa ggtatcaatg agctcacaaa 120

atgatggctt cttcgccgtc cacctcaaag agggctcaga agcagctagt aaaggagact 180

ttctcttcag cagtgatcac ctgattgaaa tggncnccaa ncnnctatcg cacaactctc 240

agccaaacca aacagaagct caatattgag atttccgatg agttcctggt acagttcaga 300

caggacaaag tatgtgtgaa gtttattcag ggaaaccaga aaaatgggag tgtcccaaca 360

tgtaaacgaa aaaacaaccg tctccttgaa gttgctgtcc cttaactggc gcctcctctc 420

tactttcatg gacttgttcc tttgtaatag tgca 454

<210> 155
<211> 249
<212> DNA
<213> Homo sapiens

<400> 155
gtgtgaaggt gggctcacgg ctgactctct gtgcccaggg ctgtgctgcc atcctggata 60

caggcacacc tgtcatcgta ggacccactg aggagatccg ggccctgcat gcagccattg 120

ggggaatccc cttgctggct ggggagtaca tcatccggtg ctcagaaatc ccaaagctcc 180

ccgcagtctc actcctcatt gggggggtct ggtttaatct cacggcccag gattacgtca 240

tccagtttg 249

<210> 156
<211> 231
<212> DNA
<213> Homo sapiens

<220>
<221> misc_feature
<222> (132)..(132)
<223> n is a, c, g, or t

<400> 156
tccagtacct gtgtggatcc tcggcgacgt tttcttgggg gcgtatgtga ccgtcttcga 60

ccgcggggac atgaagagcg gcgcacgagt gggactggcg cgcgctcgcc ctcgcggagc 120

ggacctggga angcgcgaga ccgcgcaggc gcagtaccgc gggtgccgcc caggtgatgc 180

gcatgcgcac cgggtagcag agctagcgct actcagtaaa aatccaatat t 231

<210> 157
<211> 175
<212> DNA
<213> Homo sapiens

<220>
<221> misc_feature
<222> (89)..(89)
<223> n is a, c, g, or t

<400> 157
gatgtatcca gttaaagcag gcaatagatg aaaataaaaa tgctcttcaa aaattaagca 60

aagctgatga atctgcacct gttgcaaanc tataatcaga gaaaagaaga ggagcatact 120

cttttggaca agcttaccca acaactgcag ggccttgctg tgacaataag cagag 175

<210> 158
<211> 546
<212> DNA
<213> Homo sapiens

<400> 158
   agtggcatga aggactctac tgtggtgtgg ccccctctgc aaagtgcatt tggagaccag 60
   gttccatgcc aacaaactat gagctgtact atggcttcac aaggtttgct attgagctca 120
   atgagttaga tccagtacta aaagatctcc ttccaccaac agacgcccgg ttccggccag 180
   atcaaagatt tttggaagaa ggaaatttag aagctgcagc atcagagaag caaagagtag 240
   aggaactcca gagatctcgg agacgatata tggaagaaaa caatcttgaa catataccaa 300
   aattttttaa aaaagttatt gatgccaatc aaagagaagc ctgggtttct aacgacacct 360
   actgggagct tcgaaaggac cctgggttta gcaaagtaga cagccctgtt ctttggtaga 420
   ctgggaatgt agagctagcc aacatatcac attctgaatg aataaataac tatgcacaat 480
   tatgtttctt atagctatgt gtggtttctg ggtcaactga aaacctacca tttgcttttc 540
   tattca 546

<210> 159
<211> 378
<212> DNA
<213> Homo sapiens

<220>
<221> misc_feature
<222> (27)..(27)
<223> n is a, c, g, or t

<220>
<221> misc_feature
<222> (29)..(38)
<223> n is a, c, g, or t

<400> 159
tgactttcaa tacttcccct gaaggangnn nnnnnnnnag aaatgtctgt tttgaggaaa 60

gggctttgaa ttccccagat actgaacaat ttgtgtttgt gactgatgga gaatttcagg 120

aatgaatgag aaagcctttg cgaaactatg caacagttta catcagtcat gtgaagtatt 180

tgtctaaaac agagcaaact gaagaccaaa ttattctcct gttgaggtcc gtggatggca 240

gatttaaagg gaagaaccac aaaggcttgc aaagatagga gaggctccat ctctaatgca 300

tgtagaagct ccttacgggt gcccatcaag agcatagctt ggaagccacc atgctgtgcg 360

gaactgcgtc agggcaaa 378

<210> 160
<211> 555
<212> DNA
<213> Homo sapiens

<220>
<221> misc_feature
<222> (372)..(387)
<223> n is a, c, g, or t

<220>
<221> misc_feature
<222> (475)..(475)
<223> n is a, c, g, or t

<220>
<221> misc_feature
<222> (481)..(496)
<223> n is a, c, g, or t

<220>

<221>  misc_feature
<222>  (500)..(500)
<223>  n is a, c, g, or t

<220>
<221>  misc_feature
<222>  (502)..(503)
<223>  n is a, c, g, or t

<220>
<221>  misc_feature
<222>  (511)..(511)
<223>  n is a, c, g, or t

<400>  160
gagttccaca gacagctcgg ggcccctgga ggaggcagag gaggcccccc agctgatgcg      60

gaccaagagc gacgccagtt gcatgagcca gaggaggccc aagtgccgcg cccccggtga     120

ggcccagcgc atccggcgac accggttctc tatcaacggc cacttctaca atcataagac     180

ctccgtgttt actccagcct atggatccgt gaccaatgtg agggtcaaca gcaccatgac     240

aaccctgcag gtgctcaccc tgctgctgaa caaatttagg gtggaagatg gccccagtga     300

gttcgcactc tacatcgttc acgagtctgg ggagcggaca aaattaaaag actgcgagta     360

cccgctgatt tnnnnnnnnn nnnnnnngcc atgtgagaag atcgccagga tcttcctgat     420

ggaagctgac ttgggcgtgg aagtccccca tgaagtcgct cagtacatta agttngaaat     480

nnnnnnnnnn nnnnnntttn tnnaaaaatt naaaagaaga ggaagaaaga gaaataatca     540

aacttgacca tgaag                                                      555


<210>  161
<211>  534
<212>  DNA
<213>  Homo sapiens

<400>  161
gaaagacttc aacaagagcc aggccatcga gtcagtggaa aggaaaaagc aagagaaaaa      60

gcaagaacaa gaggacaact tggccgagat caccaacctc ctgcgtgggg acctgctctc     120

cgagaacccg cagcaggcag ccagctcctt cgggccccac cgcgtggtcc ctgaccgctg     180

gaagggcatg acccaggagc agctggagca gatccgccta gtccagaagc agcaaatcca     240

ggagaagctg aggctccagg aagaaaagcg ccagcgagac ctggactggg accggcggag     300

gattcagggg gctcgcgcca ccctgctgtt tgagcggcag cagtggcggc ggcagcgcga     360

cctgcgcaga gctctggaca gcagcaacct cagcctggcc aaggagcagc atttgcagaa     420

aaaatatatg aatgaagtct atacaaatca acccacggga gactatttca cacaatttaa     480

tacaggaagt cgataatgag gaacacaccc ttgttcccgt cattcacgta taaa           534


<210>  162
<211>  448

<212>  DNA
<213>  Homo sapiens

<220>
<221>  misc_feature
<222>  (294)..(294)
<223>  n is a, c, g, or t

<220>
<221>  misc_feature
<222>  (348)..(348)
<223>  n is a, c, g, or t

<400>  162
gcaaacactc tatatcaaga cccccctccc cgagagctgg ttgttaaaca tttgtcagca      60

caccactgct gctgggagac agagagaaga aactgataca cgtgagcact gaaacatctt     120

agttccaggg aggtagcatt tgtgttgtat ttctcagaag caagtgtcct agtgtaatgt     180

gcacaagtgg catgaagttt aacacctaaa agactgcttc tctgcttgtt ctgtctaatt     240

tctgtgattt caacacttga gaaagatgtt tactttgcta ctaaacttat tggnagtgat     300

ttgtaaagtt cactgtcacc tgtgggcagt agttcagcta aattcccncc aggaaagtgg     360

actgtagctt ttcctgccta actggcaaaa tgaaaatatg ctgaaaagaa tacatcttaa     420

ctaggcacca gaatagctca tttccaag                                        448

<210>  163
<211>  337
<212>  DNA
<213>  Homo sapiens

<220>
<221>  misc_feature
<222>  (38)..(38)
<223>  n is a, c, g, or t

<220>
<221>  misc_feature
<222>  (49)..(49)
<223>  n is a, c, g, or t

<220>
<221>  misc_feature
<222>  (55)..(55)
<223>  n is a, c, g, or t

<220>
<221>  misc_feature
<222>  (58)..(58)
<223>  n is a, c, g, or t

<220>
<221>  misc_feature
<222>  (111)..(111)
<223>  n is a, c, g, or t

<220>
<221>  misc_feature
<222>  (147)..(147)
<223>  n is a, c, g, or t

<400>  163
aaccttaggt cctactgtac tcttggtgaa tcacagcnct ttagagctnc ctcanggncc      60

taagcatctt ttgttagaaa attctggctg aaattcctca tgagatgggt ncagggaacc     120

ttggagtggc agcacttgtg tcacatncta aaggcactcc tctgactaaa gagaagtggc     180

agctgagcac actgcctgcc cagggatttt cttctgctct aaaggacatt attaagacaa     240

ctggtctgta ctgtattgcc tgatagtatc atatcaatgt caatttcctc attttggtga     300

ttgttctaat aagaatacgt aagaatgtgt ccttgtt                              337


<210>  164
<211>  408
<212>  DNA
<213>  Homo sapiens

<400>  164
ggttccaggc tttgcatctg gagcctttac cggttgactg ttgccttcca cacaaacagc      60

ctctgaaaag cactttctcc atacataatt ctggagaaga tgagggatct tgccctccag     120

gagccttcct tcctccccca atgaggaaat cagtcactgc actggtgcaa aggcaagcag     180

attggaattt ctgctcttca ccgattttct cagggaaaga ccccttcccc ttgccagcag     240

aggaacctgt agttttttcc atttctttct tcagaaccaa agtatgtatc actcctcatg     300

ctcacaggga ttgacaggag agaattcacc aggatcttag ctcaaaagac acagcctcag     360

aatggccaga tggattgcac gaaacctgac ttggattcac catcttcc                  408


<210>  165
<211>  506
<212>  DNA
<213>  Homo sapiens


<220>
<221>  misc_feature
<222>  (147)..(147)
<223>  n is a, c, g, or t

<400>  165
cagtgtggct gttggaactc atgtcttcca acaagtcaaa gcttccattc caagtgcaga      60

aaagacctcc tcaactgatg caggtccagc tgaggaattt cctgaacctg aggaaaatgc     120

tgcaagagaa gaaaggaaat taaaagncga cacggagaaa agggaacaga aggatgaaga     180

aatccctgaa aatgcaaaca atgaaaagat gtccatggaa acactactca aagtgttcaa     240

agggggaagt gaagcacagg actccaatag atttgccagc cacctaaaga tagagaacat     300

ttatgcagag ggcttcataa aaacatttca agacctaggt gccaagaacc tggagccaat     360

tgaagtcgct gttctcttga agcatccttt tattcaagac ctgatttcaa attattcaga 420

ctataagttt cctgatatta aaataattct ccaaaggagt gaacatgtac aaggaagtga 480

tggagagaga tcaccttcaa gacata 506

<210> 166
<211> 315
<212> DNA
<213> Homo sapiens

<220>
<221> misc_feature
<222> (157)..(157)
<223> n is a, c, g, or t

<220>
<221> misc_feature
<222> (184)..(184)
<223> n is a, c, g, or t

<220>
<221> misc_feature
<222> (186)..(186)
<223> n is a, c, g, or t

<220>
<221> misc_feature
<222> (210)..(210)
<223> n is a, c, g, or t

<220>
<221> misc_feature
<222> (234)..(234)
<223> n is a, c, g, or t

<220>
<221> misc_feature
<222> (237)..(237)
<223> n is a, c, g, or t

<220>
<221> misc_feature
<222> (263)..(263)
<223> n is a, c, g, or t

<400> 166
   gcacgagcgg gctcggtggt tgggtccgcg gcggctcggg gtccgcccgc ggctgccggt 60
   gcgagcgggg cccggctccc ctcctccccc gcccgccgcc gccgctgtga ttgggtggaa 120
   gatggcgctg gccggatgga aatcctaatg acagtcncca aattcgcctc catctgtacc 180
   atgngngcca atgcttcggc attagagaan gagattggtc cagaacagtt tccngtnaat 240
   gagcactatt ttggattagt canttttggg aatacctgct actgcaattc agttcttcaa 300
   gcactttatt tttgt 315

<210>  167
<211>  468
<212>  DNA
<213>  Homo sapiens


<220>
<221>  misc_feature
<222>  (129)..(129)
<223>  n is a, c, g, or t

<400>  167
gaacaaacgt ccagcaagat actttaatcc tctattttgc tcttccttgt ccattgctgc      60

tgttaatgta ttgctgacct ctttcacagt tggctctaaa gaatcaaaag aaaaaaactt     120

tttatttcnt ttgctattaa aactactgtt cattttgggg gctgggggaa gtgagcctgt     180

ttggatgatg gatgccattc cttttgccca gttaaatgtt caccaatcat tttaactaaa     240

tactcagact tagaagtcag atgcttcatg tcacagcatt tagtttgttc aacagttgtt     300

tcttcagctt cctttgtcca gtggaaaaac atgatttact ggtctgacaa gccaaaaatg     360

ttatatctga tattaaatac ttaatgctga tttgaagaga tagctgaaac caaggctgaa     420

gactgtttta ctttcagtat tttcttttcc tcctagtgct atcattag                  468


<210>  168
<211>  447
<212>  DNA
<213>  Homo sapiens

<400>  168
acacagggaa gtgactttgc tacaaataat gttgctgtgt taagtattca tattaaatac      60

atgccttcta tatggaacat ggcagaaaga ctgaaaaata acagtaatta attgtgtaat     120

tcagaattca taccaatcag tgttgaaact caaacattgc aaaagtgggt ggcaatattc     180

agtgcttaac acttttctag cgttggtaca tctgagaaat gagtgctcag gtggatttta     240

tcctcgcaag catgttgtta taagaattgt gggtgtgcct atcataacaa ttgttttctg     300

tatcttgaaa aagtattctc cacattttaa atgttttata ttagagaatt ctttaatgca     360

cacttgtcaa atatatatat atagtaccaa tgttaccttt ttattttttg ttttagatgt     420

aagagcatgc tcatatgtta ggtactt                                         447


<210>  169
<211>  473
<212>  DNA
<213>  Homo sapiens


<220>
<221>  misc_feature
<222>  (90)..(90)
<223>  n is a, c, g, or t

<400>  169
gtgcctataa tttgccagta gccacagatt aagattatat cttatatatc agcagattag      60

ctttagctta gggggagggt gggaaagttn gggggggggg ttgtgaagat ttagggggac     120

cttgatagag aactttataa acttctttct ctttaataaa gacttgtctt acaccgtgct     180

gccattaaag gcagctgttc tagagtttca gtcacctaag tacacccaca aaacaatatg     240

aatatggaga tcttccttta cccctcaact ttaatttgcc cagttatacc tcagtgttgt     300

agcagtactg tgatacctgg cacagtgctt tgatcttacg atgccctctg tactgacctg     360

aaggagacct aagagtcctt tccctttttg agtttgaatc atagccttga tgtggtctct     420

tgttttatgt ccttgttcct aatgtaaaag tgcttaactg cttcttggtt gta            473


<210>  170
<211>  438
<212>  DNA
<213>  Homo sapiens

<400>  170
tacgaggttc ctagagctct gctgaccctt ggccgaaact ctaaaatgta cctattaaag      60

ataaatgctt ctaccaaagt aaaactctgt gagttgtttc agggcagaat gtaccagcca     120

gtcagcgttg tttaacaaaa taatcagatt tttgcctagc actcggtttt ggtggagctg     180

acgattttga gggctgaggc tggttaggta gctggaatgt gcctatgtga ccagctcact     240

tgcagacacc ctgccggaag cagagcttaa tcttcctagg actgaggtct tagcacatgt     300

actggtggag tttccagacc accagtatga ataaaagctt gttctgtgtg acccagcaag     360

tggaaggaca aagaactgtg agcctcagat ctttggacct ttccaatgcg tctctttctc     420

ctgttattgc tgcaatgt                                                   438


<210>  171
<211>  468
<212>  DNA
<213>  Homo sapiens


<220>
<221>  misc_feature
<222>  (38)..(39)
<223>  n is a, c, g, or t

<220>
<221>  misc_feature
<222>  (168)..(168)
<223>  n is a, c, g, or t

<400>  171
ggagctcgat tcacgtacag cacttctgtg gggtttgnna aaaaaaaaag gaaaacaaca      60

gaagaacaca tcatatgcaa ctaatgatct cattatttaa gagtcccctg ttacttcttt     120

agtcatttcc tttgactctg ctacagatag gattatagga tgatgctnca aaggggacct     180

tgaacctatt caccattatt tgtctcttta agctggcaaa cccatcatta aatagcacat 240

aaaatagcaa tcatatggga taagtagtac agcttcagta atcaatgggc agtggcacta 300

gaaaaatctt gagcacagtg aatgacctat cctgcaaaca tctaatggat ctctaaaggg 360

taacaaaccc tataaattct ggcttactgc acatatttag tgtgttttaa gataggatcc 420

taactgtata tatttataac taatgtaagg accctacttt attgccaa 468

<210> 172
<211> 254
<212> DNA
<213> Homo sapiens

<400> 172
tgccctccag tagagtgaga ttccgtctca aaaaaaaaaa aaaaaaagaa gaaatggaga 60

tacaaactta ctacctacct ccttacaacc taccctcaca gtattactgt gaataaaagt 120

gtgtgtagca ctgggaacac tattcacaga gcactcatga atgtttgttc tttgttatta 180

gttactagag aggcaaatgt ctgccagggc tgaataatat gtgtgaattg gtgattgtcg 240

cacatatcta aaga 254

<210> 173
<211> 483
<212> DNA
<213> Homo sapiens

<220>
<221> misc_feature
<222> (26)..(26)
<223> n is a, c, g, or t

<220>
<221> misc_feature
<222> (28)..(28)
<223> n is a, c, g, or t

<220>
<221> misc_feature
<222> (41)..(41)
<223> n is a, c, g, or t

<220>
<221> misc_feature
<222> (51)..(51)
<223> n is a, c, g, or t

<220>
<221> misc_feature
<222> (58)..(58)
<223> n is a, c, g, or t

<220>
<221> misc_feature
<222> (61)..(61)

<223>  n is a, c, g, or t

<220>
<221>  misc_feature
<222>  (83)..(83)
<223>  n is a, c, g, or t

<220>
<221>  misc_feature
<222>  (445)..(450)
<223>  n is a, c, g, or t

<400>  173
ctcagcctcc taaggagcta tttttngnat tttagtagag ncagggtttc nccgtgtngg      60

ncaggctggt ctcgaactcc tgncctcaag tgatccaccc gccttggcct cccaaagtgc     120

tgggattaca ggtgtgagcc accgtgccca gctagattct ttacttttta ataaagttca     180

aaatagcttt taataaggtt gtcaaatttg accaaaattt catagaagtc caaaatatta     240

tgcctctttc tgtagatcaa atgaaatatg gcccaattta caagggtata caccttcatg     300

gtagatgaat atagatttta aactagtttt aaaaggaata tgaatgattt aattgtagtt     360

tctgctgcca aaaagtctcc ataattattt tttaataact tataactacc ttgatcttta     420

cctgtacaca cacacacaca cacannnnnn tgcacagagt aatttcactt agaatcctgg     480

tca                                                                   483


<210>  174
<211>  516
<212>  DNA
<213>  Homo sapiens

<400>  174
ttaccctcta tttaaatgct ttgaaaaaca gtgcattgac aatgggttga tatttttctt      60

taaaagaaaa atataattat gaaagccaag ataatctgaa gcctgtttta ttttaaaact     120

ttttatgttc tgtggttgat gttgtttgtt tgtttgtttc tattttgttg gttttttact     180

ttgttttttg ttttgttttg ttttgttttg catactacat gcagttcttt aaccaatgtc     240

tgtttggcta atgtaattaa agttgttaat ttatatgagt gcatttcaac tatgtcaatg     300

gtttcttaat atttattgtg tagaagtact ggtaattttt ttatttacaa tatgtttaaa     360

gagataacag tttgatatgt tttcatgtgt ttatagcaga agttatttat ttctatggca     420

ttccagcgga tattttggtg tttgcgaggc atgcagtcaa tattttgtac agttagtgga     480

cagtattcag caacgcctga tagcttcttt ggcctt                               516


<210>  175
<211>  329
<212>  DNA
<213>  Homo sapiens

<400>  175

gcctgacgaa acctagctat tgtaagctag tttcttcagt tattcttttc cctatccctg      60

tctaagctag atatatacag agaggtccag ttttatgaat gcttattaga tggaatttta      120

taagtctaat gcagtatcag aatagcaaat atgtgtattg cccagtttaa tttactgagg      180

tcaaattatc atgggcactt ggctatcccc tcctacccat gcacttatct gtaggtgtgt      240

tatcaactga tgattttctg gagtttttga cccattacca tttaagatat acagagaatt      300

ttatggtagt ttagcccact attctgagc                                        329

<210>  176
<211>  493
<212>  DNA
<213>  Homo sapiens

<220>
<221>  misc_feature
<222>  (55)..(55)
<223>  n is a, c, g, or t

<220>
<221>  misc_feature
<222>  (57)..(57)
<223>  n is a, c, g, or t

<220>
<221>  misc_feature
<222>  (59)..(59)
<223>  n is a, c, g, or t

<220>
<221>  misc_feature
<222>  (64)..(64)
<223>  n is a, c, g, or t

<220>
<221>  misc_feature
<222>  (68)..(68)
<223>  n is a, c, g, or t

<220>
<221>  misc_feature
<222>  (70)..(70)
<223>  n is a, c, g, or t

<220>
<221>  misc_feature
<222>  (81)..(81)
<223>  n is a, c, g, or t

<400>  176
atttaagatc tagtagccac gctaaaaaac aagtaaaatt aattttaata tattnancnc      60

aatncatngn agatattatt ncagtaagat atttttcatt cttatgaaac taagtctgtc      120

tggtttgcat atctcagttg agacactaaa gttttactga taatacttaa tccatgttta      180

gatttcataa aatctaaatt tgcaaagtaa atttacatac ccatgttggt ctaaatgtgc      240

ttaaaagttt tccaataact gaaatgagta tcagttttaa atttaaaaat ttgttacttg 300

gttactccat atttcaggta ttagtggcta ccatattggt cagagcagat tcataggatg 360

atttcccagg tttgaagtgt gtgtatattt ctggtattat aagatgtcag actggttttt 420

ggagaaatat gttgtcttaa tttttacctg ctttgatatg acctttctgt gtgtacgtgt 480

gacatttctt tat 493

<210> 177
<211> 156
<212> DNA
<213> Homo sapiens

<400> 177
ggagggggag gtggagtggc agtgtgttcc acccagcagt ccctgtgttt gacctcttcg 60

tacactctca gttgtattag ctctcagaat gaaaacgaga tacttttccc caatctatgt 120

acgttgttct taagaatgta cacttggcca ggtgca 156

<210> 178
<211> 364
<212> DNA
<213> Homo sapiens

<400> 178
gatttgaggt acaccccaag tgcctggcag ctaaacacat tgccattagt gccacatcat 60

ttagcttaag gctctactaa gtcagtaatt actttagtgg cattatgagt aagtacagat 120

gaactggcag tcgcttcagg aaagggacac atggtgagtc ctgatgatgg gcagggaggg 180

aaggaaggtg gggtgtcggg ccctgcagtg gggtgtgggg gagctgcaga acttggggca 240

ctagtagggt acaccatatc aggccaggag gaaaaaggag aaatcaaagt tggggagtgg 300

tagggaagga aaagggtctg tagtgaatgg aacgtaactc ttccctattg gtgattttcc 360

tttg 364

<210> 179
<211> 519
<212> DNA
<213> Homo sapiens

<220>
<221> misc_feature
<222> (297)..(297)
<223> n is a, c, g, or t

<400> 179
ggaggtttaa tacggccaat ggatcttgta taaagtctac gtaagtttta atttaccaga 60

gctataagat ggttaaggta gactagtggg gactgctaca gataaaatga gatacaaaga 120

acatttgcaa ataaatgcca taaaattaca gtagcttgga attttagtga atcatggccc 180

ttgtgttatc tagaatgcta attattcaag ctgttcctaa acttaagtat gacatataag 240

attaaaatct tggggggaaa aactgttcag atgaaaagtc aagtcaagaa gtttccncaa 300

aagaaaagaa aaatcctaaa aaccatctag ctgttgttac attaaattta tttctcacct 360

ccattaaaag ggtttttgct ttggagtttt gttaactttc gttctttgga gtaataatat 420

ttctcttgtg tatggcgctg aatacatttg tcaataatac gtcaaaaaaa aaaacacttg 480

gcttcttaat acttggaaat acgtacatat tccttacta 519

<210> 180
<211> 341
<212> DNA
<213> Homo sapiens

<220>
<221> misc_feature
<222> (46)..(46)
<223> n is a, c, g, or t

<220>
<221> misc_feature
<222> (97)..(97)
<223> n is a, c, g, or t

<220>
<221> misc_feature
<222> (116)..(116)
<223> n is a, c, g, or t

<220>
<221> misc_feature
<222> (128)..(128)
<223> n is a, c, g, or t

<220>
<221> misc_feature
<222> (132)..(132)
<223> n is a, c, g, or t

<220>
<221> misc_feature
<222> (172)..(172)
<223> n is a, c, g, or t

<220>
<221> misc_feature
<222> (174)..(174)
<223> n is a, c, g, or t

<220>
<221> misc_feature
<222> (211)..(211)
<223> n is a, c, g, or t

<220>
<221> misc_feature
<222> (227)..(227)
<223> n is a, c, g, or t

<220>
<221>  misc_feature
<222>  (229)..(229)
<223>  n is a, c, g, or t

<400>  180
ccccatgggt agaaccttcc tgattggctg tgggttgtat caaaanacct ttgactgggt      60

gaggctgaga tgggtgtggt ggttccagtg agtcagncca ggttcccttc cccagnccct     120

ggtccctnag gnagatgagg accgaaaggc ttaggtgact tgatggggag cncnctttca     180

ggggccctgg aagaaatgtt tgtaattgtt ntgctttttc catgagntnt ggagagcacc     240

caccaccaag ccccctgaaa cccaccagtc cctacagcct ggtctttctc cccaccttcc     300

cactcccaaa cacatttcca cagcaaactc cttgggaagg g                         341


<210>  181
<211>  523
<212>  DNA
<213>  Homo sapiens

<400>  181
ccttcaactg gaataatttc acttcagaaa aaaaagttaa gcatttccaa aacaaataaa      60

agttgagtgt gttactaacc actggaacag tgctaaagga aatgtaaaag aaagtctatc     120

acgtccaaaa atgacaatat atgctgcaca gcgtcataaa agcatatgaa aatagaaagc     180

tctctattaa aggtaaatac ataaacagct atagaaacct ctactgtcat aatgatggtg     240

cacaaaactt tcacgttatt gctatggaat ttaaaaaatg aagcagaaag ctgcataaat     300

atgggttcat agatactcaa taagaaaaga taacaagtga tattaataac aaagtggggg     360

tataaagagg taaagctttg cattccattg aaatatagtc attatatatt gtcataactt     420

taagatgttt tatgaagtct ttatttctca agatgattac aaaaaaacct gtagatggta     480

tgcagaagca aatgagaaag aaattgaatc atgtcactac aaa                       523


<210>  182
<211>  255
<212>  DNA
<213>  Homo sapiens


<220>
<221>  misc_feature
<222>  (59)..(59)
<223>  n is a, c, g, or t

<400>  182
gcatagttaa gtaaatcacc ccaatttttt ctaatattta agtgaacatt taaagatang      60

catttcttaa tgttaatttc ttttctttca tgtaatcttg tcagtagaca aatgatggtg     120

actatattct gtaataagta catatattat gattaaaaaa aaaagcgtgt actatctgcc     180

caaagagatc ataagcactt tggagctata aacttcttgt ctatttctac ttccatggta 240

ctgatattct cttct 255

## Description

### TECHNICAL FIELD

The present invention relates to markers for detecting human follicular helper T cells. The present invention also relates to methods for detecting human follicular helper T cells using these markers.

### BACKGROUND ART

Follicular helper T cells (hereinafter also referred to as "Tfh cells") were discovered in follicular germinal center of secondary lymphoid tissue such as lymph node and spleen as helper T cells which activate B cells and promote antibody production. Tfh cells are also known as cells which express a chemokine receptor CXCR5 (chemokine (C-X-C motif) receptor 5) and secrete a cytokine IL-21 (interleukin 21) (see Non-Patent Literatures 1 and 2).

Tfh cells are generally useful for prevention of infection. It is believed however that, in autoimmune diseases such as systemic lupus erythematosus (SLE), Tfh cells provide inappropriate activation signal to autoreactive B cells to allow production of autoantibodies (see King C. et al., *supra*)*.* There are some reports on that, in autoimmune diseases such as SLE and Sjogren's syndrome, Tfh cells may be exuded from lymphatic tissue to peripheral blood. Namely, the result obtained by measuring Tfh cells may be used for diagnoses and examinations of diseases associated with Tfh cells such as autoimmune diseases, e.g. SLE or Sjogren's syndrome.

One of the methods for detecting Tfh cells utilizes marker molecules which are specifically expressed in Tfh cells. For example, Chtanova T. et al. (Non-Patent Literature 3) found genes specifically expressed in Tfh cells by carrying out gene expression assay of Tfh cells isolated from human tonsil, cultured Th1 and Th2 cells from human umbilical cord blood and memory T cells (effector memory T cells (T_{EM}) and central memory T cells (T_{CM})) isolated from human peripheral blood. WO 2010/003002 (Patent Literature 1) discloses that genes specifically expressed in Tfh cells were found by gene expression assay of Tfh cells isolated from murine splenocytes and cultured Th1, Th2 and The17 cells.

### Citation List

### Patent Literature

Patent Literature 1: WO 2010/003002

### Non-Patent Literature

Non-Patent Literature 1: King C. et al., "T follicular helper (TFH) cells in normal and dysregulated immune responses", Annu. Rev. Immunol., vol.26, p.741-766 (2008)
Non-Patent Literature 2: Spolski R. et al., "IL-21 and T follicular helper cells", Int. Immunol., vol.22, p.7-12 (2009)
Non-Patent Literature 3: Chtanova T. et al., "T follicular cells express a distinctive transcriptional profile, reflecting their role as non-Th1/Th2 effector cells that provide help for B cells", J. Immunol., vol. 173, p.68-78 (2004)

### SUMMARY OF INVENTION

### Technical Problem

Chtanova T. et al. compared Th1, Th2 cells and memory T cells with Tfh cells in the gene expression assay and did not compare Tfh cells with other Th cells (for example, Th9, Th 17 and Th22 cells) (see Chtanova T. et al., *supra*)*.* The genes described in WO 2010/003002 as being expressed in Tfh cells may have different gene expression profile from human Tfh cells as they were identified from murine Tfh cells. In the gene expression assay carried out in this document, Th1, Th2 cells and Th 17 cells were compared with Tfh cells but Tfh cells were not compared with other Th cells (for example, Th9 and Th22 cells).

Thus the present inventors aimed to identify genes specifically expressed in human Tfh cells compared with other Th cells (Th1, Th2, Th9, Th17, Th22 and Treg cells) by using Tfh cells isolated from human peripheral blood and cultured under optimal conditions and to provide the identified genes as new molecular markers for allowing detection of human Tfh cells.

### Solution to Problem

The present inventors first isolated Tfh cells from peripheral blood of healthy adults and cultured the cells. The present inventors then identified the genes specifically expressed in the obtained Tfh cells to complete the present invention.

Thus the present invention provides a polynucleotide marker for detecting human Tfh cells, which is a polynucleotide having a base sequence of at least one gene selected from:
a gene encoding a membrane protein represented by CD9 (CD9 molecule), TM4SF1 (transmembrane 4 L six family member 1), IL23R (interleukin 23 receptor), ART3 (ADP-ribosyltransferase 3), ELOVL7 (ELOVL family member 7, elongation of long chain fatty acids), KCNS3 (potassium voltage-gated channel, delayed-rectifier, subfamily S, member 3), LHFP (lipoma HMGIC fusion partner), PAWR (PRKC, apoptosis, WT1, regulator), THBS1 (thrombospondin 1), B4GALT6 (UDP-Gal:betaGlcNAc beta 1,4- galactosyltransferase, polypeptide 6), C3orf52 (chromosome 3 open reading frame 52), CD28 (CD28 molecule), CDH3 (cadherin 3, type 1, P-cadherin), CLIC2 (chloride intracellular channel 2), EMP2 (epithelial membrane protein 2), EPHA4 (EPH receptor A4), FAM26F (Family with sequence similarity 26, member F), FCGR1B (Fc fragment of IgG, high affinity Ib, receptor (CD64)), FLT1 (fms-related tyrosine kinase 1), FUT7 (fucosyltransferase 7 (alpha (1,3) fucosyltransferase)), GABBR1 (gamma-aminobutyric acid (GABA) B receptor, 1), UBD (ubiquitin D), GAP43 (growth associated protein 43), GPC4 (glypican 4), GPR26 (G protein-coupled receptor 26), GPR84 (G protein-coupled receptor 84), IL12RB2 (interleukin 12 receptor, beta 2), KIAA1244 (KIAA1244), KISS1R (KISS1 receptor), KITLG (KIT ligand), LST1 (leukocyte specific transcript 1), MAP1B (microtubule-associated protein 1B), MARS (methionyl-tRNA synthetase), NTRK3 (neurotrophic tyrosine kinase, receptor, type 3), PLSCR1 (phospholipid scramblase 1), PPP2CB (protein phosphatase 2 (formerly 2A), catalytic subunit, beta isoform), RHOU (ras homolog gene family, member U), SGMS2 (sphingomyelin synthase 2), SLC35E4 (solute carrier family 35, member E4), SORBS1 (sorbin and SH3 domain containing 1), TAC1 (tachykinin, precursor 1), TMCC2 (transmembrane and coiled-coil domain family 2), TMEM213 (transmembrane protein 213), TMTC (transmembrane and tetratricopeptide repeat containing 1) or TUSC3(tumor suppressor candidate 3);

a gene encoding an extracellular/secretory protein represented by APOD (apolipoprotein D), CXCL9 (chemokine (C-X-C motif) ligand 9), CXCL10 (chemokine (C-X-C motif) ligand 10), CXCL11 (chemokine (C-X-C motif) ligand 11), FGF2 (fibroblast growth factor 2 (basic)), IL2 (interleukin 2), PTHLH (parathyroid hormone-like hormone), SERPING1 (serpin peptidase inhibitor, clade G (C1 inhibitor), member 1), CCL18 (chemokine (C-C motif) ligand 18 (pulmonary and activation-regulated)), CCL2 (chemokine (C-C motif) ligand 2), COL6A2 (collagen, type VI, alpha 2), CSF2 (colony stimulating factor 2), FGF1 (fibroblast growth factor 1 (acidic)), FGF18 (fibroblast growth factor 18), IFNB1 (interferon, beta 1, fibroblast), IL8 (interleukin 8), INHBA (inhibin, beta A), MDK (midkine (neurite growth-promoting factor 2)), MMP12 (matrix metallopeptidase 12 (macrophage elastase)), PPIA (peptidylprolyl isomerase A (cyclophilin A)), PRG4 (proteoglycan 4), SPARC (secreted protein, acidic, cysteine-rich (osteonectin)), STC2 (stanniocalcin 2) or TNFRSF11B (tumor necrosis factor receptor superfamily, member 11b);

a gene encoding an intracellular protein represented by ANXA3 (annexin A3), DGKI (diacylglycerol kinase, iota), EFS (embryonal Fyn-associated substrate), MYH6 (myosin, heavy chain 6, cardiac muscle, alpha), MYH7 (myosin, heavy chain 7, cardiac muscle, beta), MYO5B (myosin VB), PCGF2 (polycomb group ring finger 2), PNMA2 (paraneoplastic antigen MA2), RGS20 (regulator of G-protein signaling 20), ALDH1L2 (aldehyde dehydrogenase 1 family, member L2), BSPRY (B-box and SPRY domain containing), C10orf10 (chromosome 10 open reading frame 10), CHMP4C (chromatin modifying protein 4C), DPYSL4 (dihydropyrimidinase-like 4), ELL (elongation factor RNA polymerase II), FBXO17 (F-box protein 17), SARS2 (seryl-tRNA synthetase 2, mitochondrial), FERMT2 (fermitin family homolog 2), GBP4 (guanylate binding protein 4), HIST1H1A (histone cluster 1, H1a), INSC (inscuteable homolog), IRF4 (interferon regulatory factor 4), KLF4 (Kruppel-like factor 4 (gut)), MAST4 (microtubule associated serine/threonine kinase family member 4), METTL3 (methyltransferase like 3), MGST1 (microsomal glutathione S-transferase 1), NEXN (nexilin (F actin binding protein)), PHLDA2 (pleckstrin homology-like domain, family A, member 2), PLOD2 (procollagen-lysine, 2-oxoglutarate 5-dioxygenase 2), POLR1C (polymerase (RNA) I polypeptide C, 30kDa), RAI 14 (retinoic acid induced 14), RPL 10 (ribosomal protein L10), RTKN (rhotekin), S 100A9 (S100 calcium binding protein A9), SOCS3 (suppressor of cytokine signaling 3), SPHK1 (sphingosine kinase 1), STAG3 (stromalantigen 3), TEAD4 (TEA domain family member 4), TOM1L1 (target of myb1-like 1), UCHL1 (ubiquitin carboxyl-terminal esterase L1 (ubiquitin thiolesterase)), VPS53 (vacuolar protein sorting 53 homolog), WDR74 (WD repeat domain 74), XAF1 (XIAP associated factor 1), ZNF334 (zinc finger protein 334) or ZNF503 (zinc finger protein 503);

a gene represented by DNAJC12 (DnaJ (Hsp40) homolog, subfamily C, member 12), DOK5 (docking protein 5), LOC400043 (hypothetical LOC400043), MYO1B (myosin IB), SPEF2 (sperm flagellar 2), YAP1 (Yes-associated protein 1, 65kDa), C8orf47 (chromosome 8 open reading frame 47), CA13 (carbonic anhydrase XIII), CCDC77 (coiled-coil domain containing 77), CT45A1 (cancer/testis antigen family 45, member A1), CT45A2 (cancer/testis antigen family 45, member A2), CT45A3 (cancer/testis antigen family45, member A3), CT45A4 (cancer/testis antigen family 45, member A4), CT45A5 (cancer/testis antigen family 45, member A5), CT45A6 (cancer/testis antigen family 45, member A6), LOC100133581 (hypothetical protein LOC100133581), EFR3B (EFR3 homolog B), FLJ31958 (hypothetical LOC143153), GADD45B (growth arrest and DNA-damage-inducible, beta), GAS5 (growth arrest-specific 5 (non-protein coding)), KIAA0895 (KIAA0895), LM04 (LIM domain only 4), LOC339751 (hypothetical protein LOC339751), LOC340184 (hypothetical protein LOC340184), MAK (male germ cell-associated kinase), MIR155HG (MIR155 host gene (non-protein coding)), NAPSB (napsin B aspartic peptidase pseudogene), NPHP1 (nephronophthisis 1), OSBPL6 (oxysterol binding protein-like 6), RASSF4 (Ras association (RalGDS/AF-6) domain family member 4), RIBC2 (RIB43A domain with coiled-coils 2), RIMKLA (ribosomal modification protein rimK-like family member A), SH3TC2 (SH3 domain and tetratricopeptide repeats 2), USP12 (ubiquitin specific peptidase 12) or ZBED2 (zinc finger, BED-type containing 2); and
a gene consisting of a base sequence represented by any of SEQ ID NOs: 171, 182 and 172 to 181;
or a variant or fragment thereof.

The present invention also provides a protein marker for detecting Tfh cells, which is a protein encoded by at least one gene described above or a functionally equivalent variant or fragment thereof.
The present invention further provides a method for detecting human Tfh cells comprising detecting at least one polynucleotide marker for detecting human Tfh cells or the protein marker for detecting human Tfh cells described above in a sample containing cells.

### Advantageous Effects of Invention

Detection of at least one polynucleotide marker or protein marker for detecting human Tfh cells of the present invention allows specific detection of human Tfh cells.

### BRIEF DESCRIPTION OF DRAWINGS

Fig. 1 is histograms (particle distribution) showing the percentage of CD9 positive cells within human peripheral blood-derived cultured Th1, Th2 and Tfh cells; and
Fig. 2 is a graph showing enrichment ratio of Tfh cells in the CXCR5 fractionated sample and the CXCR5/CD9 fractionated sample.

### DESCRIPTION OF EMBODIMENTS

The polynucleotide marker for detecting human Tfh cells of the present invention (hereinafter also referred to as "the present polynucleotide marker") is a polynucleotide having the base sequence of at least one gene selected from the above genes or a variant or fragment thereof.
The present polynucleotide marker is a polynucleotide which has been found to be specifically present in Tfh cells compared to other helper T cells derived from human peripheral blood (Th1, Th2, Th9, Th17 and Th22 cells and regulatory T (Treg) cells) or a variant or fragment thereof.

The present invention also encompasses the use of a polynucleotide having the base sequence of at least one gene selected from the above genes or a variant or fragment thereof, as a marker for detecting human Tfh cells.

The present polynucleotide marker is preferably a polynucleotide having the base sequence of at least one gene selected from:
a gene encoding a membrane protein represented by CD9, TM4SF1, IL23R, ART3, ELOVL7, KCNS3, LHFP, PAWR or THBS1;
a gene encoding an extracellular/secretory protein represented by APOD, CXCL9, CXCL10, CXCL11, FGF2, IL2, PTHLH or SERPING1;
a gene encoding an intracellular protein represented by ANXA3, DGKI, EFS, MYH6, MYH7, MYO5B, PCGF2, PNMA2 or RGS20;
a gene represented by DNAJC12, DOK5, LOC400043, MYO1D, SPEF2 or YAP1; and
a gene consisting of the base sequence represented by SEQ ID NO: 171 or 182;
or a variant or fragment thereof.

More preferably, the present polynucleotide marker is a polynucleotide having the base sequence of the gene encoding CD9 or a variant or fragment thereof (hereinafter referred to as "polynucleotide marker for CD9").

As used herein, the term "gene" has the equivalent meaning as the term generally used in the art and refers to a part of a genome which is to be transcribed to mRNA and translated to a protein.

As used herein, the gene consisting of the base sequence represented by any of SEQ ID NOs: 171, 182 and 172 to 181 is a gene from which the base sequence represented by any of these SEQ ID NOs or mRNA containing a complementary base sequence thereof is transcribed. Namely, the gene consisting of the base sequence represented by any of SEQ ID NOs: 171, 182 and 172 to 181 includes a gene consisting of a complementary base sequence of the base sequence represented by any of SEQ ID NOs: 171, 182 and 172 to 181.

As used herein, a membrane protein means a protein contained in a membrane fraction of a cell. An extracellular/ secretory protein means a protein synthesized intracellularly and located at outside of a cell membrane or secreted to outside of a cell membrane. An intracellular protein means a protein which is mainly located in a cell.

As used herein, the phrase "a polynucleotide is "specifically expressed" in Tfh cells" means that the expression of the polynucleotide in Tfh cells is significantly higher than the expression of the polynucleotide in cells other than Tfh cells.
Specifically, the above phrase means that the expression of the polynucleotide in Tfh cells is about three times or more of the expression of the polynucleotide in cells other than Tfh cells. Preferably, the expression of the polynucleotide in Tfh cells is about three times or more of the expression of the polynucleotide in helper T cells other than Tfh cells (Th1 cells, Th2 cells, Th9 cells, Th17 cells, Th22 and Treg cells).

The base sequences of the present polynucleotide markers per se are already known. These can be obtained from, for example, UniGene (a database provided by National Center for Biotechnology Information: NCBI). Entrez gene IDs, UniGene IDs, Protein IDs and Transcript IDs of the base sequences of the present polynucleotide markers are shown in Table 16.
The "Probe set ID" is an identification number for specifying a probe set for gene identification used in GeneChip® of Affymetrix. The Probe set IDs shown in Table 16 of the present specification are the identification numbers for probe sets mounted on the "Human Genome U133 Plus 2.0" array. The target sequences of the Probe sets are represented by SEQ ID NOs: 1 to 182.

As used herein, a "variant" of a polynucleotide means a polynucleotide containing a mutation which does not change the nature of the protein encoded by the gene. The mutation includes deletion or substitution of one or more nucleotides from or addition of one or more nucleotides to the base sequence of the above gene.

The variant has a sequence identity of generally at least 80%, preferably at least 85%, more preferably at least about 90%, still more preferably at least 95% with the base sequence of the above gene.
As used herein, the sequence identity of a base sequence and an amino acid sequence means the value calculated by using BLASTN, BLASTP, BLASTX or TBLASTN (for example, available at http://www.ncbi.nlm.nih.gov) with the default settings.

As used herein, a "fragment" of a polynucleotide means a polynucleotide having a contiguous partial sequence of the base sequence of the above gene and having a length which allows specific hybridization thereof with a probe for detecting human Tfh cells described hereinbelow.

The present polynucleotide marker may be either DNA or RNA, or any of the gene per se (DNA), mRNA, cDNA and cRNA.

It is also possible to detect human Tfh cells by detecting at least one protein encoded by the gene as the present polynucleotide marker. Thus the present invention also encompasses the protein encoded by at least one gene described above. Namely, the protein marker for detecting human Tfh cells of the present invention (hereinafter also referred to as the "present protein marker") is a protein encoded by at least one gene selected from the above genes or a functionally equivalent variant or fragment thereof.

Amino acid sequences of the protein markers can be obtained based on the base sequences of the polynucleotide markers retrieved from UniGene described above and the like. The amino acid sequences can also be obtained from databases provided by NCBI or the like as described above. The Protein ID numbers from NCBI for the amino acid sequences of the present protein markers are shown in Table 16.

A functionally equivalent variant of a protein means a protein containing a mutation which does not change the function of the protein. The mutation includes deletion or substitution of one or more amino acids from or addition of one or more amino acids to the known amino acid sequence of the above protein.
The functionally equivalent variant of the protein has a sequence identity of generally at least 80%, preferably at least 85%, more preferably at least about 90%, still more preferably at least 95% with the known amino acid sequence of the above protein.
A fragment of a protein means a polypeptide having a contiguous partial sequence of the known amino acid sequence of the above protein and having a length which allows specific recognition by an antibody or nucleic acid aptamer for detecting human Tfh cells described hereinbelow.

In a preferred embodiment of the present invention, the present protein marker is a protein having an amino acid sequence of CD9 or a functionally equivalent variant or fragment thereof (hereinafter referred to as the "protein marker for CD9").

The present invention also encompasses the use of a protein encoded by the gene as the present polynucleotide marker or a functionally equivalent variant or fragment thereof, as a marker for detecting human Tfh cells.

A molecule which can specifically hybridize to the present polynucleotide marker is useful as a probe for detecting human Tfh cells because it can be used for detecting the marker. The probe may be any of nucleic acid probes such as DNA or RNA and peptide probes which can specifically hybridize to the present polynucleotide marker. The probe for detecting human Tfh cells is particularly preferably a nucleic acid probe, particularly a DNA probe for detecting the polynucleotide marker.

As used herein, the phrase "a molecule "can specifically hybridize"" means the molecule can hybridize to a target nucleic acid molecule (the above polynucleotide marker) under the stringent condition.
As used herein, the stringent condition means a condition which allows hybridization of the probe for detecting human Tfh cells to the target polynucleotide marker with an extent detectably higher than the hybridization thereof to a polynucleotide other than the target polynucleotide marker (for example, at least two times higher than the background).
The stringent condition usually varies depending on sequences and various environments. Generally, the stringent condition is selected so that it is about 5°C lower than a thermal melting point (Tm) of a predetermined sequence under a predetermined ionic strength and pH. The Tm is a temperature at which 50% of probes complementary to the base sequence of the target nucleic acid molecule hybridize in equilibrium to the target nucleic acid molecules (under a predetermined ionic strength, pH and nucleic acid composition).

The stringent condition may be a condition which is used for hybridization between polynucleotides in well known hybridization methods in the art between polynucleotides such as PCR, microarray, Southern blotting and the like.
Specifically, the stringent condition may include a Na ion concentration (or other salt) of lower than about 1.5 M, more specifically 0.01 to 1.0 M at pH 7.0 to 9.0 and at least about 30°C. For example, the stringent condition in microarray includes hybridization in 50% formamide, 1 M NaCl and 1% SDS at 37°C and washing in 0.1 x SSC at 60 to 65°C.
The stringent condition in PCR may include pH 7.0 to 9.0, 0.01 to 0.1 M of Tris HCl, a K ion concentration (or other salt) of 0.05 to 0.15 M with at least about 55°C.

The sequence of the nucleic acid probe for detecting human Tfh cells can be appropriately selected by a person skilled in the art based on the common technical knowledge in the art and the base sequence of the present polynucleotide marker, so that the probe can specifically hybridize to the marker.
Such a sequence can be determined by, for example, using a conventionally available primer design software (for example, available from Primer3 (http://frodo.wi.mit.edu/cgi-bin/primer3/primer3.cgi)) or DNASISPro (Hitachi Software Engineering Co., Ltd.).

The nucleic acid probe for detecting human Tfh cells can be prepared by well known polynucleotide synthesis methods in the art.

The nucleic acid probe for detecting human Tfh cells may be labeled with a labeling substance which is conventionally used in the art. By using the labeled probe, the present polynucleotide marker, and thus human Tfh cells can be conveniently detected.
The labeling substance may be a labeling substance conventionally used in the art including a radioactive isotope such as ³²P, a fluorescent material such as fluorescein, an enzyme such as alkaline phosphatase or horse radish peroxidase, biotin or the like.

Human Tfh cells can be specifically detected by using the nucleic acid probe for detecting human Tfh cells alone or two or more probes in combination. For example, one or more probes may be immobilized on a substrate using the well known method in the art to prepare a DNA chip or microarray in order to detect the polynucleotide marker(s) for detecting human Tfh cells.
The nucleic acid probe for detecting human Tfh cells may be a primer set of two or more primers for amplifying the polynucleotide marker by PCR.

A molecule which can specifically bind to the present protein marker is useful for detection of human Tfh cells because it can be used for detecting the marker. The molecule may be any of nucleic acid aptamers such as DNA or RNA and antibodies, preferably antibodies which can specifically bind to the protein marker.
When the present protein marker is an enzyme, the marker can be detected by carrying out the reaction between the enzyme and a corresponding substrate to develop color, luminescence, fluorescence and the like.

The antibody for detecting human Tfh cells described above can be prepared by well known procedures as described below, for example. A DNA molecule encoding a protein having the amino acid sequence of the present protein marker, which is based on the base sequence of the gene as the present polynucleotide marker or the amino acid sequence of the present protein marker, is integrated into an appropriate expression vector. The obtained expression vector is introduced into an appropriate host cell and the obtained transformant is cultured to obtain the protein of interest. The obtained protein is purified and used as an immunogen to immunize an appropriate mammal such as rat or mouse optionally with an adjuvant. An antibody producing cell which produces an antibody directed to the immunogen of interest is screened from spleen cells of the immunized animal. The obtained antibody producing cell is fused with a myeloma cell to obtain a hybridoma of which screening gives an antibody producing hybridoma which produces an antibody specifically binding to the protein encoded by the gene. The antibody of interest can be obtained by culturing the obtained antibody producing hybridoma.

The nucleic acid aptamer which can be used for detecting human Tfh cells can be prepared according to well known procedures in the art as described below, for example. A nucleic acid library containing base sequences of random nucleic acid molecules is prepared according to the well known technique, which is then subjected to a technique such as systematic evolution of ligands by exponential enrichment (SELEX) and the like to select a nucleic acid aptamer specifically binding to the protein of interest (present protein marker).

The molecule which can specifically bind to the protein marker for detecting human Tfh cells may be labeled with a labeling substance which is conventionally used in the art. For example, by using the labeled antibody for detecting human Tfh cells, the protein marker for detecting human Tfh cells, and thus human Tfh cells can be conveniently detected.
The labeling substance may be a labeling substance conventionally used in the art including a radioactive isotope such as ³²P, a fluorescent material such as fluorescein, an enzyme such as alkaline phosphatase or horse radish peroxidase, biotin or the like.

The present polynucleotide marker and protein marker may be used in combination with a molecule which is known in the art that it is specifically expressed in Tfh cells, in order to detect Tfh cells. Such a known molecule may include, for example, CXCR5, IL-21 and the like.
Accordingly, in an embodiment of the present invention, a combination of the polynucleotide marker for CD9 and a polynucleotide having the base sequence of the gene encoding CXCR5 or a variant or fragment thereof can be used as the polynucleotide marker for detecting human Tfh cells. In another embodiment of the present invention, a combination of the protein marker for CD9 and a protein having the amino acid sequence of CXCR5 or a functionally equivalent variant or fragment thereof can be used as the protein marker for detecting human Tfh cells.
The above combinations allow detection of Tfh cells with higher accuracy than detection thereof with a sole molecule which is known to be specifically expressed in Tfh cells.

The present invention also encompasses a method for detecting human Tfh cells (hereinafter also referred to as the present method) by detecting at least one polynucleotide marker for detecting human Tfh cells or protein marker for detecting human Tfh cells according to the present invention in a sample containing cells.
In an embodiment of the present method, it is preferable to detect human Tfh cells by detecting the polynucleotide marker or protein marker for CD9.
In another embodiment of the present method, it is preferable to detect more than one polynucleotide marker or protein marker for detecting human Tfh cells in order to improve the detection sensitivity.
In the present method, human Tfh cells in a sample may be detected by detecting a combination of the present polynucleotide marker or protein marker and a molecule which is known to be specifically expressed in Tfh cells as the marker for detecting human Tfh cells. Such a combination is preferably a combination of CD9 and CXCR5 described above.

In the present method, a sample containing cells may include a biological sample obtained from human and a sample containing cultured cells. The biological sample may include blood, tissue, synovial fluid, cerebrospinal fluid, pleural fluid, ascites and the like.

An embodiment of the method for detecting the presence of the present polynucleotide marker is described hereinbelow.
A sample containing cells is processed by the well known method in the art which uses phenol extraction and ethanol precipitation or a commercially available DNA extraction kit to extract nucleic acid molecules (DNAs or RNAs).
The obtained nucleic acid sample is detected for the presence of the present polynucleotide marker. This detection is carried out preferably with the nucleic acid probe for detecting human Tfh cells described above.
The present polynucleotide marker can be detected by the well known method in the art including nucleic acid amplification methods such as PCR, RT-PCR, real time PCR, LAMP (Loop-mediated isothermal amplification) and the like; hybridization methods such as Southern hybridization, Northern hybridization, FISH (fluorescence *in situ* hybridization) and the like; and DNA chip and microarray methods. Such a method is carried out under the stringent condition described above and hybridization of the nucleic acid probe for detecting human Tfh cells can be detected by detecting, for example, the labeling substance described above to detect the presence of the present polynucleotide marker.

An embodiment of the method for detecting the protein marker for detecting human Tfh cells is described hereinbelow.
When, for example, the protein marker to be detected is an intracellular protein, protein is extracted from cells by the well known method in the art. Protein can be extracted from cells by the well known method in the art such as ultrasonic cell homogenization, solubilization of cells with a cell solubilizing solution and the like. The present protein marker in the protein sample can be detected with the molecule which specifically binds to the protein marker. Specifically, the protein marker can be detected by the well known method in the art such as ELISA, Western blotting and the like. In this detection, it is preferable that the antibody for detecting human Tfh cells described above is used as the molecule which specifically binds to the present protein marker.

When, for example, the present protein marker to be detected is a secretory protein, the protein marker secreted in a sample containing cells can be detected with the molecule which specifically binds to the protein marker.
Alternatively, after collecting cells (lymphocytes) from a sample containing cells and stimulating the cells with an anti-CD3 antibody, anti-CD28 antibody, concanavalin A, phytohemagglutinin (PHA), phorbol myristate acetate (PMA), ionomycin and the like, the protein marker secreted from the cells can be detected with the molecule which specifically binds to the protein marker.
Specifically, the present protein marker can be detected by the well known method in the art such as ELISA, Western blotting and the like. In this detection, it is preferable that the antibody for detecting human Tfh cells described above is used as the molecule which specifically binds to the present protein marker.

When, for example, the present protein marker to be detected is a protein existing on cell surface (cell membrane), the protein marker existing on the cell surface in a sample containing cells can be detected with the molecule which specifically binds to the protein marker.
Alternatively, after collecting a membrane fraction of cells from a sample containing cells, the present protein marker in the obtained membrane fraction can be detected with the molecule which specifically binds to the protein marker. Specifically, the protein marker can be detected by the well known method in the art such as ELISA, Western blotting, a method based on flow cytometry (FCM) and the like.
In this detection, it is preferable that the antibody for detecting human Tfh cells described above is used as the molecule which specifically binds to the present protein marker.

When, for example, the present protein marker is detected by FCM, the following procedures may be carried out.
A sample containing cells is first contacted with the antibody for detecting human Tfh cells labeled with an appropriate labeling substance. When exist, human Tfh cells bind to the labeled antibody on the cell surface. By passing the sample containing cells bound to the labeling substance through a flow cytometer, human Tfh cells can be detected. Optionally, the human Tfh cells bound to the labeling substance can be sorted and fractionated with a cell sorter.
The method based on FCM per se is well known to a person skilled in the art and he/she can appropriately choose the reaction conditions.

As described above, human Tfh cells can be specifically detected by detecting at least one polynucleotide marker or protein marker for detecting human Tfh cells according to the present embodiment. Thus, for example, detection of the above marker in a sample containing cells such as tissue collected from a subject allows specific detection of Tfh cells in the sample. By using the above marker, Tfh cells can be isolated from a sample containing various cells.
It is further possible that the marker for detecting human Tfh cells may be used for diagnoses and/or examinations of patients for diseases which Tfh cells are believed to be involved such as autoimmune diseases including SLE, Sjogren's syndrome and the like. Thus, the present invention also encompasses a marker for diagnosis and /or examination of a patient for a disease which Tfh cells are believed to be involved which is the polynucleotide marker or protein marker for detecting human Tfh cells and the use thereof. The present invention also encompasses a method for diagnosing and/or examining a subject for a disease which Tfh cells are believed to be involved by using the marker.

### Examples

The present invention is described in more detail by way of Examples which do not limit the present invention.

Example 1: Analysis of highly expressed genes in human peripheral blood-derived cultured Tfh cells

### 1. Isolation of naive CD4 positive T cells from human peripheral blood

Buffy coats obtained from peripheral blood of healthy adults were layered on Ficoll-paque plus solution (GE Healthcare Biosciences) and centrifuged to obtain the monocyte fraction. The CD4 positive cells were partially purified from the fraction by using magnetic beads (Miltenyi Biotec) coupled with an anti-CD4 antibody.
The thus obtained CD4 positive cells were stained with fluorescence-labeled antibodies shown in Table 1 prior to isolate CD4⁺CD25-CD45RA⁺CD45RO- naive CD4 positive T cells (hereinafter also referred to as "naive CD4 positive T cells") with a cell sorter (FACS Aria: Becton Dickinson).

**Table 1**

| Antigen | Fluorescence labeling substance | Clone | Manufacturer |
|---|---|---|---|
| CD4 | FITC | OKT4 | BioLegend |
| CD25 | PE-Cy7 | BC96 | eBioscience |
| CD45RO | PE | UCHL1 | BioLegend |
| CD45RA | APC | HI100 | BioLegend |

### 2. Differentiation culture of Tfh and Th9 cells from naïve CD4 positive T cells

The residual adult peripheral blood-derived naïve CD4 positive T cells obtained in the above step 1. were seeded in a 96-well plate at a density of 1.0 x 10⁵ cells/0.3 ml/well. The medium used was the Yssel medium.
In order to allow activation and proliferation of the above cells, antibody beads were added to each well at 0.5 x 10⁵. The cells were added with cytokines and neutralizing antibodies suitable for respective differentiation culture of Tfh cells and Th9 cells (see Table 2) and cultured in an incubator at 37°C and 5% CO₂. The concentration of all cytokines used is 10 ng/ml and the concentration of all neutralizing antibodies used is 2.5 µg/ml. The cytokines and neutralizing antibodies used were purchased from R&D Systems and Biolegend.

**Table 2**

| **Cell** | **Cytokine** | **Neutralizing antibody (clone)** |
|---|---|---|
| Th9 | TGF-β1, IL-4, IL-2 | **Anti** IFN- *γ* **antibody** (R4-6A2) |
| Tfh | TGF-β1, IL-12, IL-2 | **Anti** IL-4**antibody** (MP4-25D2), **Anti** IFN- *γ* **antibody** (R4-6A2), **Anti** IL-6 **antibody** (MQ2-13A5) |

| | | |
|---|---|---|
| **Cytokines are from R&D Systems and all neutralizing antibodies are from Biolegend.** | | |

After three days from the initiation of culture, the cells were diluted by three times with the medium containing the above cytokines and antibodies and cultured for further 4 days (7 days in total) to obtain Tfh cells and Th9 cells (Tfh cells obtained in this step are hereinafter referred to as "Tfh cells (1)").
The obtained Tfh cells (1) and Th9 cells were divided into two portions, each one of which was washed with the Yssel medium and PBS followed by collection of the cells by centrifugation and freezing at -80°C for storage of the cells until the next RNA extraction step. These cells are designated as Tfh cells (1) and Th9 cells "without activation stimulation", respectively. The other portion of the cells were respectively added with the antibody beads described above and cultured for further 3 hours to re-activate the cells. The cells were then recovered by centrifugation and frozen for storage at -80°C in a similar manner. These cells were designated as Tfh cells (1) and Th9 cells "with activation stimulation", respectively.

### 3. Isolation of Tfh cells (2) from human peripheral blood

Buffy coats obtained from peripheral blood of healthy adults were layered on Ficoll-paque plus solution (GE Healthcare Biosciences) and centrifuged to obtain the monocyte fraction. The CD4 positive cells were partially purified from the fraction by using magnetic beads (Miltenyi Biotec) coupled with an anti-CD4 antibody. The thus obtained CD4 positive cells were stained with fluorescence-labeled antibodies shown in Table 3 prior to isolate Tfh cells with a cell sorter (FACS Aria: Becton Dickinson) (the Tfh cells obtained in this step are hereinafter referred to as "Tfh cells (2)"). The settings of gating for isolation are shown in Table 4.

**Table 3**

| Antigen | Fluorescence labeling substance | Clone | Manufacturer |
|---|---|---|---|
| CD4 | APC-Cy7 | RPA-T4 | BD Biosciences |
| CD25 | PE-Cy7 | BC96 | eBioscience |
| CXCR3 | Alexa fluor™ 488 | 1C6/CXCR3 | BD Biosciences |
| CXCR5 | PerCP-Cy5.5 | TG2/CXCR5 | Bio Legend |
| CCR4 | APC | TG6/CCR4 | Bio Legend |
| CCR6 | PE | 11A9 | BD Biosciences |

**Table 4**

| **Cell** | **Gating** |
|---|---|
| Tfh(2) | CD4^{high} CD25^{low-negative} CXCR3⁻ CXCR5⁺ CCR4⁻ CCR6⁻ |

### 4. Culture of Tfh cells (2)

The adult peripheral blood-derived Tfh cells (2) obtained in the above step 3. were seeded in a 96-well plate at a density of 1.0 x 10⁵ cells/0.3 ml/well. The medium used was the Yssel medium.
In order to activate and proliferate the above cells, magnetic beads coated with an anti-CD2/3/28 antibody (Miltenyi Biotec) (hereinafter also referred to as "antibody beads") were added to each well at 0.5 x 10⁵. The cells were added with cytokines and neutralizing antibodies suitable for differentiation culture of the Tfh cells (2) (Table 5) and cultured in an incubator at 37°C and 5% CO₂.

**Table 5**

| **Cell** | **Cytokine** | **Neutralizing antibody** |
|---|---|---|
| Tfh(2) | TGF-β1, IL-12, IL-2 | **Anti** IL-4 **antibody** (MP4-25D2), **Anti** IL-6 **antibody** (MQ2-13A5), **Anti** IFN-γ(R4-6A2) |

The concentration of all cytokines used is 10 ng/ml and the concentration of all neutralizing antibodies used is 2.5 µg/ml. The cytokines and neutralizing antibodies used were purchased from R&D Systems and Biolegend. After three days from the initiation of culture, the cells were diluted by three times with the medium containing the above cytokines and antibodies and cultured for further 4 days (7 days in total) to obtain Tfh cells (2).
The obtained Tfh cells (2) were divided into two portions, one of which was washed with the Yssel medium and PBS followed by collection of the cells by centrifugation and freezing at -80°C for storage of the cells until the next RNA extraction step. These cells are designated as Tfh cells (2) "without activation stimulation". The other portion of the cells was added with the antibody beads described above and cultured for further 3 hours to re-activate the cells. The cells were then recovered by centrifugation and frozen for storage at -80°C in a similar manner. These cells were designated as Tfh cells (2) "with activation stimulation".

### 5. Isolation of Th1, Th2, Treg, Th17 and Th22 cells from human peripheral blood

### (1) Isolation of Th1, Th2, Th17 and Th22 cells from human peripheral blood

Buffy coats obtained from peripheral blood of healthy adults were layered on Ficoll-paque plus solution (GE Healthcare Biosciences) and centrifuged to obtain the monocyte fraction. The CD4 positive cells were partially purified from the fraction by using magnetic beads (Miltenyi Biotec) coupled with an anti-CD4 antibody. The thus obtained CD4 positive cells were stained with fluorescence-labeled antibodies shown in Table 6 prior to isolate Th1, Th2, Th 17 and Th22 cells respectively with a cell sorter (FACS Aria: Becton Dickinson). The settings of gating for isolation are shown in Table 7.

**Table 6**

| Antigen | Fluorescence labeling substance | Clone | Manufacturer |
|---|---|---|---|
| CD4 | APC-Cy7 | RPA-T4 | BD Biosciences |
| CD25 | PE-Cy7 | BC96 | eBioscience |
| CXCR3 | Alexa fluor™ 488 | 1C6/CXCR3 | BD Biosciences |
| CCR4 | APC | FAB1567A | R&D systems |
| CCR6 | PE | 11A9 | BD Biosciences |
| CD45RA | APC | HI100 | BioLegend |
| CCR10 | PE | 6588-5 | BioLegend |

**Table 7**

| **Cell** | **Gating** |
|---|---|
| Th1 | CD4^{high} CD25^{low-negative} CXCR3⁺ CCR6⁻ CCR4⁻ |
| Th2 | CD4^{high} CD25^{low-negative} CXCR3⁻ CCR6⁻ CCR4⁺ |
| Th17 | CD4^{high} CD25^{low-negative} CXCR3⁻ CCR6⁺ CCR4⁺ |
| Th22 | CD4^{high} CD25^{low-negative} CD45RA⁻ CXCR3⁻ CCR10⁺ |

The details for the above procedures for sorting can be found in Acosta-Rodriguez EV. et al. ("Surface phenotype and antigenic specificity of human interleukin 17-producing T helper memory cells.", Nat Immunol., vol. 8, p.639-646 (2007)) and Trifari S. et al. ("Identification of a human helper T cell population that has abundant production of interleukin 22 and is distinct from TH-17, TH1 and TH2 cells.", Nat Immunol., vol. 10, p.864-871 (2009)).

### (2) Isolation of Treg cells from human peripheral blood

The CD4 positive cells obtained in the similar manner as the above step (1) were stained with fluorescence-labeled antibodies shown in Table 8 prior to purify Treg cells which were CD4^{high} CD25^{high} CD 127^{internal-negative} cells on the cell sorter described above.

**Table 8**

| Antigen | Fluorescence labeling substance | Clone | Manufacturer |
|---|---|---|---|
| CD4 | FITC | OKT4 | eBioscience |
| CD25 | PE-Cy7 | BC96 | eBioscience |
| CD45RO | PE | UCHL1 | BioLegend |
| CD127 | Alexa fluor™ 647 | HIL-7R-M21 | BD Biosciences |

The details for the above procedures for sorting can be found in Weihong Liu. et al. ("CD 127 expression inversely correlates with FoxP3 and suppressive function of human CD4+ Treg cells.", J Exp Med., vol. 203, p.1701-1711 (2006)).

### 6. Culture of Th1, Th2, Treg, Th17 and Th22 cells

### (1) Culture of Th1, Th2, Th17 and Th22 cells

The adult peripheral blood-derived Th1, Th2, Th17 and Th22 cells obtained in the above step 5.(1) were respectively seeded in a 96-well plate at a density of 1.5 x 10⁵ cells/0.3 ml/well. The medium used was the Yssel medium (IMDM, 1% type AB human serum, 0.25% BSA, 1.8 mg/l 2-aminomethanol, 40 mg/l transferrin, 5mg/l insulin, 2mg/l linoleic acid, 2mg/l oleic acid, 2mg/l palmitic acid and 1% penicillin/streptomycin). In order to activate and proliferate the above cells, magnetic beads coated with an anti-CD2/3/28 antibody (Miltenyi Biotec) (hereinafter also referred to as "antibody beads") were added to each well at 0.75 x 10⁵. The cells were added with cytokines and neutralizing antibodies suitable for differentiation culture of Th1, Th2, Th17 and Th22 cells, respectively, and cultured in an incubator at 37°C and 5% CO₂. The cytokines and neutralizing antibodies used are shown in Table 9.

**Table 9**

| **Cell** | **Cytokine** | **Neutralizing antibody (clone)** |
|---|---|---|
| Th1 | IL-12, IL-2 | **Anti** IL-4 **antibody** (MP4-25D2) |
| Th2 | IL-4, IL-2 | **Anti** IFN- *γ* **antibody** (R4-6A2) |
| Th17 | TGF- *β* 1, IL-6, IL-23, IL-21, IL-1 *β,* TNF *α*, IL-2 | **Anti** IL-4 **antibody** (MP4-25D2), **Anti** IFN- *γ* **antibody** (R4-6A2) |
| Th22 | IL-6, TNFα, IL-2 | **Anti** IL-4 **antibody** (MP4-25D2), **Anti** IFN- *γ* **antibody** (R4-6A2), **Anti** TGF-*β* **antibody** (9016) |

The concentration of cytokines was 50 ng/ml for IL-6 and 10 ng/ml for those other than IL-6. The concentration of the antibodies was 10 µg/ml for the anti-IFN-γ antibody and 2.5 µg/ml for the anti-IL-4 and anti-TGF-β antibodies. The cytokines and neutralizing antibodies were purchased from R&D systems and eBioscience, respectively. After three days from the initiation of culture, the cells were diluted by three times with the medium containing the above cytokines and antibodies and cultured for further 7 days (10 days in total) to obtain Th1, Th2, Th17 and Th22 cells. The obtained cells were respectively divided into two portions, each one of which was washed with the Yssel medium and PBS followed by collection of the cells by centrifugation and freezing at -80°C for storage of the cells until the next RNA extraction step. These cells are designated as Th1, Th2, Th17 and Th22 cells "without activation stimulation". The other portion of the cells was added with the antibody beads described above and cultured for further 3 hours to re-activate the cells. The cells were then recovered by centrifugation and frozen for storage at -80°C in a similar manner. These cells were respectively designated as Th1, Th2, Th17 and Th22 cells "with activation stimulation".

### (2) Culture of Treg cells

The Treg cells obtained in the above step 5. (2) were cultured in the Yssel medium as described in the above step 6.(1) and activated with the antibody beads. To the medium were added cytokines IL-2 and TGF-β1 (R&D systems) and neutralizing antibodies anti-IFN-γ and anti-IL-4 antibodies (eBioscience) and anti-IL-6 antibody (BD bioscience). The concentration of these cytokines and neutralizing antibodies was 10 ng/ml and 5 µg/ml, respectively. After three days from the initiation of culture, the cells were added with the cytokines and neutralizing antibodies at the same amounts as those at the initiation of culture. After additional three days of culture, the cells were divided into two portions, each of which was added with or without the antibody beads and cultured for further 3 days to obtain Treg cells "with activation stimulation" or "without activation stimulation". The respective Treg cells were collected by centrifugation and frozen at -80°C for storage until the next RNA extraction step.

### 7. Verification of purity of differentiation cultured cells by flow cytometry

To the respective cell suspensions containing the cells after differentiation culture (respectively containing 2.5 x 10⁵ cells) were added phorbol myristate acetate (PMA: 50 ng/ml) and ionomycin (1 µM) to stimulate the cells. After 4 hours, the cells were added with brefeldin A (10 µg/ml) and cultured for additional 2 hours. The cells were washed with phosphate buffered saline (PBS) and fixed with 4% paraformaldehyde. After fixation, the cells were treated with a saponin buffer (0.5% saponin, 0.5% bovine serum albumin (BSA), 1 mM sodium azide (in PBS)) in order to increase permeability of the cell membrane. The cells were allowed to react with a fluorescence (FITC, PE, APC, PerCP-Cy5.5, Alexa647 etc.)-labeled anti-IFN-γ, anti-IL-4, anti-IL-17, anti-Foxp3, anti-IL-9, anti-IL-22 or anti-IL-21 antibody. After the reaction, the cells were washed with the saponin buffer and then with a 0.5% BSA-containing PBS and analyzed on FACS Canto II (Becton Dickinson) to confirm the purity of the cells. The items used for confirmation of purity were respectively as follows: Th1 cells: IFN-γ, Th2 cells: IL-4, Th17 cells: IL-17A, Treg cells: Foxp3 (transcription factor), Th9 cells: IL-9, Th22 cells: IL-22 and Tfh cells (1) and (2): IL-21.

### 8. Extraction of total RNA

In order to extract total RNA from the cells obtained in the above steps 1., 2., 4. and 6., the RNeasy Plus Mini kit and the RNeasy micro kit (QIAGEN) were respectively used. The specific procedures followed the instructions attached to each kit.

### 9. Microarray expression assay

Total RNA (10 to 100 ng) extracted from the cells in the step 7. was reverse-transcribed to cDNA and further transcribed to biotinylated cRNA with Two-Cycle Target Labeling and Control Reagents (Affymetrix). The amplified biotinylated cRNA (20 µg) was then fragmented. The specific procedures followed the instructions attached to the kit.
The biotinylated cRNA (15 µg) from the respective cells obtained as above was used as a sample and added to the GeneChip Human Genome U-133 Plus 2.0 Array (Affymetrix), which was then transferred to the GeneChip Hybridization Oven 640 (Affymetrix) in order to carry out hybridization at 45°C and 60 rpm for 16 hours. After hybridization, the microarray was washed and fluorescence-labeled on the GeneChip Fluidic Station 450 (Affymetrix) and scanned on the GeneChip Scanner 3000 7G (Affymetrix) to acquire fluorescence intensity data.

### 10. Selection of genes specifically expressed in human Th22 cells

The fluorescence intensity data obtained in the above step 8. was standardized with MAS5 algorithm on the expression assay software GeneSpring GX Ver.11 (Agilent Technologies) in order to obtain relative fluorescence intensity of genes in the cells. The relative fluorescence intensity corresponds to the amount of gene expression in the cells. The obtained relative fluorescence intensity of the genes in Tfh cells (1) was compared with the relative fluorescence intensity in naïve CD4 positive T cells, Th1, Th2, Treg, Th17, Th9 and Th22 cells. The genes which have the relative fluorescence intensity in Tfh cells (1) three times or more than the relative fluorescence intensity in all naive CD4 positive T cells, Th1, Th2, Treg, Th17, Th9 and Th22 cells and which are expressed significantly (the genes which showed "p value < 0.05" in ANOVA test for the relative fluorescence intensity between 8 groups of naive CD4 positive T cells, Th1, Th2, Treg, Th17, Th9 and Th22 cells and Tfh cells (1)) were identified as the genes specifically expressed in Tfh cells (1).
In the similar manner, the genes which have the relative fluorescence intensity in Tfh cells (2) three times or more than the relative fluorescence intensity in all naive CD4 positive T cells, Th1, Th2, Treg, Th 17, Th9 and Th22 cells and which are expressed significantly (the genes which showed "p value < 0.05" in ANOVA test for the relative fluorescence intensity between 8 groups of naive CD4 positive T cells, Th1, Th2, Treg, Th 17, Th9 and Th22 cells and Tfh cells (2)) were identified as the genes specifically expressed in Tfh cells (2). The number of samples used in this selection step is shown in Table 10.

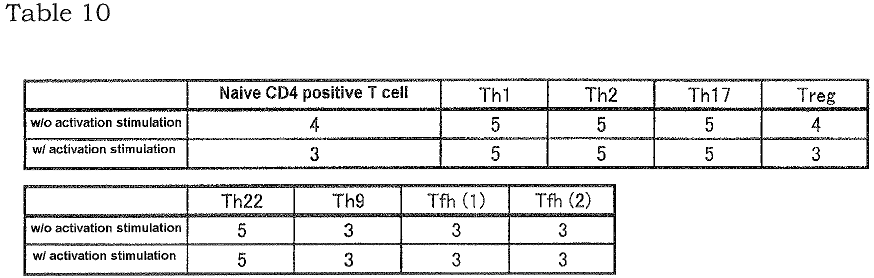

The genes which are specifically expressed in Tfh cells (1) and Tfh cells (2) under the conditions "with activation stimulation" and "without activation stimulation" and the ratio of expression amount of the genes between the cells are shown in Tables 11 to 14. The genes shown in Tables 11 and 12 are the genes which are specifically expressed in Tfh cells (1) "without activation stimulation" and "with activation stimulation", respectively. The genes shown in Tables 13 and 14 are the genes which are specifically expressed in Tfh cells (2) "without activation stimulation" and "with activation stimulation", respectively. "Localization" in the Tables means intracellular localization of proteins encoded by the genes.

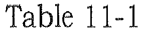

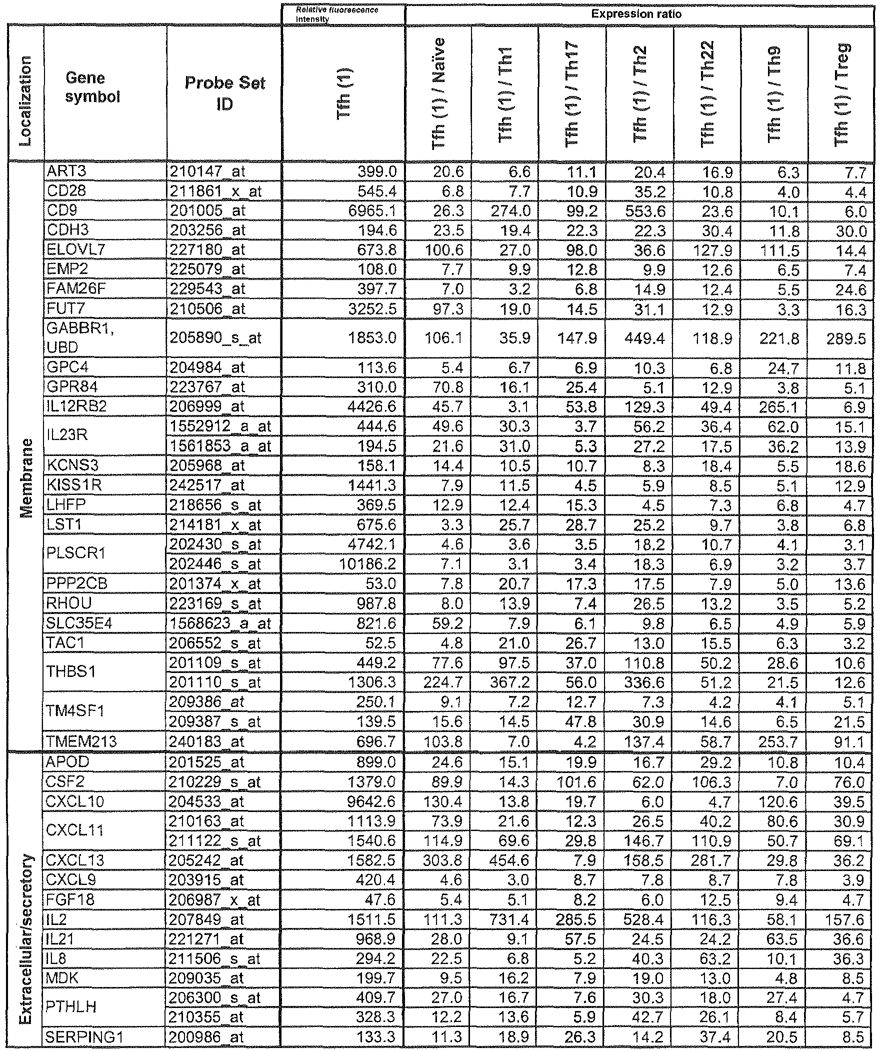

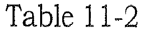

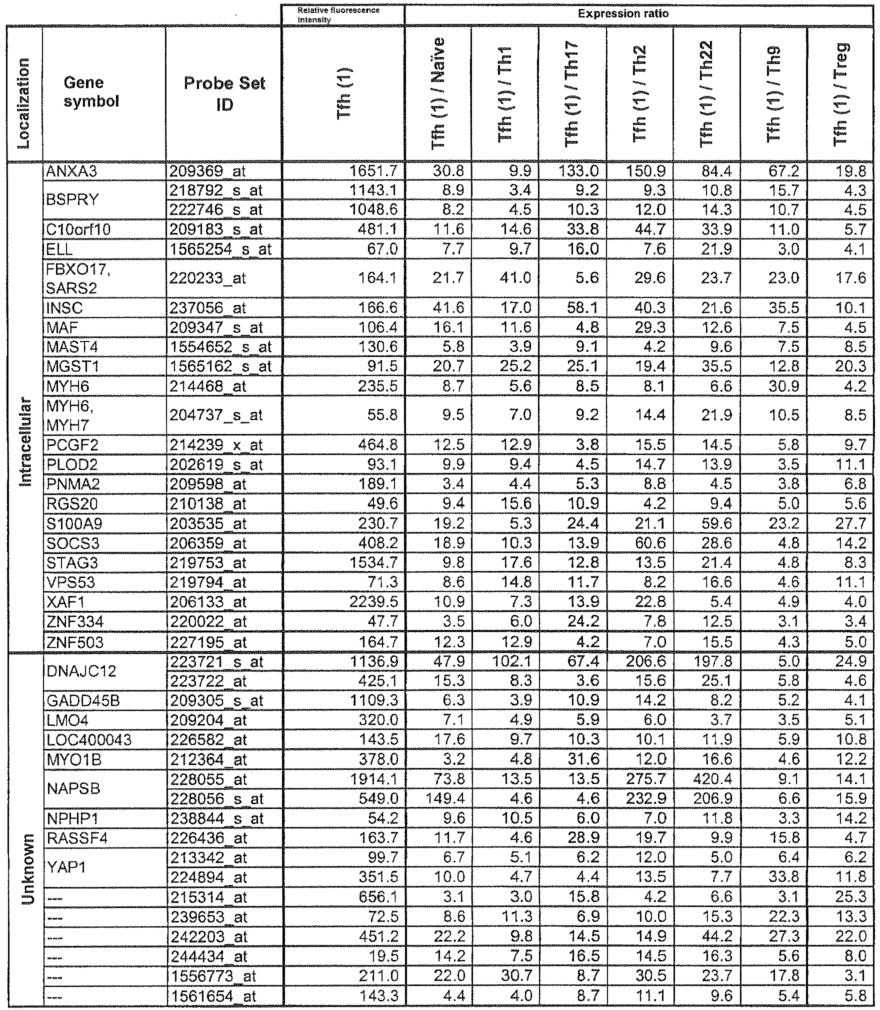

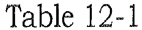

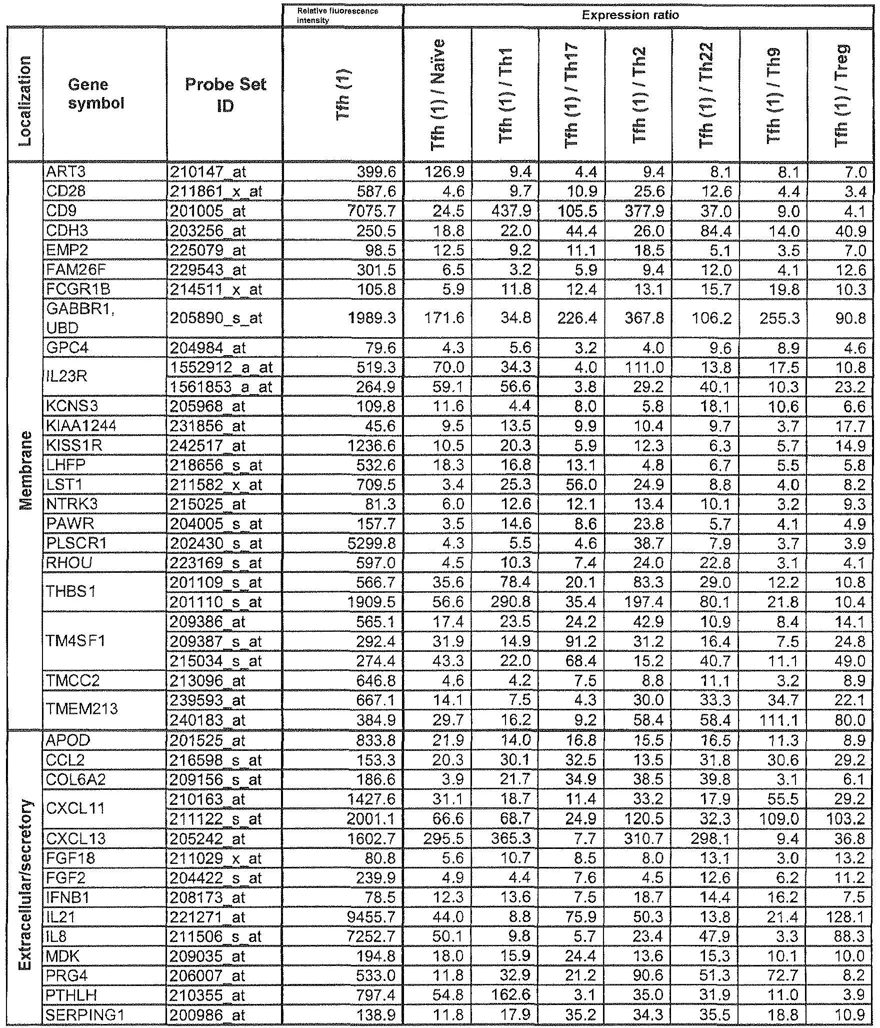

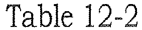

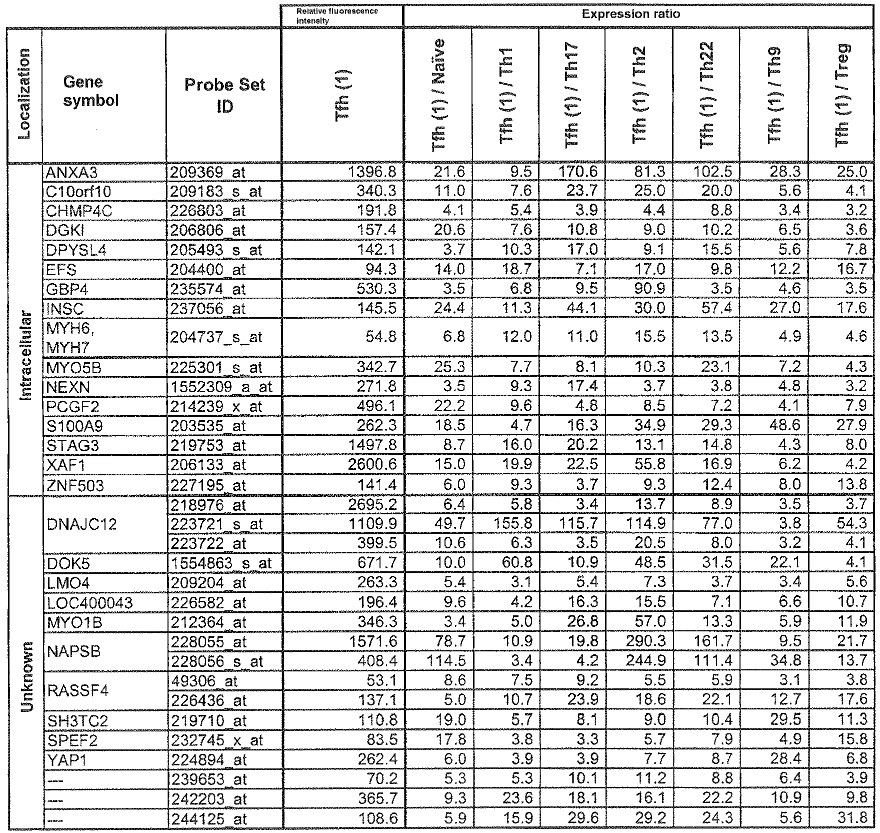

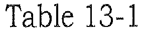

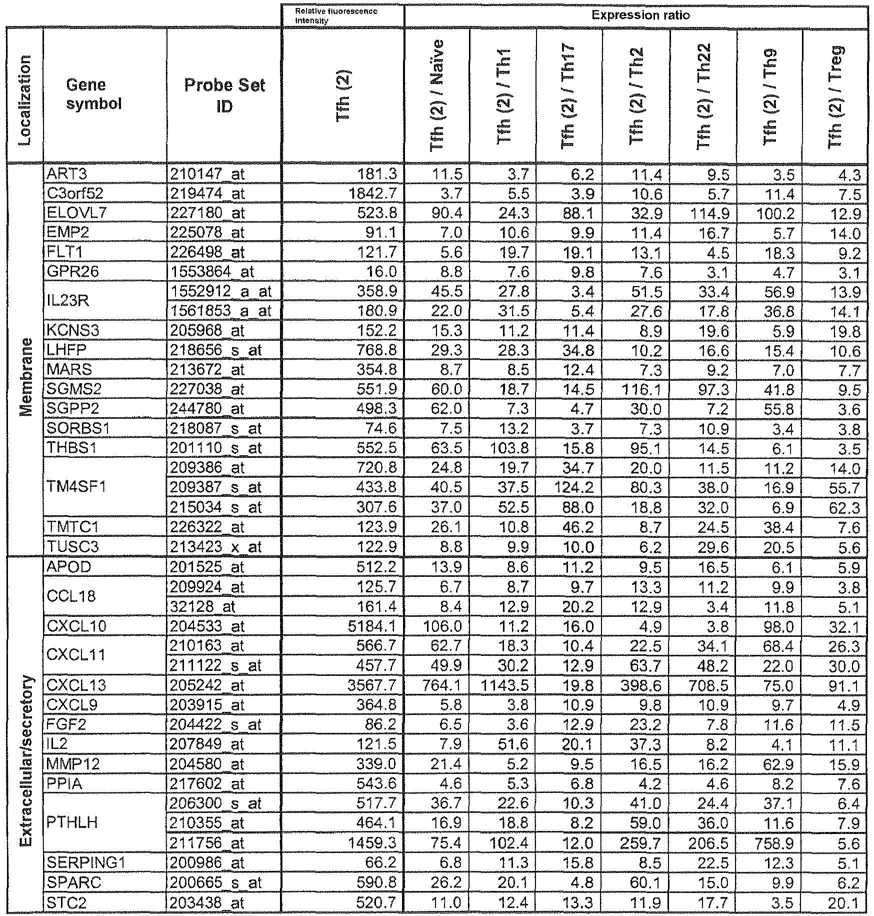

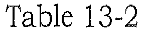

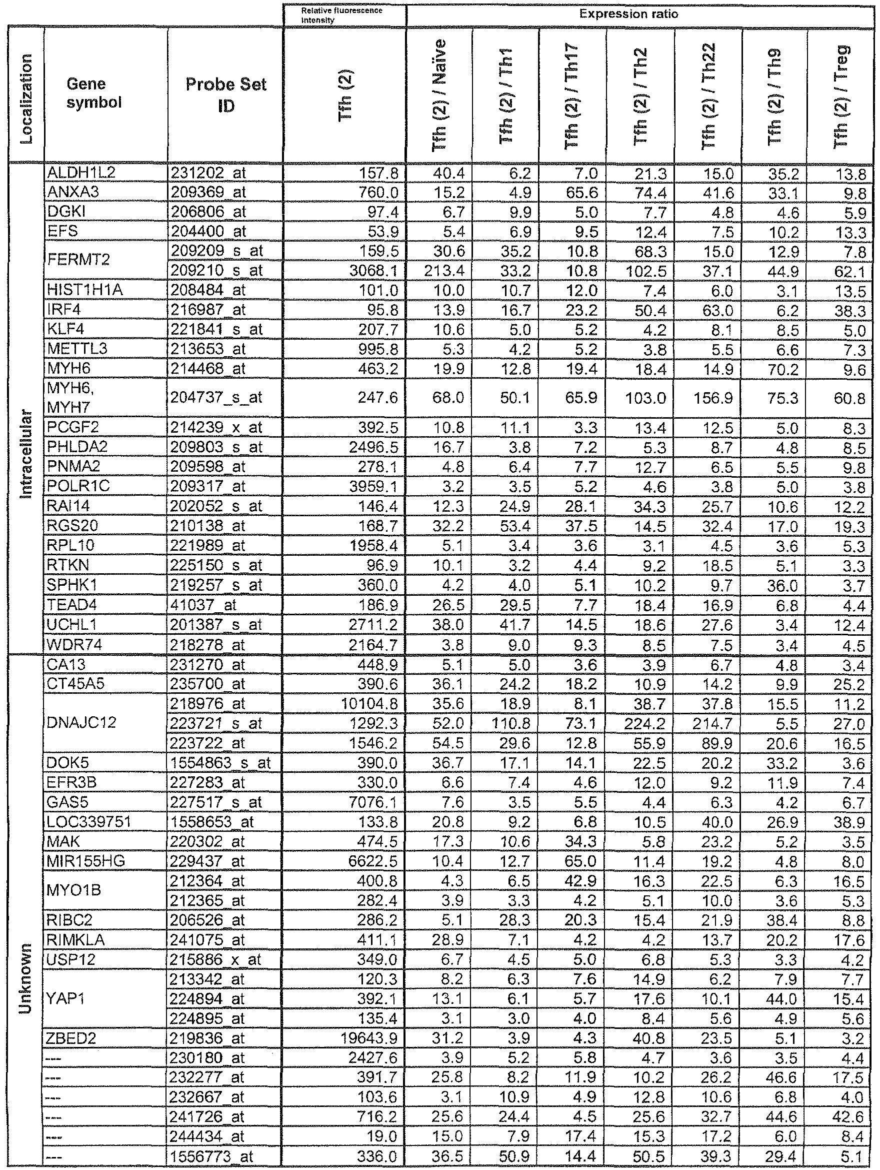

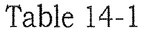

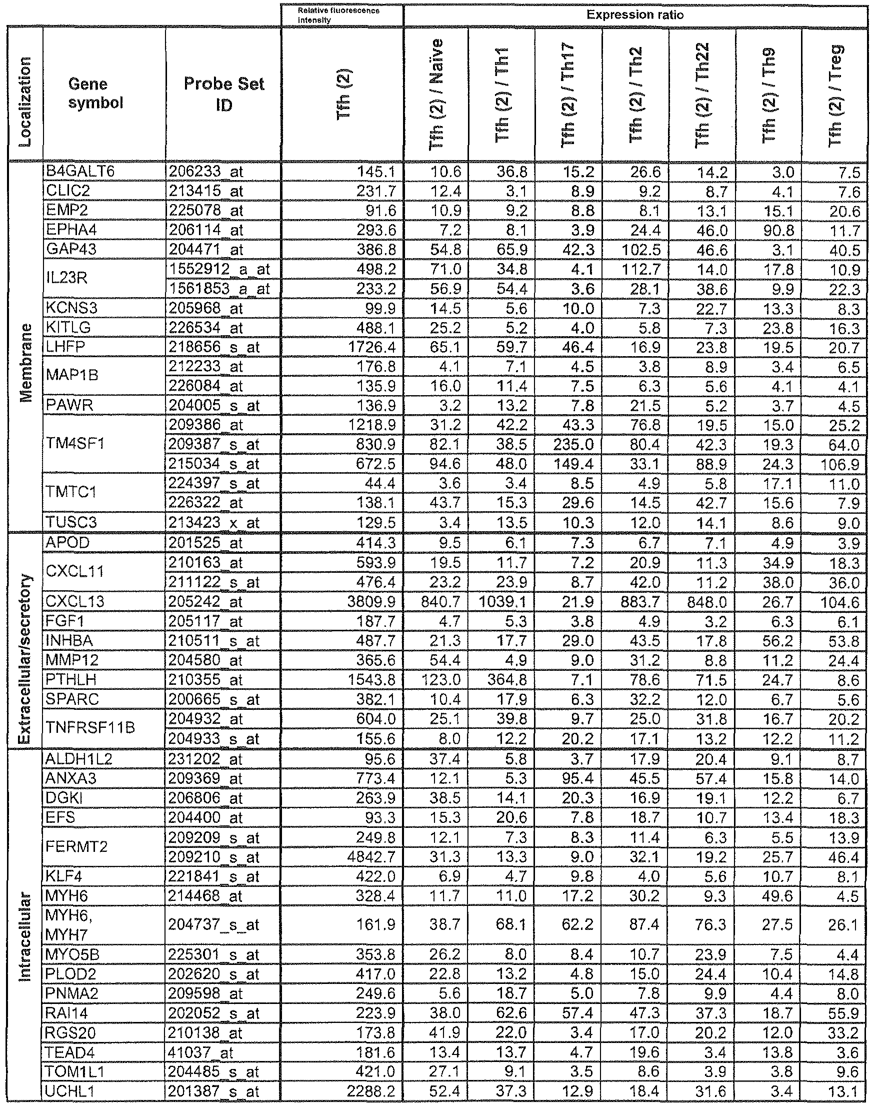

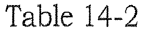

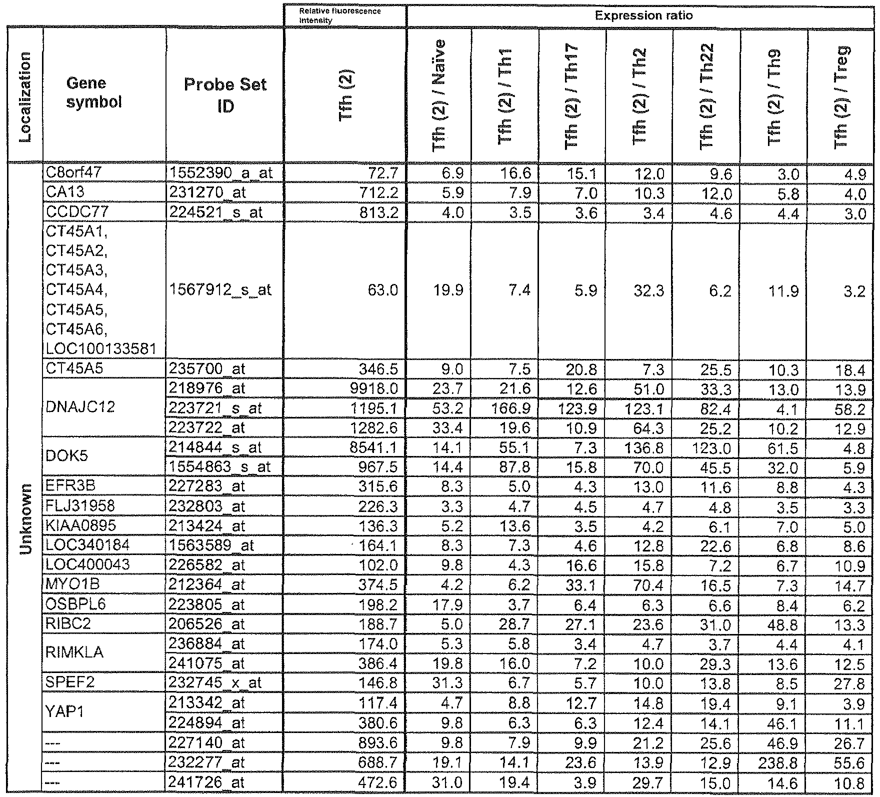

In the present Example, the genes which were found to be specifically expressed in Tfh cells by Chtanova T. et al. (see Chtanova T. et al., *supra*), i.e. SGPP2 (sphingosine-1-phosphate phosphatase 2), CXCL13 (chemokine (C-X-C motif) ligand 13), IL-21 (interleukin 21) and MAF (v-maf musculoaponeurotic fibrosarcoma oncogene homolog) were also identified. These known genes are shown in Table 15.

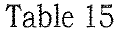

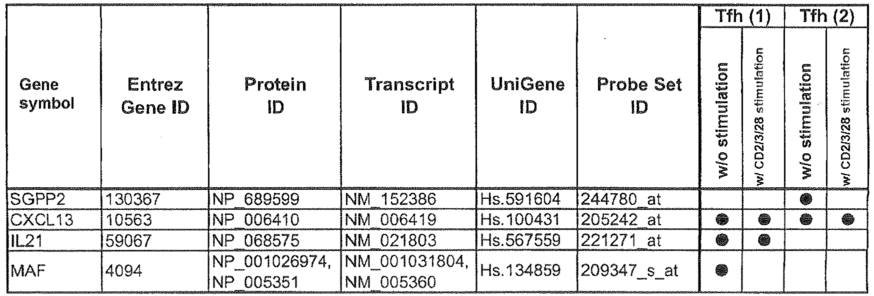

As described above, the genes which have been known to be specifically expressed in Tfh cells could also be detected in the present Example. Therefore, it is apparent that the genes specifically expressed in Tfh cells can be identified according to the present approach for gene search using the Tfh cells and other subsets of Th cells as described above.
The present inventors have identified 161 genes which are specifically expressed in Tfh cells corresponding to the genes identified as above excluding 4 genes shown in Table 15 as the polynucleotide markers for detecting Tfh cells according to the present invention.

The list of the genes as the present polynucleotide marker is shown in Table 16. In Table 16, the genes marked with "•" are specifically expressed in Tfh (1) and Tfh (2) cells with or without activation stimulation with anti-CD2/3/28 antibody.

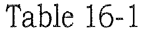

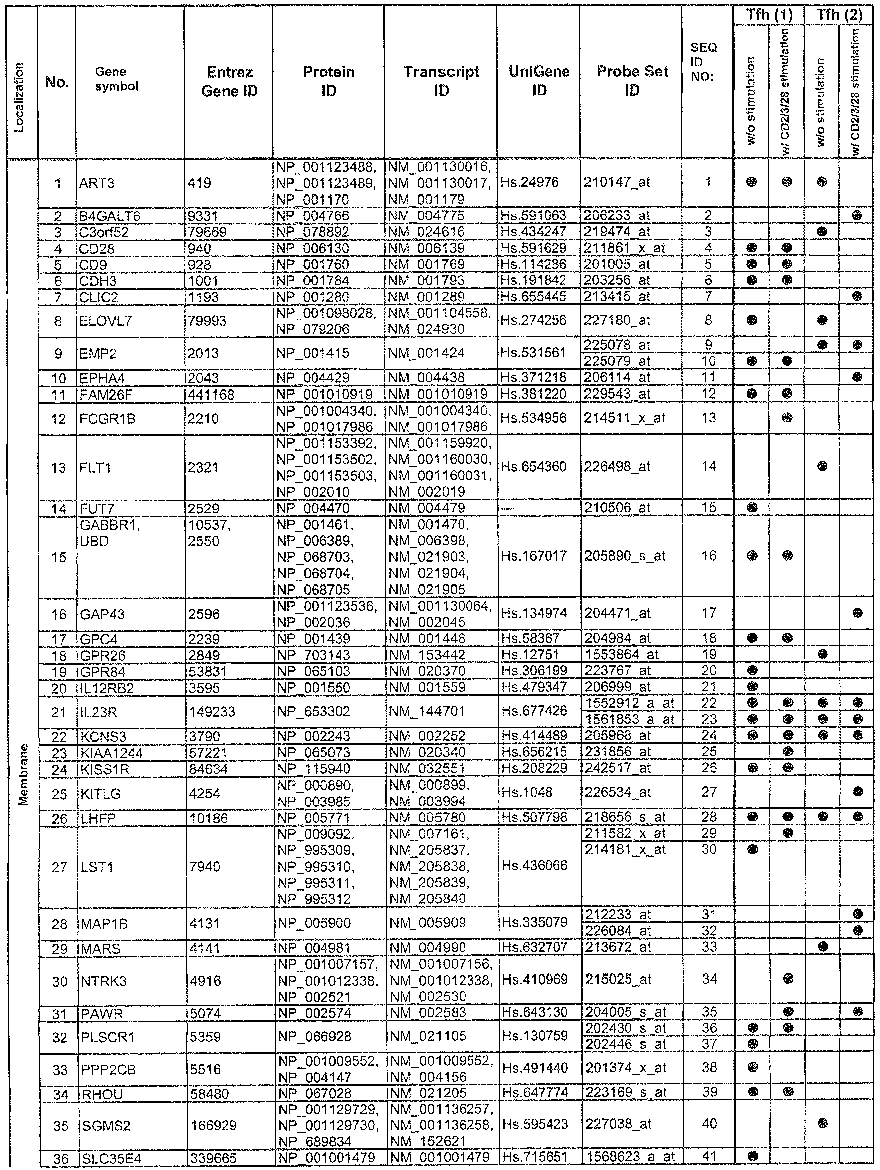

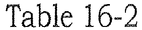

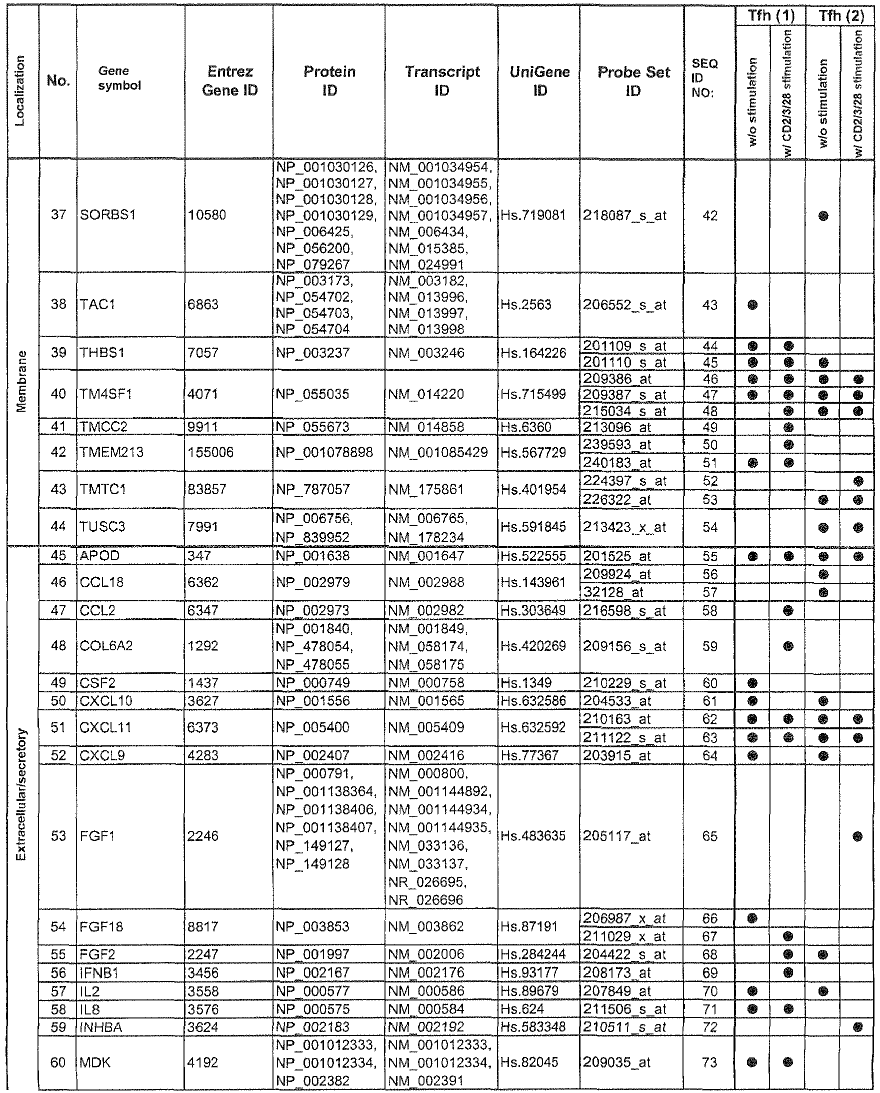

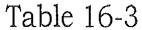

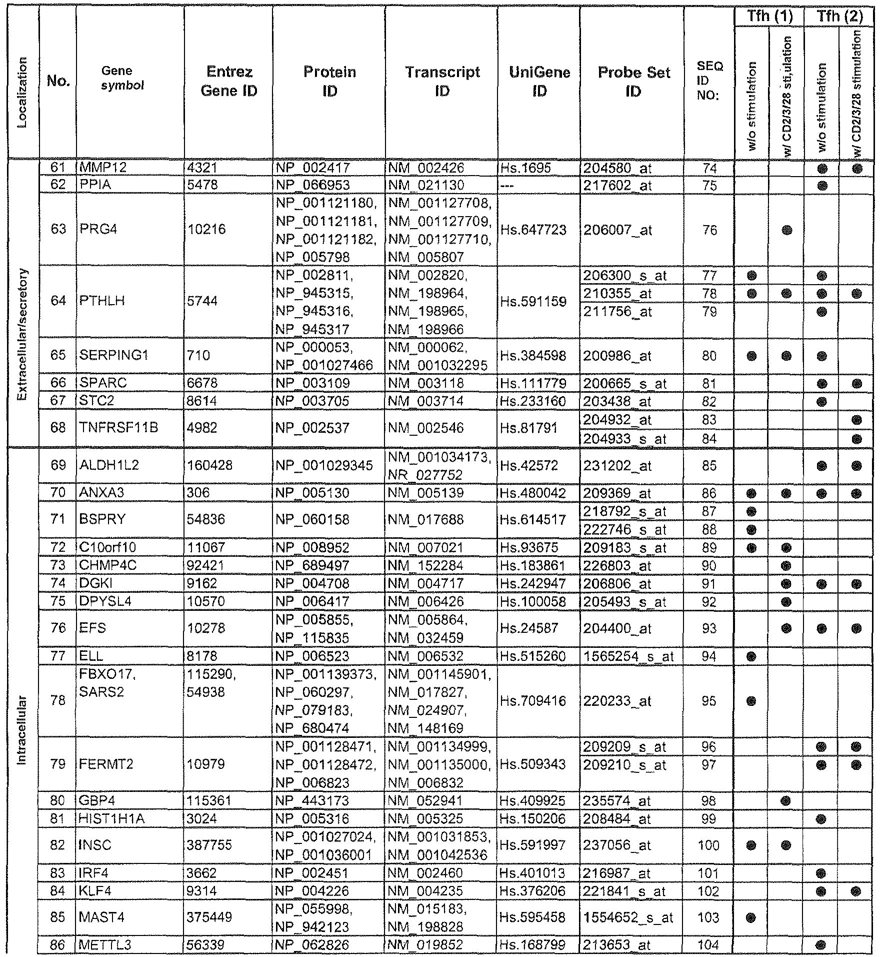

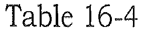

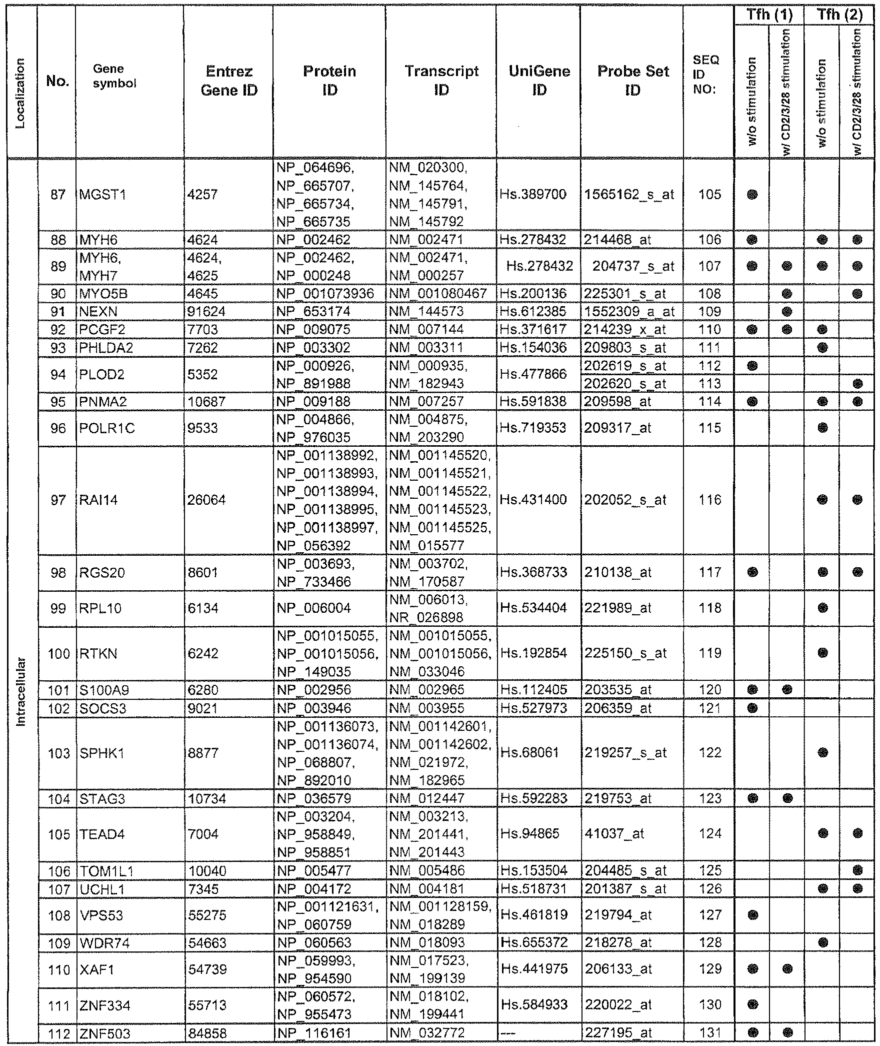

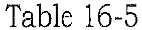

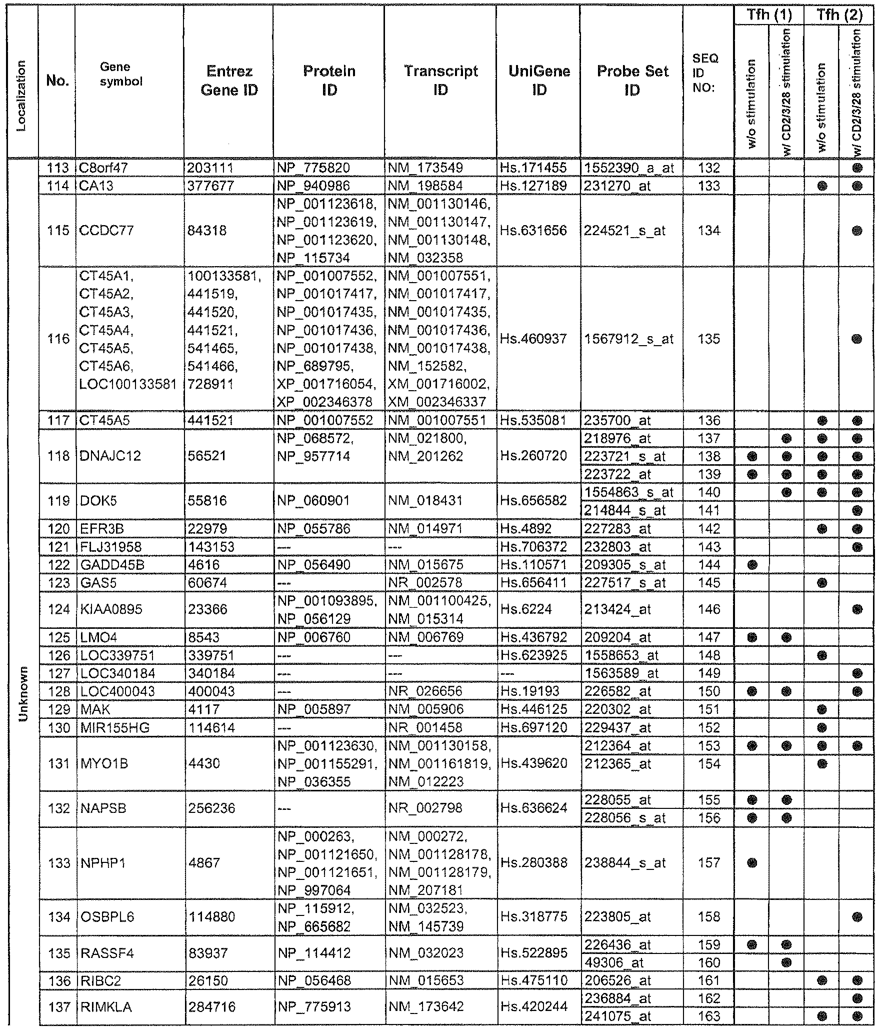

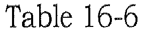

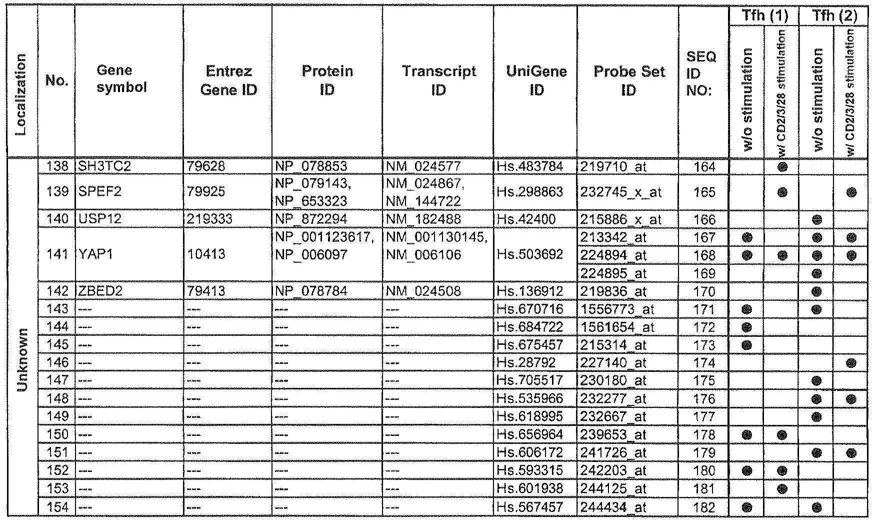

As apparent from Table 16, 161 genes of the present invention are useful as the polynucleotide markers for detecting Tfh cells.

### Example 2: Expression assay of protein markers for detecting Tfh cells in human peripheral blood-derived cultured Th1, Th2 and Tfh cells

### 1. Preparation of Th cells

Tfh cells, Th1 cells and Th2 cells in human peripheral blood were separated with a cell sorter (FACS Aria: Becton Dickinson). The separated respective Th cells were seeded in a 96-well plate at a density of 1.0 x 10⁵ cells/0.3 ml/well. The medium use was the Yssel medium. In order to allow activation and proliferation of Th cells, magnetic beads coated with an anti-CD2/3/28 antibody (Miltenyi Biotec) were added to each well at 0.5 x 10⁵. The cells were added with cytokines and neutralizing antibodies suitable for differentiation culture of respective Th cells and cultured in an incubator at 37°C and 5% CO₂. The antibodies and settings for gating used for separation and culture conditions for the Th cells are the same as Example 1.

### 2. Preparation of sample for CD9 measurement

The prepared respective Th cells (5 x 10⁶ cells/ml) were added with a PE-labeled anti-human CD9 antibody (BioLegend) at a final concentration of 0.2 µg/ml and incubated at 4°C for 20 minutes. After the reaction, the Th cells were added with PBS containing 0.5% BSA and recovered by centrifugation. The washed Th cells were suspended in PBS containing 0.5 µg/ml 7-aminoactinomycin D (7-AAD) and 0.5% BSA to prepare a sample for CD9 measurement (5 x 10⁶ cells/ml). A negative control sample (5 x 10⁶ cells/ml) was prepared by adding a PE-labeled murine IgG 1 isotype control (Biolegend) at a final concentration of 1.0 µg/ml instead of the anti-CD9 antibody and incubating at 4°C for 20 minutes.

### 3. Preparation of sample for CXCR5 measurement

Samples for CXCR5 measurement for the respective Th cells (5 x 10⁶ cells/ml) were prepared in the same manner as the preparation of sample for CD9 measurement as above 2. except that a PerCP/Cy5.5-labeled anti-CXCR5 antibody (BD Biosciences) at a final concentration of 1.0 µg/ml was used instead of the PE-labeled anti-CD9 antibody.
A negative control sample (5 x 10⁶ cells/ml) was prepared by adding a PerCP/Cy5.5-labeled anti-mouse IgG2b antibody (BioLegend) at a final concentration of 1.0 µg/ml instead of the PerCP/Cy5.5-labeled anti-CXCR5 antibody and incubating at 4°C for 20 minutes.

### 4. Preparation of sample for IL-21 measurement

In order to measure IL-21 positive cells in the prepared respective Th cell samples, samples for measurement of IL-21 were prepared from the respective samples. To the samples which were adjusted to 2.5 x 10⁵ cells/ml were added phorbol myristate acetate (PMA: 50 ng/ml) and ionomycin (1 µM) to stimulate the cells. After 4 hours, the cells were added with brefeldin A (10 µg/ml) and cultured for additional 2 hours. The cells were washed with phosphate buffered saline (PBS) and fixed with 4% paraformaldehyde. After fixation, the cells were treated with a saponin buffer (0.5% saponin, 0.5% bovine serum albumin (BSA), 1 mM sodium azide (in PBS)) in order to increase permeability of the cell membrane. The cells were allowed to react with a fluorescence (PE)-labeled anti-IL-21 antibody. After the reaction, the cells were washed with the saponin buffer and then with the 0.5% BSA-containing PBS and then suspended in the 0.5% BSA-containing PBS to prepare the samples for IL-21 measurement.
A negative control sample was prepared by adding the PE-labeled anti-mouse IgG 1 antibody (BioLegend) at a final concentration of 1.0 µg/ml instead of the PE-labeled anti-IL-21 antibody and incubating at 4°C for 20 minutes.

### 5. Expression assay of CD9 protein using flow cytometer

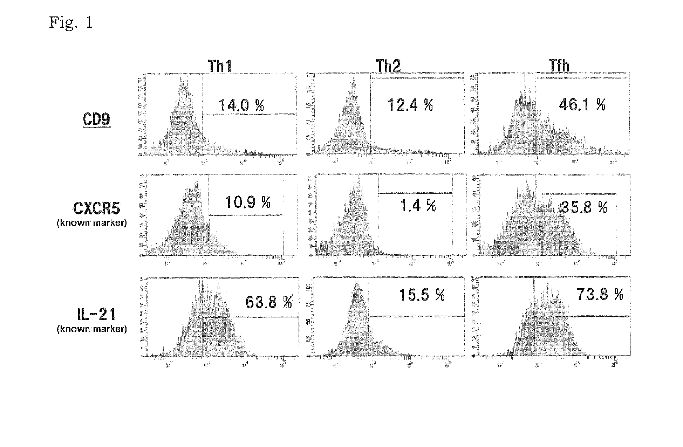

The samples for CD9 measurement, CXCR5 measurement and IL-21 measurement prepared as above were analyzed with the FACSCanto II (BD Biosciences) and FACS DIVA software (BD Biosciences). The histograms (particle distribution) of fluorescence intensity obtained from the analyses are shown in Fig. 1. In Fig. 1, the vertical axis of histograms represents the number of cells and the horizontal axis represents fluorescence intensity. The numbers shown in upper right of the histograms represent the ratio (%) of marker positive cells relative to total cells in the respective samples. The positive cells and negative cells were determined based on the maximum fluorescence intensity in negative controls. Namely, the cells having the fluorescence intensity higher than the maximum fluorescence intensity in a negative control were determined as positive cells. On the other hand, the cells having the fluorescence intensity at or lower than the maximum fluorescence intensity in the negative control were determined as negative cells. The positive cell ratio was calculated as the ratio of the positive cells relative to total number of cells.

According to Fig. 1, it is found that CD9 is specifically and highly expressed in Tfh cells than in Th1 cells and Th2 cells. It is also found that the positive cell ratio of Tfh cells in the sample for CD9 measurement is higher than the sample for CXCR5 measurement, which is a known marker for Tfh cells. Moreover, the positive cell ratio of the sample for IL-21measurement, which is a known marker for Tfh cells, is high for not only Tfh cells but also for Th1 cells, while the positive cell ratio of the sample for CD9 measurement is low for Th 1 cells and Th2 cells and high only for Tfh cells. In summary, it is found that CD9 protein can be suitably used as a protein marker for detecting Tfh cells.

### Example 3: Analysis of Tfh cell ratio in Tfh cell detection marker fraction

### 1. Preparation of fractionated sample

The cultured Tfh cells prepared in Example 2 were stained with the PerCP/ Cy5.5-labeled anti-CXCR5 antibody to prepare a sample for CXCR5 measurement. The cultured Tfh cells were stained with the PerCP/Cy5.5-labeled anti-CXCR5 antibody and the PE-labeled anti-human CD9 antibody to prepare a sample for CXCR5/CD9 measurement. CXCR5 positive cells were separated from the sample for CXCR5 measurement with a cell sorter (FACS Aria: Becton Dickinson). CXCR5 positive and CD9 positive cells were separated from the sample for CXCR5/CD9 measurement with the cell sorter.
The separated cells were respectively seeded in a 96-well plate at a density of 1.5 x 10⁵ cells/0.3 ml/well or less. The medium used was the Yssel medium containing IL-2 (R&D systems) at a final concentration of 10 ng/ml and the cells were cultured in an incubator at 37°C and 5% CO₂ for 3 days to prepare a CXCR5 fractionated sample and a CXCR5/CD9 fractionated sample.

### 2. Sample processing for measurement of IL-21 positive cells

In order to measure IL-21 positive cells in the cultured Tfh cell sample before fractionation as described in 1., and the CXCR5 fractionated sample and the CXCR5/CD9 fractionated sample after fractionation, samples for IL-21 measurement were prepared therefrom. To the samples adjusted to 2.5 x 10⁵ cells/ml were added phorbol myristate acetate (PMA: 50 ng/ml) and ionomycin (1 µM) to stimulate the cells. After 4 hours, the cells were added with brefeldin A (10 µg/ml) and cultured for additional 2 hours. The cells were then washed with phosphate buffered saline (PBS) and fixed with 4% paraformaldehyde. After fixation, the cells were treated with a saponin buffer (0.5% saponin, 0.5% bovine serum albumin (BSA), 1 mM sodium azide (in PBS)) in order to increase permeability of the cell membrane. The cells were allowed to react with a fluorescence (PE)-labeled anti-IL-21 antibody. After the reaction, the cells were washed with the saponin buffer and with the 0.5% BSA-containing PBS and then suspended in the 0.5% BSA-containing PBS to prepare the samples for IL-21 measurement.
A negative control sample was prepared by adding the PE-labeled anti-mouse IgG 1 antibody (BioLegend) at a final concentration of 1.0 µg/ml instead of the PE-labeled anti-IL-21 antibody and incubating at 4°C for 20 minutes.

### 3. Measurement of IL-21 positive cells

The samples for IL-21 measurement prepared as in the above 2. were analyzed with the FACSCanto II (BD Biosciences) and FACS DIVA software (BD Biosciences). The ratio of IL-21 positive cells relative to total number of cells in the samples for IL-21 measurement was calculated to obtain the Tfh cell ratio (%).
The positive cells and negative cells were determined based on the maximum fluorescence intensity in the negative control. Namely, the cells having the fluorescence intensity higher than the maximum fluorescence intensity in the negative control were determined as positive cells.
On the other hand, the cells having the fluorescence intensity at or lower than the maximum fluorescence intensity in the negative control were determined as negative cells. The positive cell ratio was calculated as the ratio of the positive cells relative to total number of cells.

### 4. Calculation of enrichment ratio of Tfh cells

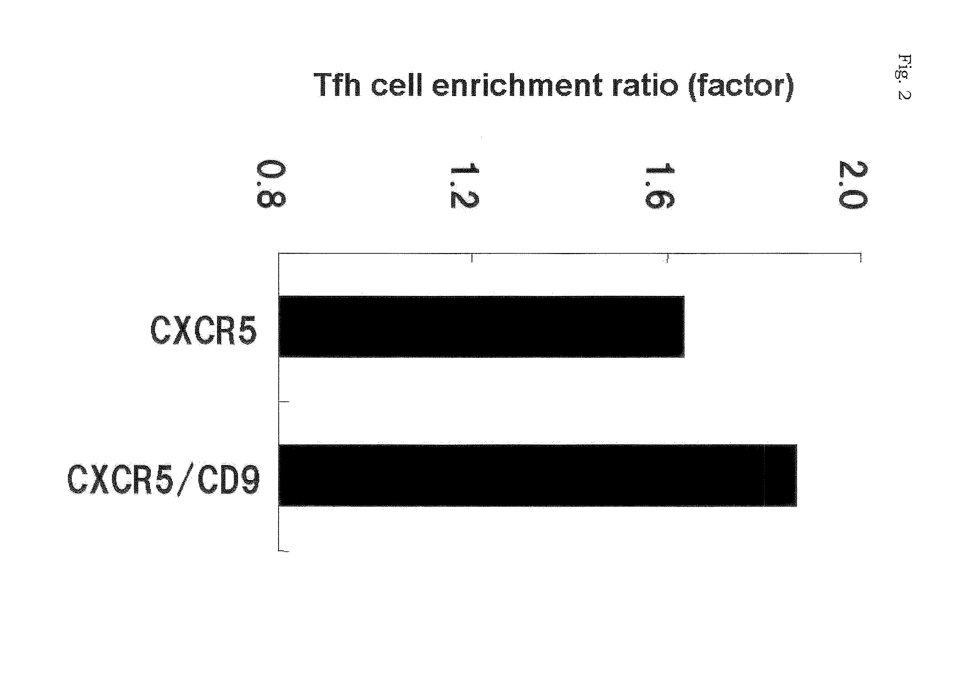

Based on the Tfh cell ratio (%) of the samples obtained in the above 3., enrichment ratio of Tfh cells in the Tfh cell detection marker fractions was calculated. Specifically, the ratio of the Tfh cell ratio (%) of the respective fractions relative to the Tfh cell ratio (%) before fractionation was calculated. The obtained ratios are shown in Fig. 2 as the enrichment ratios of Tfh cells in the CXCR5 fractionated sample and CXCR5/CD9 fractionated sample. According to Fig. 2, Tfh cells were enriched to a higher concentration in the CXCR5/CD9 fraction than CXCR5 which is a known marker for Tfh cells. Therefore, it is found that the combination of CD9 protein and CXCR5 protein is useful as a protein marker for detecting Tfh cells compared to the CXCR5 protein alone.

The present application relates to Japanese Patent Application No. 2010-253140 filed on November 11, 2010, whose claims, specification and abstract are incorporated herein by reference.

## Claims

1. A polynucleotide marker for detecting human follicular helper T cells, which is a polynucleotide having a base sequence of at least one gene selected from:
a gene encoding a membrane protein represented by CD9, TM4SF1, IL23R, ART3, ELOVL7, KCNS3, LHFP, PAWR, THBS1, B4GALT6, C3orf52, CD28, CDH3, CLIC2, EMP2, EPHA4, FAM26F, FCGR1B, FLT1, FUT7, GABBR1, UBD, GAP43, GPC4, GPR26, GPR84, IL12RB2, KIAA1244, KISS1R, KITLG, LST1, MAP1B, MARS, NTRK3, PLSCR1, PPP2CB, RHOU, SGMS2, SLC35E4, SORBS1, TAC1, TMCC2, TMEM213, TMTC1 or TUSC3;
a gene encoding an extracellular/ secretory protein represented by APOD, CXCL9, CXCL10, CXCL11, FGF2, IL2, PTHLH, SERPING1, CCL18, CCL2, COL6A2, CSF2, FGF1, FGF18, IFNB1, IL8, INHBA, MDK, MMP12, PPIA, PRG4, SPARC, STC2 or TNFRSF11B;
a gene encoding an intracellular protein represented by ANXA3, DGKI, EFS, MYH6, MYH7, MYO5B, PCGF2, PNMA2, RGS20, ALDH1L2, BSPRY, C10orf10, CHMP4C, DPYSL4, ELL, FBXO17, SARS2, FERMT2, GBP4, HIST1H1A, INSC, IRF4, KLF4, MAST4, METTL3, MGST1, NEXN, PHLDA2, PLOD2, POLR1C, RAI14, RPL10, RTKN, S100A9, SOCS3, SPHK1, STAG3, TEAD4, TOM1L1, UCHL1, VPS53, WDR74, XAF1, ZNF334 or ZNF503;
a gene represented by DNAJC12, DOK5, LOC400043, MYO1B, SPEF2, YAP1, C8orf47, CA13, CCDC77, CT45A1, CT45A2, CT45A3, CT45A4, CT45A5, CT45A6, LOC100133581, EFR3B, FLJ31958, GADD45B, GAS5, KIAA0895, LMO4, LOC339751, LOC340184, MAK, MIR155HG, NAPSB, NPHP1, OSBPL6, RASSF4, RIBC2, RIMKLA, SH3TC2, USP12 or ZBED2; and
a gene consisting of a base sequence represented by any of SEQ ID NOs: 171, 182 and 172 to 181;
or a variant or fragment thereof.

2. The polynucleotide marker according to claim 1, which is a polynucleotide having the base sequence of the gene encoding CD9 or a variant or fragment thereof.

3. A polynucleotide marker for detecting human follicular helper T cells, which is a combination of the polynucleotide marker according to claim 2 and a polynucleotide having a base sequence of a gene encoding CXCR5 or a variant or fragment thereof.

4. A protein marker for detecting human follicular helper T cells, which is a protein encoded by at least one gene selected from the genes defined in claim 1 or by the gene defined in claim 2, or a functionally equivalent variant or fragment thereof.

5. A protein marker for detecting human follicular helper T cells, which is a combination of the protein marker according to claim 4 depending on claim 2 and a protein having an amino acid sequence of CXCR5 or a functionally equivalent variant or fragment thereof.

6. A method for detecting human follicular helper T cells, comprising detecting at least one polynucleotide marker for detecting human follicular helper T cells according to any one of claims 1 to 3 or protein marker for detecting human follicular helper T cells according to claim 4 or 5 in a sample containing cells.
